# EUROPEAN PATENT SPECIFICATION

(11) **EP 0 843 961 B1**
(45) Date of publication and mention of the grant of the patent: **24.01.2007**
(21) Application number: 96928694.7
(22) Date of filing: 29.08.1996
(51) Int. Cl.: C12N 15/00, A01K 67/027, C12N 15/90, C12N 5/10, C07K 16/00, C12N 15/08, C12N 15/06, A61K 48/00

(54) **CHIMERIC ANIMAL AND METHOD FOR CONSTRUCTING THE SAME**
CHIMÄRES TIER UND METHODE ZU DESSEN HERSTELLUNG
ANIMAL CHIMERIQUE ET PROCEDE DE CONSTITUTION

(30) Priority: 29.08.1995 JP 24234095; 15.02.1996 JP 2794096
(43) Date of publication of application: 27.05.1998
(73) Proprietor: KIRIN BEER KABUSHIKI KAISHA, Tokyo 104-8288 (JP)
(72) Inventor: TOMIZUKA, Kazuma, Kirin Beer Kabushiki Kaisha, Kanazawa-ku, Yokohama-shi, Kanagawa 236 (JP); YOSHIDA, Hitoshi, Kirin Beer Kabushiki Kaisha, Kanazawa-ku, Yokohama-shi, Kanagawa 236 (JP); HANAOKA, Kazunori, Kitasato Daigaku Rigaku-bu, Sagamihara-shi, Kanagawa 228 (JP); OSHIMURA, Mitsuo, Tottori Daigaku Igaku-bu, Yonago-shi, Tottori 683 (JP); ISHIDA, Isao, Kirin Beer Kabushiki Kaisha Kiban, Yokohama-shi, Kanagawa 236 (JP)
(74) Representative: Polz, Leo
(86) International application number: PCT/JP1996/002427
(87) International publication number: WO 1997/007671

(56) References cited:
- TOMIZUKA K ET AL: "FUNCTIONAL EXPRESSION AND GERMLINE TRANSMISSION OF A HUMAN CHROMOSOME FRAGMENT IN CHIMAERIC MICE" NATURE GENETICS,US,NEW YORK, NY, vol. 16, no. 2, 16 June 1997 (1997-06-16), pages 133-143, XP000770172 ISSN: 1061-4036
- JAKOBOVITS A ET AL: "GERM-LINE TRANSMISSION AND EXPRESSION OF A HUMAN-DERIVED YEAST ARTIFICIAL CHROMOSOME" NATURE, vol. 362, no. 6417, 18 March 1993 (1993-03-18), pages 255-258, XP002025628
- NING, Y. ET AL.: "Isolation of monochromosomal hybrids following fusion of human diploid fibroblast-derived microcells with mouse A9 cells" CYTOGENETICS AND CELL GENETICS, vol. 60, no. 1, 1992, pages 79-80, XP000671424
- LANGSTON, A. & FOURNIER, R.E.K.: "Preparation and properties of microcell hybrids" METHODS IN MOLECULAR GENETICS, vol. 1, no. part A., 1993, pages 115-133, XP000671521
- TED K. CHOI et al., NATURE GENETICS, Vol. 4, p. 117-123 (1993).
- I. JEAN McGOWAN-JORDAN et al., CANCER RES., Vol. 54, p. 2568-2572 (1994).
- P.J. SAXON et al., EMBO J., Vol. 5, No. 13, p. 3461-3466 (1986).
- M. KOI et al., JPN. J. CANCER RES., Vol. 80, p. 413-418 (1989).

## Description

### TECHNICAL FIELD

The present invention relates to chimeric mice, a method for producing the same and a method for using the same. The present invention allows chimeric mice to retain a foreign giant DNA fragment(s) of at least 1 Mb and to express the gene(s) on such a fragment(s), which was impossible heretofore. Hence, the following becomes possible by using the method.
· Production of mice which retain and express a full length of a gene encoding a biologically active substance, for example, a full length of human antibody gene. The biologically active substance, for example, a human-type antibody is useful as a pharmaceutical product.
· Analysis of functions of human giant genes (e.g., histocompatibility antigen, dystrophin, etc.) in mice.
· Production of model mice with human dominant hereditary disease and a disease due to chromosomal aberration.

### BACKGROUND ART

Techniques of expressing foreign genes in animals, that is, techniques of producing transgenic animals are used not only for obtaining information on the gene's functions in living bodies but also for identifying DNA sequences that regulate the expression of the genes (e.g., Magram et al., Nature, 315:338, 1985), for developing model animals with human diseases (Yamamura et al., "Manual of model mice with diseases" published by Nakayama Shoten, 1994), for breeding farm animals (e.g., Muller et al., Experientia, 47:923, 1991) and for producing useful substances with these animals (e.g., Velander et al., P. N.A.S., 89:12003, 1992). Mice have been used the most frequently as hosts for gene transfer. Since mice have been studied in detail as experimental animals and the embryo manipulating techniques for mice have been established, they are the most appropriate kind of mammals for gene transfer.

Two methods are known for transferring foreign genes into mice. One is by injecting DNA into a pronucleus of a fertilized egg (Gordon et al., P.N.A.S., 77:7380, 1980). The other is by transferring DNA into a pluripotent embryonic stem cell (hereinafter referred to as "ES cell") to produce a chimeric mouse (Takahashi et al., Development, 102:259, 1988). In the latter method, the transferred gene is retained only in ES cell-contributing cells and tissues of chimeric mice whereas it is retained in all cells and tissues of progenies obtained via ES cell-derived germ cells. These techniques have been used to produce a large number of transgenic mice up to now.

However, there had been a limit of the size of DNA capable of being transferred and this restricts the application range of these techniques. The limit depends on the size of DNA which can be cloned. One of the largest DNA fragments which have ever been transferred is a DNA fragment of about 670 kb cloned into a yeast artificial chromosome (YAC) (Jakobovits et al., Nature, 362:255, 1993). This experiment was carried out by fusing a YAC-retaining yeast cell with a mouse ES cell. Although it is believed that foreign DNA of up to about 2 Mb can be cloned on YAC (Den Dunnen et al., Hum. Mol. Genet., 1:19, 1992), the recombination between homologous DNA sequences occurs frequently in budding yeast cells and therefore, in some cases, a human DNA fragment containing a large number of repeated sequences is difficult to retain in a complete form. In fact, certain recombinations occur in 20-40% of the clones of YAC libraries containing human genomic DNA (Green et al., Genomics, 11:584, 1991).

In another method that was attempted, a metaphase chromosome from a cultured human cell was dissected under observation with a microscope and the fragment (presumably having a length of at least 10 Mb) was injected into a mouse fertilized egg (Richa et al., Science, 245:175, 1989). In the resulting mice, a human specific DNA sequence (Alu sequence) was detected but the expression of human gene was not confirmed. In addition, the procedure used in this method to prepare chromosomes causes unavoidable fragmentation of DNA into small fragments due to the use of acetic acid and methanol in fixing the chromosome on slide glass and the possibility that the injected DNA exists as an intact sequence is small.

In any event, no case has been reported to date that demonstrates successful transfer and expression in mice of uninterrupted foreign DNA fragments having a length of at least 1 Mb.

Useful and interesting human genes which are desirably transferred into mice, such as genes for antibody (Cook et al., Nature Genetics, 7: 162, 1994), for T cell receptor (Hood et al., Cold Spring Harbor Symposia on Quantitative Biology, Vol. LVIII, 339, 1993), for histocompatibility antigen (Carrol et al., P.N.A.S, 84:8535, 1987), for dystrophin (Den Dunnen et al., supra) are known to be such that their coding regions have sizes of at least 1 Mb. Since human-type antibodies are important as pharmaceutical products, the production of mice which retain and express full lengths of genes for human immunoglobulin heavy chains (~ 1.5 Mb, Cook et al., supra), and light chain κ (~3 Mb, Zachau, Gene, 135:167, 1993), and light chain λ (~1.5 Mb, Frippiat et al., Hum. Mol. Genet., 4:983, 1995) is desired but this is impossible to achieve by the state-of-the-art technology (Nikkei Biotec, July, 5, 1993).

Many of the causative genes for human dominant hereditary disease and chromosomal aberration which causes congenital deformity (Down's syndrome, etc.) have not been cloned and only the information on the approximate location of the genes on chromosome is available. For example, when a gene of interest is found to be located on a specific G band, which is made visible by subjecting a metaphase chromosome to Giemsa staining, the G band has usually a size of at least several Mb to 10 Mb. In order to transfer these abnormal phenotypes into mice, it is necessary to transfer chromosomal fragments of at least several Mb that surround the causative genes, but this is also impossible with the presently available techniques.

Hence, it is desired to develop a technique by which a foreign DNA longer than the heretofore critical 1 Mb can be transferred into a mouse and expressed in it.

DNA longer than 1 Mb can be transferred into cultured animal cells by the techniques available today. Such transfer is carried out predominantly by using a chromosome as a mediator. In the case of human, chromosomes have sizes of about 50-300 Mb. Some methods for chromosome transfer into cells have been reported (e.g., McBride et al., P.N.A.S., 70:1258, 1973). Among them, microcell fusion (Koi et al., Jpn. J. Cancer Res., 80:413, 1989) is the best method for selective transfer of a desired chromosome. The microcell is a structural body in which one to several chromosomes are encapsulated with a nuclear membrane and a plasma membrane. A few chromosomes (in many cases, one chromosome) can be transferred by inducing a microcell with an agent that inhibits the formation of spindle in a specific kind of cell, separating the microcell and fusing it with a recipient cell. The resulting libraries of monochromosomal hybrid cells containing only one human chromosome have been used for mapping known genes and specifying the chromosomes on which unknown tumor-suppressor genes and cellular senescence genes exist (e.g., Saxon et al., EMBO J., 5:3461, 1986). In addition, it is possible to fragment a chromosome by irradiating a microcell with γ -rays and to transfer part of the fragments (Koi et al. , Science, 260:361, 1993). As described above, microcell fusion is considered to be an appropriate method for transferring DNA of at least 1 Mb into a cultured animal cell.

The expectation that a mouse could be generated from a cultured cell turned to a real fact when the ES cell which has stable pluripotency was discovered (Evans et al., Nature, 292:154, 1981). Foreign genes, various mutations and mutations by targeted gene recombination could be introduced into the ES cell, making it possible to perform a wide variety of genetic modifications in mice (e.g., Mansour et al., Nature, 336:348, 1988). As long as the transfer of giant DNA is concerned, it has been believed that the size of the aforementioned foreign DNA fragment which can be cloned into a YAC vector is the upper limit. The prior art technology of chromosome transfer for introducing a longer DNA into cultured cells has never been applied to gene transfer into mice and this has been believed to be difficult to accomplish (Muramatsu et al., "Transgenic Biology", published by Kodansha Scientific, p.143-, 1989).

The reasons are as follows.
· The transfer of a human chromosome into a mouse ES cell of a normal karyotype as a recipient cell would be a kind of transfer of chromosomal aberration. Up to now, it has been believed that genetic aberration at chromosomal levels which is large enough to be recognizable with microscopes is generally fatal to the embryogeny in mice (Gropp et al., J. Exp. Zool., 228:253, 1983 and Shinichi Aizawa, "Biotechnology Manual Series 8, Gene Targeting", published by Yodosha, 1995).
· Available human chromosomes are usually derived from finitely proliferative normal fibroblasts or differentiated somatic cells such as cancer cells and the like. It was believed that if a chromosome derived from such a somatic cell was transferred into an undifferentiated ES cell, the transferred chromosome might cause differentiation of the ES cell or its senescence (Muller et al., Nature, 311:438, 1984; Sugawara, Science, 247:707, 1990).
· Only few studies have been reported as to whether a somatic cell-derived chromosome introduced into an early embryo can function in the process of embryonic development as normally as a germ cell-derived chromosome to ensure the expression of a specific gene in various kinds of tissues and cells. One of the big differences between the two chromosomes is assumed to concern methylation of the chromosomal DNA. The methylation is changed according to differentiation of cells and its important role in the expression of tissue-specific genes has been suggested (Ceder, Cell, 53:3, 1988). For example, it has been reported that if a methylated DNA substrate is introduced into a B cell, the methylated DNA is maintained after replication and suppresses a site-directed recombination reaction which is essential to the activation of an antibody gene (Hsieh et al., EMBO J., 11:315, 1992). In addition, it was reported that higher levels of de novo methylation occurred in established cell lines than in vivo (Antequera et al., Cell, 62:503, 1990). On the basis of the studies reported, it could not be easily expected that an antibody gene in a human fibroblast or a human-mouse hybrid cell which was likely to be methylated at a high level would be normally expressed in a mouse B cell.

It should be noted that there are two related reports of Illmensee et al. (P.N.A.S., 75:1914, 1978; P.N.A.S., 76:879, 1979). One report is about the production of chimeric mice from fused cells obtained by fusing a human sarcoma cell with a mouse EC cell and the other is about the production of chimeric mice from fused cells obtained by fusing a rat liver cancer cell with a mouse EC cell. Many questions about the results of the experiments in these two reports were pointed out and thus these reports are considered unreliable (Noguchi et al., "Mouse Teratoma", published by Rikogakusha, Section 5, 1987). Although it has been desired to perform a follow-up as early as possible, as of today when 17 years have passed since the publication of these reports, successful reproduction of these experiments has not been reported. Hence, it is believed that foreign chromosomes cannot be retained and the genes on the chromosomes cannot be expressed in mice by the method described in these reports.

Under these circumstances, it has been believed to be difficult to transfer a giant DNA such as a chromosomal fragment and express it in an animal such as mouse. Actually, no study has been made about this problem since the Illmensee's reports.

In addition, Ning et al., Cytogenetics and Cell Genetics 60(1): 79-80, 1992 relates to the isolation of monochromosomal hybrids following fusion of human diploid fibroblast-derived microcells with mouse A9 cells. Green et al., Nature Genetics 7: 13-21, 1994 relates to antigen specific human monoclonal antibodies from mice engineered with human Ig heavy and light chain YACs. Brüggemann et al., P.N.A.S. (USA) 86: 6709-6713, 1989 relates to a repertoire of monoclonal antibodies with human heavy chains from transgenic mice.

Therefore, an object of the present invention is to provide chimeric non-human animals which retain foreign chromosomes or fragments thereof and express genes on the chromosomes or fragments, and their progenies, and a method for producing the same.

It is also an object of the present invention to provide pluripotent cells containing foreign chromosomes or fragments thereof and a method for producing the pluripotent cells.

Another object of the present invention is to provide tissues and cells derived from the chimeric non-human animals and their progenies.

A further object of the present invention is to provide hybridomas prepared by fusing the cells derived from the chimeric non-human animals and their progenies with myeloma cells.

A still further object of the present invention is to provide a method for producing a biologically active substance that is an expression product of the gene on a foreign chromosome or a fragment thereof by using the chimeric non-human animals or their progenies, or their tissues or cells.

### DISCLOSURE OF THE INVENTION

As a result of the various studies conducted to achieve the above objects, the inventors succeeded in transferring chromosomes or fragments thereof derived from human normal fibroblast cells into mouse ES cells and obtaining clones which were capable of stable retention of the chromosomes or fragments. Moreover, they produced from these ES clones those chimeric mice which retained human chromosomes in normal tissues and which expressed several human genes including human antibody heavy-chain genes. It has become possible to make mice that retain and express giant DNA fragments by the series of these techniques, although this has been impossible by conventional techniques.

The subject matter of the invention is as follows.
(1) A method for producing a chimeric mouse, which comprises preparing a microcell containing foreign human chromosome fragment(s) and transferring the foreign human chromosome fragment(s) into a pluripotent cell by fusion with the microcell.
(2) A method for producing a pluripotent cell containing a foreign human chromosome fragment(s), which comprises preparing a microcell containing a foreign human chromosome fragment (s) and transferring the foreign human chromosome fragment(s) into a pluripotent cell by fusion with the microcell.
(3) A chimeric mouse retaining foreign human chromosome fragment(s) and expressing a gene(s) on the foreign human chromosome fragment (s) which can be produced by the method of (1), or its progeny retaining the foreign human chromosome fragment(s) and expressing the gene(s) on the foreign human chromosome fragment (s) thereof.
(4) A pluripotent mouse cell containing foreign human chromosome fragment(s), which can be prepared by the method of (2).
(5) Use of the pluripotent cell of (4) for producing a chimeric mouse.
(6) A mouse which can be produced by mating the chimeric mouse or its progenies of (3), said mouse retaining foreign human chromosome fragment(s) and expressing the gene(s) on the foreign human chromosome fragment(s), or its progeny retaining the foreign human chromosome fragment(s) and expressing the gene(s) on the foreign human chromosome fragment(s).
(7) A tissue from the chimeric mouse or its progeny of (3) or from the mouse or its progeny of (6).
(8) A cell from the chimeric mouse or its progeny of (3) or from the mouse or its progeny of (6).
(9) A hybridoma prepared by the fusion of the cell of (8) with a myeloma cell.
(10) A mouse retaining the foreign human chromosome fragment(s) and expressing a gene(s) on the foreign human chromosome fragment(s), which can be produced by mating the chimeric mouse or its progeny of (3) or the mouse or its progeny of (6), with a mouse in a strain deficient in said gene(s) or a gene homologous thereto, or its progeny retaining the foreign human chromosome fragment(s) and expressing the gene(s) on the foreign human chromosome fragment(s).
(11) A method for producing a biologically active substance, which comprises expressing the gene(s) on foreign human chromosome fragment(s) in the chimeric mouse or its progeny of (3) or the mouse or its progeny of (6), or a tissue or a cell thereof, and recovering the biologically active substance as an expression product.
(12) A method for producing a biologically active substance, which comprises: mating the chimeric mouse or its progeny of (3) or the mouse or its progeny of (6), with a mouse in a strain deficient in the gene(s) or a gene homologous thereto to thereby produce an offspring; expressing the gene(s) on the foreign human chromosome fragment(s) in the offspring, or a tissue or a cell thereof; and recovering the biologically active substance as an expression product.

### BRIEF DESCRIPTION OF THE DRAWINGS

Fig.1 shows the results of PCR analysis of an A9 cell retaining human chromosome #2 (fragment).
Fig.2 shows that human chromosome #22 (fragment) is retained in an E14 drug resistant cell (PCR analysis).
Fig. 3 is a photograph of electrophoresis patterns showing that human L1 sequence is retained in a chimeric mouse produced from a human chromosome #22-transferred ES cell (Southern analysis).
Fig. 4 is a photograph of electrophoresis patterns showing the presence of a human chromosome in organs of a human chromosome #22 transferred chimeric mouse (PCR analysis).
Fig. 5 is a photograph of electrophoresis patterns showing the results of the expression of human genes in a human chromosome #22 transferred chimeric mouse (RT-PCR).
Fig. 6 is a photograph of electrophoresis patterns showing the results of the expression of human genes in organs of a human chromosome #22 transferred chimeric mouse (RT-PCR).
Fig. 7 shows that human chromosome #4 (fragment) is retained in an E14 drug resistant cell (PCR analysis).
Fig. 8 is a photograph of electrophoresis patterns showing the detection of human L1 sequence in a human chromosome #4-transferred E14 cell clone (Southern analysis).
Fig. 9 is a photograph of electrophoresis patterns showing that human L1 sequence is retained in a chimeric mouse produced from a human chromosome #4-transferred ES cell (Southern analysis).
Fig. 10 shows that human chromosome #14 (fragment) is retained in a TT2 drug resistant cell (PCR analysis).
Fig. 11 is a photograph of electrophoresis patterns showing the presence of a human chromosome in organs of a chimeric mouse produced from a human chromosome #14 transferred ES cell (PCR analysis).
Fig. 12 shows the results of a test on a tail-derived fibroblast cell for resistance to G418.
Fig. 13 shows the concentration of human antibody IgM in a serum of a human serum albumin (hereinafter referred to as "HSA")-immunized chimeric mouse (ELISA).
Fig. 14 shows the concentration of human antibody IgG in a serum of an HSA-immunized chimeric mouse (ELISA).
Fig. 15 shows the results of ELISA of hybridoma clone H4B7 capable of producing human IgM.
Fig. 16 is a photograph of the results of FISH analysis of a mouse ES cell clone (TT2 cell clone PG15) retaining partial fragments of human chromosomes #2 and 14.
Fig. 17 shows that the antibody titer of anti-HSA human IgG is increased in a serum of an HSA-immunized chimeric mouse.
Fig. 18 shows that the antibody titer of anti-HSA human Igκ is increased in a serum of an HSA-immunized chimeric mouse.
Fig. 19 is a photograph of electrophoresis patterns showing the detection of human L1 sequence in a human chromosome #22-transferred TT2 cell clone (Southern analysis).
Fig. 20 shows that the antibody titer of anti-HSA human Igλ is increased in a serum of an HSA-immunized chimeric mouse.
Fig. 21 shows that a partial fragment of human chromosome #2 is retained in a progeny of a chimeric mouse into which a partial fragment of a human chromosome #2 was transferred (PCR analysis).
Fig. 22 shows the presence of a cell expressing human *µ* chain on the cell surface in a spleen of a human chromosome #14-transferred chimeric mouse (flow cytometry analysis).
Fig. 23 shows the structure of LoxP-pstNEO plasmid DNA.
Fig. 24 shows the structure of genomic DNA carrying a mouse antibody heavy chain C *µ* gene.
Fig. 25 shows the structure of genomic DNA carrying a mouse antibody light-chain κ gene.
Fig. 26 shows the structures of a mouse antibody heavy-chain targeting vector and a probe for Southern blotting, as well as a DNA fragment to be detected in homologous recombinants.
Fig. 27 shows the structures of a mouse antibody light-chain κ targeting vector and a probe for Southern blotting, as well as a DNA fragment to be detected in homologous recombinants.
Fig. 28 is a photograph of electrophoresis patterns showing the results of Southern blot analysis of mouse antibody heavy-chain homologous recombinants and high concentration G418 resistant clones derived therefrom.

### BEST MODE FOR CARRYING OUT THE INVENTION

The present invention will now be described in detail.

A mouse that retains a human chromosome fragment(s) and which expresses the gene on the chromosome fragment(s) can be produced by
(1) preparing a chromosome donor cell which retains a labeled human chromosome fragment
(2) transferring the human chromosome fragment into a pluripotent mouse cell by microcell fusion;
(3) producing a chimeric mouse from the cell; and
(4) confirming that the human chromosome fragment is retained in the chimeric mouse and that a human gene is expressed.

In this procedure, a mouse is used that retains a human chromosome fragment and which expresses the gene on the chromosome fragment (the mouse is hereinafter referred to as a "human chromosome transferred mouse").

The term "human chromosome" means a naturally occurring complex which consists of nucleic acids and proteins that are derived from human cells. There are 46 normal human chromosomes of 23 kinds (24 kinds in male), each of which contains DNAs of about 50-300 Mb in size. In the present invention, the human chromosome includes not only partial fragments which can be stably replicated and segregated as independent chromosomes but also fragments that are translocated on mouse chromosomes and which are retained stably. The size of the DNA is at least 1 Mb. The feature of the present invention resides in that a mouse can retain and express the foreign gene on a foreign human chromosome as a mediator without treatments such as cloning in an E. coli or yeast cell, or extraction of the DNA from a cell.

The term "human chromosome transferred mouse" means a mouse retaining a human chromosome fragment(s) in all or part of its normal somatic cells. The mouse expresses the gene(s) on a human chromosome fragment(s) in all or part of its normal somatic cells.

### (1) Preparation of a chromosome donor cell which retains a labeled human chromosome or a fragment thereof

A desired chromosome donor cell 1) retains a human chromosome(s) labeled with a marker also available for selection of recipient cells; 2) does not contain other human chromosomes; and 3) has a higher ability to form a microcell.

Any human-derived cell lines, cancer cells and primary culture cells can be used as materials for providing human chromosomes. Among them, normal fibroblast cells are suitable because they have a low possibility of abnormality such as deletion and amplification of chromosomes and can be readily cultured.

As for 1), human cells can be transformed with vectors that express genes for markers such as drug-resistance (e.g., G418-, puromycin-, hygromycin- or blasticidin-resistance). Promoters operating efficiently not only in human cells but also in recipient cells such as mouse ES cells are desirably used to regulate the expression of the marker used. For this purpose, herpes simplex virus thymidine kinase promoter linked with SV 40 enhancer (Katoh et al., Cell Struct. Funct., 12:575, 1987), mouse PGK-1 promoter (Soriano et al., Cell, 64:693, 1991) and the like can be used. A library of human cell transformants in which the introduced marker genes have been inserted into 46 human chromosomes of 23 kinds at random can be prepared by transformation through electroporation (Ishida et al., "Cell Technology Experiment Manual", published by Kodansha, 1992) and the like and subsequent selection of transformants.

As for 3), since many human normal cells have a very low ability to form microcells, the whole cell of the transformant may be fused with a cell having a high ability to form microcells such as mouse A9 cell (Oshimura, M., Environ. Health Perspect., 93:57, 1991) so as to provide the transformed cell with an ability to form microcells. It is known that in mouse-human hybrid cells, human chromosomes selectively disappear. The fused cell selected by the marker can retain stably the marked human chromosome.

In order to meet the condition of 2), it is desired to obtain a microcell from the fused cell and fuse it again with a mouse A9 cell. In this case, too, most of the cells selected by the marker will meet the three conditions 1), 2) and 3) above. The marked human chromosome fragments can be identified in the finally obtained mouse-human monochromosomal hybrid cells by PCR (Polymerase Chain Reaction, Saiki et al., Science, 239:487, 1988), Southern blot analysis (Ausubel et al., Current protocols in molecular biology, John Wiley & Sons, Inc., 1994), FISH analysis (Fluorescence In Situ Hybridization, Lawrence et al., Cell, 52: 51, 1988) and the like. If the transfer of a specified chromosome is desired, the above procedures are applied to each of many human cell transformant clones to select a clone in which a chromosome of interest is marked. Alternatively, the above procedures are applied to a mixture of human cell transformant clones and the identification of human chromosome fragments is carried out on a large number of the resulting mouse-human monochromosome hybrid cells.

In addition, a marker gene can be inserted into a desired site by homologous recombination of a specific DNA sequence on the chromosome which is to be transferred (Thomas et al., Cell, 51:503, 1987).

A microcell prepared from the mouse-human hybrid cell may be irradiated with γ -rays such that the marked human chromosome is fragmented and transferred into a mouse A9 cell. Even if the microcell is not irradiated with γ -rays, a partially fragmented human chromosome may be transferred at a certain frequency. In these cases, the resulting microcell fused clones retain partial fragments of the marked human chromosomes. These clones can be used when it is desired to transfer the partial fragments into recipient cells.

### (2) Transfer of the human chromosome or fragment thereof into a mouse pluripotent cell

It has been reported to date that an embyonic carcinoma cell (EC cell, Hanaoka et al., Differentiation, 48:83, 1991), an embyonic stem cell (ES cell, Evans, Nature, 292:154, 1981) or an embyonic germ cell (EG cell, Matsui et al., Cell, 70:841, 1992) that are derived from various strains of mice contribute to the normal somatic cells in mice, or are capable of the production of chimeric mice, by injection into or coculturing with a mouse early embryo. ES and EG cells have a very high ability in this respect and in many cases, they also contribute to germ cells thereby making it possible to produce progenies derived from the cells. EC cells can be obtained predominantly from teratocarcinoma; ES cells from the inner cell masses of blastocysts; and EG cells from primordial germ cells appearing at the early stage of embryogeny. These cell lines and their mutants, and any undifferentiated cells that are capable of differentiation into all or part of the normal somatic cells in mice can be used as recipient cells for the transfer of human chromosome in the present invention.

The microcells prepared from the human chromosome donor cells or the microcells irradiated with γ -rays can be used as materials for the transfer of human chromosomes into the recipient cells. The human chromosome can be transferred into the recipient cell through fusion of the recipient cell with the microcell by the method described in Motoyuki Shimizu, "Cell Technology Handbook", published by Yodosha, 1992. The microcell donor cells retain markers by which human chromosome fragments can be selected in the recipient cells. The clone containing a gene, a chromosome or a fragment of interest can be selected by PCR, Southern blot analysis, FISH method or the like in the same manner as in (1), thus all kinds of human chromosomes or fragments thereof can be transferred. Moreover, if several chromosomes or fragments thereof which contain different selection markers are transferred sequentially, a recipient cell retaining chromosome fragments can be obtained at the same time. In addition, clones having an increased number of the transferred chromosome fragment can be selected from the clones into which the human chromosome fragment has been transferred. Such selection can be accomplished by increasing the concentration of a selection drug to be added to a culture medium.

In order to determine whether the recipient cell selected by the marker (e.g., G418 resistance) on the human chromosome retains the whole or part of the chromosome retained by the donor cell, the following confirmative techniques may be employed: Southern blot analysis using the genomic DNA extracted from the selected recipient cell, with a human specific repeated sequence (L1, Alu, etc.: Korenberg et al., Cell, 53:391, 1988) or a human gene used as a probe; and chromosome analysis such as PCR method using a human gene specific primer or FISH method using a human chromosome specific probe (Lichter et al., Human Genetics, 80:224, 1988).

### (3) Production of a chimeric mouse from the human chromosome transferred ES cell

The method described in Shinichi Aizawa, "Biotechnology Manual Series 8, Gene Targeting", published by Yodosha, 1995 may be used to produce chimeric mice from the ES cell clone obtained in (2). In selecting factors for efficient production of chimeric mice, such as the developmental stage of the host embryo and its strain, it is desired to employ the conditions already reviewed for the respective ES cell clones. For example, 8 cell stage embryos derived from Balb/c (albino, CREA JAPAN, INC.) or ICR (albino, CREA JAPAN, INC.) are desirably used for CBAxC57BL/6 F1-derived TT2 cell (agouti, Yagi et al., Analytical Biochemistry, 214:70, 1993).

### (4) Confirmation of the retention of the human chromosome fragment in the chimeric mice and the expression of a human gene

The contribution of the ES cells in mice produced from the embryos into which ES cells were injected can be roughly judged by the color of their coat. However, it should be noted that the total absence of contribution to the coat color does not always lead to the conclusion that there is no contribution to other tissues. The detailed information on the retention of the human chromosome fragment in various tissues of the chimeric mice can be obtained by Southern blot analysis using the genomic DNA extracted from various tissues, by PCR or the like.

The expression of the gene on the transferred human chromosome fragment can be confirmed by the following methods. The expression of mRNA transcribed from the human chromosome fragment can be detected by RT-PCR method or northern blotting (Ausubel et al., supra) using RNAs derived from various tissues (Kawasaki et al., P.N.A.S., 85:5698, 1988). The expression at the protein level can be detected by enzyme immunoassay using an anti-human protein antibody that is rendered minimal in its ability to enter into a cross reaction with mouse homologous proteins (ELISA, Toyama and Ando, "Monoclonal Antibody Experiment Manual", published by Kodansha Scientific, 1987; Ishikawa, "Enzyme immunoassay with Superhigh Sensitivity", published by Gakkai Shuppan Center, 1993), western blotting (Ausuel et al., supra), isozyme analysis utilizing the difference in electrophoretic mobility (Koi et al., Jpn. J. Cancer Res., 80:413, 1989) or the like. The retention of the human chromosome fragment in the chimeric mice and the expression of the gene on the human chromosome fragment can be confirmed by the appearance of the cells expressing a drug resistance marker gene in primary culture cells derived from the chimeric mice.

For example, human IgM, IgG, IgA and the like in sera of the chimeric mice which are produced from ES cells retaining human chromosome #14 fragment on which a gene for human immunoglobulin heavy chain exists can be detected by enzyme immunoassay using an anti-human Ig antibody that is rendered minimal in its ability to enter into cross reaction with mouse antibody. Hybridomas capable of producing a human immonoglobulin heavy chain can be obtained by ELISA screening of hybridomas prepared by immunizing the chimeric mouse with a human-derived antigen (e.g., HSA) and fusing the spleen cells of the immunized mice with mouse myeloma cells (Toyama and Ando, "Monoclonal Antibody Experiment Manual", published by Kodansha Scientific, 1987). pluripotent cell and, subsequently, the gene on which (or a fragment thereof) is expressed in a chimeric mouse.

In the present invention, pluripotent cells into which a foreign human chromosome fragment (s) are transferred are not limited to the ES cells, EC cells and EG cells mentioned above. For example, it is possible to transfer foreign human chromosome fragment(s) into bone marrow stem cells. If these bone marrow stem cells are transplanted into a living organism, hereditary diseases, etc. may be treated.

If an ES cell retaining foreign human chromosome fragment(s) is differentiated to a germ cell in the chimeric mouse, reproduced progenies will retain the transferred chromosome fragment(s) and express the gene(s) on the chromosome fragment(s).

The chimeric mice or their progenies can be used to express the gene on the foreign human chromosome fragment and to recover the expression product, thereby producing a biologically active substance. More specifically, the chimeric mice or their progenies can be bred under the conditions for expressing the gene on the foreign human chromosome fragment to recover the expression product from the blood, ascites and the like of the animals. Alternatively, the tissues or cells of the chimeric mice, or immortalized cells derived therefrom (e.g., hybridomas immortalized by fusion with myeloma cells) can be cultured under the conditions for expressing the gene on the foreign human chromosome fragment and the expression product is thereafter recovered from the culture. Furthermore, a foreign human chromosome fragment(s) which was extracted from tissues or cells of these chimeric mice or their progenies or from immortalized cells derived therefrom; the DNA which is a component of said foreign human chromosome fragment(s); or cDNA derived from the foreign human chromosome fragment(s) thereof retained in tissues or cells of the chimeric mice or their progenies, or in immortalized cells derived therefrom may be used to transform animal cells or insect cells (e.g., CHO cells, BHK cells, hepatoma cells, myeloma cells, SF9 cells) and the transformed cells may be cultured under the conditions for expressing the gene on the foreign human chromosome fragment(s) thereof to recover the expression product (e.g., an antibody protein specific to a particular antigen) from the culture. The expression product can be collected by known techniques such as centrifugation and purified by known techniques such as ammonium sulfate fractionation, partition chromatography, gel filtration chromatography, adsorption chromatography, preparative thin-layer chromatography and the like. The biologically active substance includes any kinds of substances encoded on foreign human chromosomes, for example, human antibodies. For example, the human antibody gene on the foreign human chromosome fragment can be cloned from spleen cells of the chimeric mouse or immortalized cells such as hybridomas derived therefrom and transferred into Chinese hamster ovary cells (CHO), myeloma cells or the like to produce a human antibody (Lynette et al., Biotechnology, 10:1121-, 1992; Bebbington et al., Biotechnology, 10:169-, 1992).

The chimeric mice or their progenies that retain fragments of human chromosomes #2, 14 and/or 22 which can be produced by the method of the present invention can retain the greater part of the functional sequences of respective genes for human antibody heavy chain on chromosome #14, light chain κ on chromosome #2 and light chain λ on chromosome #22. Hence, they can produce a wide repertory of antibodies which are more similar to human antibody repertory, compared with known transgenic mice into which parts of human antibody gene have been transferred by using yeast artificial chromosomes and the like (Green et al., Nature Genetics, 7, 13-, 1994; Lonberg et al., Nature, 368, 856-, 1994). Also, the chimeric mice and their progenies retaining two human chromosome fragments of #2+#14, #22+#14 or other combination and the mice and their progenies retaining three human chromosome fragments of #2+#14+#22 or other combination which are obtainable by mating said chimeric mice and their progenies retaining two human chromosome fragments, as produced by the method of the invention, can produce complete human antibodies both heavy- and light-chains of which are derived from human. These mice can recognize human-derived antigens as foreign substances to cause an immunoreaction with the antigens, thereby producing antigen-specific human antibodies. These properties can be utilized to produce human monoclonal and polyclonal antibodies for therapeutic treatments (Green et al, supra; Longberg et al., supra). On the other hand, in order to obtain a human antibody having high affinity for a particular antigen more efficiently, it is desirable to produce a mouse which produces a human antibody but not a mouse antibody (Green et al., supra; Lonberg et al., supra). In the present invention, this is achieved typically by the following Method A or B using known techniques.
Method A: a method using a mouse antibody-deficient ES cell and a mouse antibody-deficient host embryo for chimera production.
Method B: a method in which a progeny retaining a human chromosome fragment is obtained from a human chromosome fragment-transferred chimeric mouse, followed by mating said progeny with a mouse in a strain deficient in a mouse antibody gene.

A typical example for each of Methods A and B will be described below specifically.

### Specific procedures for Method A

1. One of the mouse antibody heavy-chain genes present in two copies in a mouse ES cell is disrupted by homologous recombination in gene targeting (Joyner et al., "Gene Targeting", published by IRL PRESS, 1993). A marker gene, such as a G 418 resistance gene, sandwiched with two copies of a sequence which can be removed later by site-specific recombination [for example, loxP sequence (see recombination with Cre recombinase in Sauer et al., supra; and see also the use of FLP recombinase-FRT sequence in O'Gorman, Science, 251;1351-, 1991)] is inserted at the site where the targeted gene is disrupted.
2. The resultant drug-resistant mouse ES cells in which one antibody heavy-chain gene was disrupted is cultured in the presence of the drug at a high concentration. Then, those clones which became high concentration drug-resistant are selected. By screening these clones, clones in which both antibody heavy-chain genes were disrupted can be obtained (Shinichi Aizawa, supra).
3. An enzyme gene (e.g., a Cre recombinase gene (Sauer et al., supra)) which causes a site-specific recombination between the recombination sequences inserted at both the ends of the drug-resistance gene in step 1 above is transiently transferred into the mouse ES cells from step 2 above in which both antibody heavy-chain genes were disrupted. Then, drug-sensitive clones are selected in which the drug-resistance genes inserted at the sites of both heavy-chain genes were deleted as a result of recombination between the IoxP sequences [Seiji Takatsu et al., " Experimental Medicine (extra number): Basic Technologies in Immunological Researches", p. 255-, published by Yodosha, 1995].
4. The same procedures in steps 1-3 above are repeated for the mouse antibody light-chain κ gene to finally obtain drug-sensitive clones which are completely deficient in antibody heavy-chain and light-chain κ.
5. Human chromosome #14 fragment containing a human antibody heavy-chain gene and marked with a drug-resistance gene (e.g., G418 resistance gene) is transferred into the clone from step 4 above (antibody heavy-chain and light-chain κ -deficient mouse ES cell) by microcell fusion.
6. Human chromosome #2 fragment or #22 fragment or both containing a human antibody light-chain gene(s) and marked with a drug-resistance gene different from the one used in step 5 above (e.g., puromycin resistance gene) are transferred into the clone obtained in step 5 above by microcell fusion.
7. Chimeric mice are produced from the ES cells obtained in step 6 above by using embryos obtained from a mouse in a strain having no ability to produce its own antibody (e.g., RAG-2 knockout mouse, Shinkai et al., Cell, 68:855-, 1992; membrane-type µ chain knockout mouse, Kitamura et al., Nature, 350:423-, 1991) as host embryos.
8. Most of the functional B lymphocytes in the resultant chimeric mice are derived from the ES cells [Seiji Takatsu et al., "Experimental Medicine (extra number): Basic Technologies in Immunological Researches", p. 234-, published by Yodosha, 1995]. Since those B lymphocytes are deficient in mouse heavy-chain and light-chain κ, they produce human antibodies alone mainly as a result of the expression of the functional human antibody genes on the transferred chromosome fragments.

### Specific procedures for Method B

1. Chimeric mice retaining a human chromosome fragment containing human antibody heavy-chain, light-chain κ or light-chain λ are used to produce a progeny which stably retains the human chromosome fragment and which can transmit it to the next generation.
2. A mouse in a strain which is homozygous regarding the deficiency in mouse antibody heavy-chain and light-chain κ and which retains human chromosome fragments containing human antibody heavy-chain (#14) + light-chain κ (#2), heavy-chain (#14) + light-chain λ (#22) or heavy-chain (#14) + light-chain κ (#2) + light-chain λ (#22) is obtained by mating the mouse in a strain expressing human antibody heavy-chain or light-chain from step 1 above or a mouse in a strain expressing both human antibody heavy and light-chains obtained by mating the mice from step 1, with a mouse in a strain deficient in its own antibody genes (e.g., the membrane-type *µ* chain knockout mouse mentioned above; light-chain κ knockout mouse, Chen et al., EMBO J., 3:821-, 1993). Since mice in the resultant strain are deficient in mouse antibody heavy-chain and light-chain κ genes, they produce human antibodies alone mainly as a result of the expression of the functional human antibody genes on the transferred chromosome fragments.

Both Method A and Method B may be used not only to yield human antibodies but also to yield products of any genes located on a foreign human chromosome fragment efficiently.

The present invention will now be explained in greater detail with reference to the following examples, which do not limit the scope of the present invention.

### Example 1

### Production of chromosome donor cell retaining human chromosome (fragment) labeled with G418 resistance

Plasmid pSTneoB containing a G418 resistance gene (Katoh et al., Cell Struct. Funct., 12:575, 1987; Japanese Collection of Research Biologicals (JCRB), Deposit Number: VE 039) was linearized with restriction enzyme SalI (TAKARA SHUZO CO., LTD.) and introduced into human normal fibroblast cell HFL-1 (obtained from RIKEN Cell Bank, RCB0251). The HFL-1 cells were treated with trypsin and suspended in Dulbecco's phosphate-buffered saline (PBS) at a concentration of 5x10⁶ cells/ml, followed by electroporation using a Gene Pulser (Bio-Rad Laboratories, Inc.) in the presence of 10*µ*g of DNA (Ishida et al., "Cell Technology Experiment Procedure Manual", published by Kodansha, 1992). A voltage of 1000 V was applied at a capacitance of 25 *µ*F with an Electroporation Cell of 4 mm in length (165-2088, Bio-Rad Laboratories, Inc.) at room temperature. The electroporated cells were inoculated into an Eagle's F12 medium (hereinafter referred to as "F12") supplemented with 15% fetal bovine serum (FBS) in 3-6 tissue culture plastic plates (Corning) of 100 mmφ. After one day, the medium was replaced with a F12 supplemented with 15% FBS and containing 200 µ g/ml of G418 (GENENTICIN, Sigma). The colonies formed after 2-3 weeks were collected in 52 groups each consisting of about 100 colonies. The colonies of each group were inoculated again into a plate of 100 mmφ and cultured.

Mouse A9 cells (Oshimura, Environ. Health Perspect., 93:57, 1991; JCRB 0211) were cultured in Dulbecco's modified Eagle's medium (hereinafter referred to as "DMEM") supplemented with 10% FBS in plates of 100 mm φ. The G418 resistant HFL-1 cells of 52 groups were cultured in F12 supplemented with 15% FBS and 200 µg/ml of G418 in plates of 100 mmφ. The mouse A9 cells and HFL-1 cells were treated with trypsin and one fourth to one half of both cells were mixed. The mixed cells were inoculated into a plate of 100 mmφ and cultured in a mixture of equal amounts of DMEM containing 10% FBS and F12 containing 15% FBS for a period ranging from a half day to one day. Cell fusion was carried out in accordance with the method described in Shimizu et al., "Cell Technology Handbook", published by Yodosha, p.127-, 1992. The cell surface was washed twice with DMEM and then treated sequentially with 2 ml of a PEG (1:1.4) solution for 1 minute and with 2 ml of PEG (1:3) for 1 minute. After the PEG solution was sucked up, and the cells were washed three times with a serum-free DMEM, followed by cultivation in DMEM supplemented with 10% FBS for 1 day. The cells were dispersed by treatment with trypsin and suspended in a double selective medium (10% FBS supplemented DMEM) containing ouabain (1x10⁻⁵ M, Sigma) and G418 (800µ g/ml), followed by inoculation in 3 plates of 100 mm φ. After about 3 weeks cultivation, the colonies formed were treated with trypsin to disperse the cells, which were cultured in a selective medium (10% FBS supplemented DMEM) containing G418 (800 *µ*g/ml).

The cells were dispersed by treatment with trypsin and two groups of the cells were collected, followed by cultivation in 6 centrifuge flasks (Coaster, 3025) of 25 cm² until the cell density reached 70-80% confluence. The medium was replaced with a medium (20% FBS supplemented DMEM) containing Colcemid (0.05 *µ*g/ml, Demecolcine, Wako Pure Chemicals Co., Ltd) and the cells were cultured for 2 days to form microcells. After the culture medium was removed, a cytochalasin B (10 µ g/ml, Sigma) solution preliminarily warmed at 37°C was filled in the 25 cm² centrifuge flask, which were inserted into an acryl centrifuge container, followed by centrifugation at 34°C at 8,000 rpm for 1 hour. The microcells were suspended in a serum-free medium and purified by passage through a filter. To the mouse A9 cells cultured to 80% confluence in the flask of 25 cm², the purified micorcells were added and the two kinds of cells were fused with a PEG solution. The fused cells were cultured in a G418 containing selective medium and colonies formed were isolated. Human chromosomes #2, 4, 14 and 22 retained in the respective clones were identified by the methods described in (1)-(3) below. All other experimental conditions such as operating procedures and reagents were in accordance with Shimizu et al., "Cell Technology Handbook", published by Yodosha, p127-.

### (1) PCR analysis

The isolated cells were cultured and genomic DNA was extracted from the cells with a Puregene DNA Isolation kit (Gentra System Co.). PCR was performed using the genomic DNA as a template with human chromosome specific primers to select the clones retaining human chromosome #2, 4, 14 or 22. The PCR amplification was conducted with about 0.1 *µ* g of the genomic DNA as a template, using a thermal cycler (GeneAmp 9600, Perkin-Elmer Corp.) in accordance with the method described in Innis et al., "PCR Experiment Manual", published by HBJ Publication Office, 1991. Taq polymerase was purchased from Perkin-Elmer Corp. and the reaction was performed in a cycle of 94°C, 5 minutes and 35 cycles of denaturing at 94 °C, 15 seconds, annealing at 54-57°C, 15 seconds (variable with the primers) and extension at 72°C, 20 seconds. The gene on each chromosome (O'Brien, Genetic Maps, 6th edition, Book 5, Cold Spring Harbor Laboratory Press, 1993) and polymorphic markers (Polymorphic STS Primer Pair, BIOS Laboratories, Inc.; Weissenbach et al., Nature 359:794, 1992; Walter et al., Nature Genetics, 7:22, 1994) were used as primers. The primers for the genes were prepared on the basis of nucleotide sequences obtained from data bases such as GenBank, EMBL and the like. The names of the polymorphic primers and the sequences of the primers for the genes will be shown for the respective chromosomes in the following examples (#2, Example 1; #4, Example 6, #14, Example 9; #22, Example 2). The following genetic markers and polymorphic makers (Polymorphic STS Primer Pairs: D2S207, D2S177, D2S156 and D2S159, BIOS Laboratories, Inc.) were used to identify chromosome #2.
Cκ (immunoglobulin kappa constant) : 5'-TGGAAGGTGGATAACGCCCT (SEQ ID NO:1), 5'-TCATTCTCCTCCAACATTAGCA (SEQ ID NO:2)
FABP1 (fatty acid binding protein-1 liver) : 5'-GCAATCGGTCTGCCGGAAGA (SEQ ID NO:3), 5'-TTGGATCACTTTGGACCCAG (SEQ ID NO:4)
Vk3-2 (immunoglobulin kappa variable) : 5'-CTCTCCTGCAGGGCCAGTCA (SEQ ID NO:5), 5'-TGCTGATGGTGAGAGTGAACTC (SEQ ID NO:6)
Vk1-2 (immunoglobulin kappa variable) : 5'-AGTCAGGGCATTAGCAGTGC (SEQ ID NO:7), 5'-GCTGCTGATGGTGAGAGTGA (SEQ ID NO:8)

### (2) Fluorescence in situ hybridization (FISH)

FISH analysis was conducted with probes specific to human chromosomes #2, 4, 14 and 22 (CHROMOSOME PAINTING SYSTEM, Cambio Ltd.) in accordance with the method described in Matsubara et al., "FISH Experiment Protocol", published by Shujunsha, 1994.

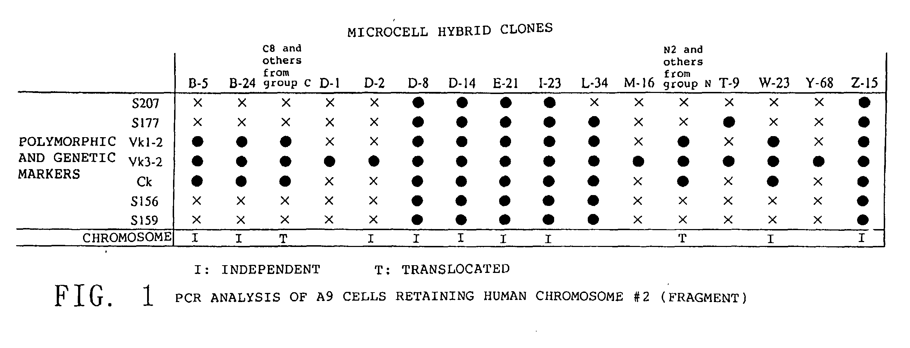

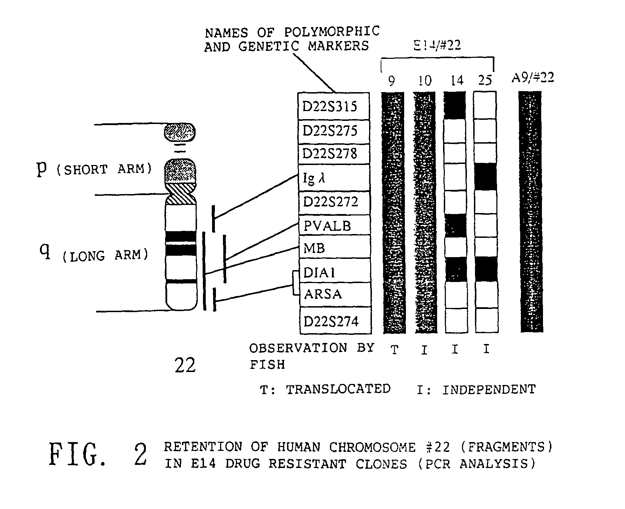

For example, at least one clone retaining chromosome #2 was obtained in 10 groups out of 26 groups (745 clones). Among them, only 5 clones were positive to all the used primers specific to chromosome #2. FISH analysis was conducted with these clones. FISH analysis was conducted with probes specific to human chromosomes #2 (CHROMOSOME PAINTING SYSTEM, Cambio Ltd.) in accordance with the method described in Matsubara et al., "FISH Experiment Protocol", published by Shujunsha, 1994. In the cells positive to all the primers, an intact form of human chromosome #2 was observed. In some of the clones positive to part of the primers, an independent chromosome smaller than human chromosome #2 was observed or a cell having a chromosome in a form fusing with chromosomes other than human chromosome #2 was observed (Fig. 1). In Fig. 1, the names of the clones are shown in the horizontal line and the primers used in the PCR are shown in the left longitudinal line. ● shows positive clones and × shows negative clones. The forms of human chromosome #2 observed by FISH are shown in the bottom line. No description means no performance of experiment.

A9 cells retaining human chromosomes #4, 14 and 22 were obtained by the same procedure.

### Example 2

### Transfer of human chromosome #22 into mouse ES cells by microcell fusion

The mouse A9 cell clones retaining human chromosome #22 (hereinafter referred to as "A9/#22") from Example 1 were used as chromosome donor cells. Mouse ES cell line E14 (obtained from Martin L. Hooper; Hooper et al., Nature, 326:292, 1987) was used as a chromosome recipient cell. E14 cells were cultured in accordance with the method described in Aizawa Shinichi, "Biomanual Series 8, Gene Targeting", published by Yodosha, 1995 and G418 resistant STO cell line (obtained from Prof. Kondo Hisato, Osaka University) treated with mitomycin C (Sigma) was used as a feeder cell. In the first step, microcells were prepared from about 10⁸ cells of A9/#22 in accordance with the method reported by Shimizu et al. "Cell Technology Handbook", published by Yodosha, 1992. The total amount of the resulting microcells were suspended in 5 ml of DMEM. About 10' cells of E14 were dispersed with trypsin and washed three times with DMEM and suspended in 5 ml of DMEM. The cells were then mixed with the microcells and the mixture was centrifuged at 1,250 rpm for 10 minutes to remove the supernatant. The precipitate was dispersed by tapping and 0.5 ml of a PEG solution (1: 1.4) [5 g of PEG 1000 (Wako Pure Chemicals Co., Ltd.) and 1 ml of DMSO (Sigma) as dissolved in 6 ml of DMEM] was added. The mixture was left to stand at room temperature for 1 minute and 30 seconds and 10 ml of DMEM was added slowly. Immediately thereafter, the resulting mixture was centrifuged at 1,250 rpm for 10 minutes to remove the supernatant. The precipitate was suspended in 30 ml of a medium for ES cells and inoculated into 3 tissue culture plastic plates (Corning) of 100 mm in diameter into which feeder cells were inoculated. After 24 hours, the medium was replaced with a medium supplemented with 300 *µ*g/ml of G418 (GENETICIN, Sigma) and medium replacements were thereafter conducted daily. Drug resistant colonies appeared in 1 week to 10 days. The frequency of appearance was 0-5 per 10⁷ of E14 cells. The colonies were picked up and grown. The cells were suspended in a storage medium (a medium for ES cells + 10% DMSO (Sigma)) at a concentration of 5 x 10⁶ cells per ml and stored frozen at -80°C . At the same time, genomic DNA was prepared from 10⁶-10⁷ cells of each drug resistant clone with a Puregene DNA Isolation Kit (Gentra System Co.).

Human chromosome #22 was fragmented by irradiating the microcells with γ rays (Koi et al., Science, 260:361, 1993). The microcells obtained from about 10⁸ cells of A9/#22 were suspended in 5 ml of DMEM and irradiated with γ rays of 60 Gy on ice with a Gammacell 40 (Canadian Atomic Energy Public Corporation) at 1.2 Gy/min for 50 minutes. The fusion of γ ray-irradiated microcells and the selection of drug resistant clones were conducted by the same procedure as in the case of the unirradiated microcells. As a result, the frequency of the appearance of the drug resistant clones was 1-7 per 10⁷ of E14 cells. The drug resistant clones were stored frozen and DNA was prepared from the clones by the same procedure as in the case of the unirradiated microcells.

The retention of the transferred chromosomes in the unirradiated microcell-transferred drug resistant clones E14/#22-9 and E14/#22-10, and in the γ ray-irradiated microcell-transferred drug resistant clones E14/#22-14 and E14/#22-25 was confirmed by the methods described in (1)-(3) below.

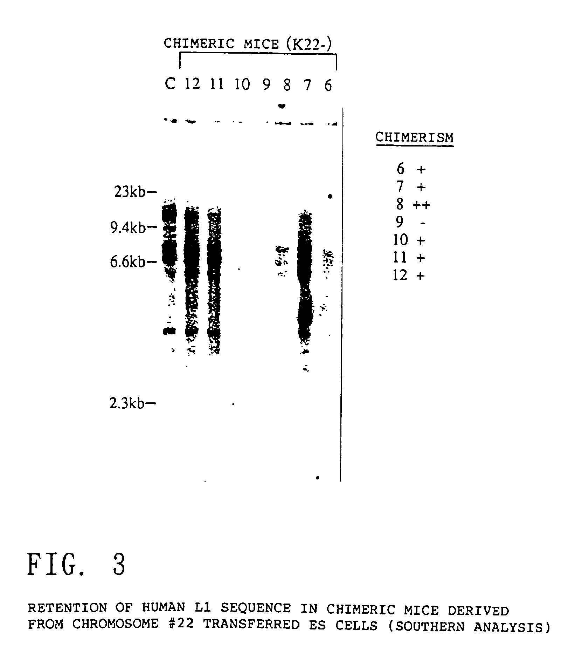

### (1) PCR analysis (Fig. 2)

The presence of the gene on human chromosome #22 (Genetic Maps, supra) and polymorphic markers (Polymorphic STS Primer Pairs: D22S315, D22S275, D22S278, D22S272 and D22S274, BIOS Laboratories, Inc.; Nature 359:794, 1992) was detected by a PCR method using the genomic DNA of the drug resistant clone as a template. The sequences of oligonucleotide primers for the genes prepared on the basis of nucleotide sequences obtained from data bases such as GenBank, EMBL and the like are described below.
PVALB (parvalbumin) : 5'-TGGTGGCTGAAAGCTAAGAA (SEQ ID NO:9), 5'-CCAGAAGAATGGTGTCATTA (SEQ ID NO:10)
MB (myoglobin) : 5'-TCCAGGTTCTGCAGAGCAAG (SEQ ID NO:11), 5'-TGTAGTTGGAGGCCATGTCC (SEQ ID NO:12)
DIA1 (cytochrome b-5 reductase) : 5'-CCCCACCCATGATCCAGTAC (SEQ ID NO:13) 5'-GCCCTCAGAAGACGAAGCAG (SEQ ID NO:14)
Igλ (immunoglobulin lambda) : 5'-GAGAGTTGCAGAAGGGGTGACT (SEQ ID NO:15), 5'-GGAGACCACCAAACCCTCCAAA (SEQ ID NO:16)
ARSA (arylsulfatase A) : 5'-GGCTATGGGGACCTGGGCTG (SEQ ID NO:17), 5'-CAGAGACACAGGCACGTAGAAG (SEQ ID NO:18)

PCR amplification (Innis et al., supra) was conducted by using about 0.1 *µ* g of the genomic DNA as a template with the above 10 kinds of the primers. As a result, amplification products having expected lengths were detected with all the primers in the case of the two unirradiated clones and with part of the primers in the case of the γ ray-irradiated two clones. The results are shown in Fig. 2. In Fig. 2, a schematic chromosome map based on the G bands of human chrosome #22 and the location of some markers on bands are shown at the left side (O'Brien, GENETIC MAPS, 6th edition, BOOK 5, etc.). The arrangement of the genetic and polymorphic markers shows approximate positional relationships on the basis of the presently available information (Science, HUMAN GENETIC MAP, 1994; Nature Genetics, 7:22, 1994; Nature 359:794, 1992, etc.) and the order is not necessarily correct. With respect to four kinds of the G418 resistant E14 cell clones, the markers for which the expected amplification products were detected by PCR are shown by ■ and the markers for which the expected amplification products were not detected are shown by □. The results of the observation by FISH analysis are shown at the bottom side. A9/#22 is a chromosome donor cell.

### (2) Southern blot analysis

Southern blot analysis of about 2*µ*g of the genomic DNA digested with restriction enzyme BglII (TAKARA SHUZO CO., LTD.) was conducted by using human specific repeated sequence L1 (10⁴-10⁵ copies were present per haploid genome, obtained from RIKEN DNA Bank; Nucleic acids research, 13;7813, 1985; pUK19A derived EcoRI-BamHI fragment of 1.4 kb) as a probe in accordance with the method described in Ausubel et al., Current Protocols in Molecular Biology, John Wiley & Sons, Inc., 1994. As a result, a large number of bands hybridized with the human L1 sequence were detected in DNA of each drug resistant clone. With respect to the unirradiated 2 clones, their patterns and the quantitative ratio of human chromosomal DNA to mouse genomic DNA which could be presumed from the density of the respective bands were the same as those of A9/#22. The total signal intensity of the bands of the γ -ray irradiated clones correlated with the degree of the deletion confirmed by the PCR analysis, as compared with that of A9/#22.

### (3) Fluorescence in situ hybridization (FISH)

FISH analysis was conducted with probes specific to human chromosomes #22 (CHROMOSOME PAINTING SYSTEM, Cambio Ltd.) in accordance with the method described in Matsubara et al., "FISH Experiment Protocol", published by Shujunsha, 1994. As a result, in almost all of the observed metaphase spreads, human chromosome #22 was detected in the form of translocation to the mouse chromosome with respect to E14/#22-9 and in the form of an independent chromosome with respect to the three other clones.

The results of the above experiments demonstrate that the obtained G418 resistant clones E14/#22-9 and E14/#22-10 retained all or most part of human chromosome #22 whereas the clones E14/#22-14 and E14/#22-25 retained partial fragments of human chromosome #22.

### Example 3

### Production of chimeric mice from the ES cells retaining human chromosome #22 fragment

General procedures for obtaining mouse embryos, cultivation, injection of the ES cells into the embryos, transplantation to the uteri of foster mothers were carried out in accordance with the method described in Aizawa Shinichi, "Biomanual Series 8, Gene Targeting", published by Yodosha, 1995. The cells in a frozen stock of the G418 resistant ES clone E14/#22-9 which was confirmed to retain human chromosome #22 were thawed, started to culture and injected into blastcyst-stage embryos obtained by mating a C57BL/6XC3H F1 female mouse (CREA JAPAN, INC.) with a C3H male mouse (CREA JAPAN, INC.); the injection rate was 10-15 cells per embryo. Two and half days after a foster mother [ICR or MCH(ICR)] mouse (CREA JAPAN, INC) was subjected to a pseudopregnant treatment, about ten of the ES cell-injected embryos were transplanted to each side of the uterus of the foster mother. The results are shown in Table 1.

**Table 1. Production of chimeric mice from the ES cells retaining human chromosome #22 fragments**

| ES cell clone/human chromosome | G418 resistant clone No. | Number of ES cell-injected blastocyst stage embryos | Number of offspring mice | Number of chimeric mice | Contribution to coat color | | |
|---|---|---|---|---|---|---|---|
| | | | | | <-10% | 10-30% | 30%< |
| E14/#22 | 9 | 166 | 29 | 16 | 7 | 3 | 6 |

As a result of the transplantation of a total 166 of injected embryos, 29 offspring mice were born. Chimerism in the offsprings can be determined by the extent of E14 cell-derived pale gray coat color in the host embryo-derived agouti coat color (dark brown). Out of the 29 offsprings, 16 mice were recognized to have partial pale gray coat color, indicating the contribution of the E14 cells. The maximum contribution was about 40% in K22-22.

These results show that the mouse ES cell clone E14/#22-9 retaining a human chromosome #22 fragment maintains the ability to produce chimera, that is, the ability to differentiate into normal tissues of mouse.

### Example 4

### Confirmation of retention of human chromosomal DNA in various tissues of the chimeric mice derived from the ES cells retaining human chromosome #22 fragment

In addition to the determination of coat color in Example 3, the retention of the transferred chromosome fragment was confirmed by PCR analysis using a template genomic DNA prepared from the tail of the chimeric mouse. The tail was obtained from the chimeric mouse at least 3 weeks old in accordance with the method described in Motoya Katsuki, "Development Technology Experiment Manual", published by Kodansha Scientific, 1987. Genomic DNA was extracted from the tail with a Puregene DNA Isolation Kit. Out of the polymorphic primers used in Example 2, PVALB and D22S278 were used, with the extracted genomic DNA as a template, to confirm the amplification products. The analysis was conducted with 10 of the mice in which the contribution to coat color was observed. As a result, the products of amplification with at least either of the primers were detected in all the mice.

Southern blot analysis was conducted in the same manner as in Example 2 by using human L1 sequence as a probe with 2 µ g of the genomic DNA derived from the tails of the 6 chimeric mice and one non-chimeric mouse. As a result, the presence of a large number of human L1 sequence was observed in all the chimeric mice and their patterns were similar to those of E14/#22-9. The quantitative ratio to mouse genome was about 10% at maximum (Fig. 3). In Fig. 3, 2µg of genomic DNA digested with BgIII was used in each lane. Human L1 sequence labeled with ³²P was used as a probe and signals were detected with Image Analyzer BAS2000 (Fuji Photo Film Co., Ltd.). The lanes represent the genomic DNA derived from the tails of the chimeric mice (K22-6, 7, 8, 9, 10, 11 and 12; 9 is the non-chimeric mouse) and control DNA (C which is a mixture of E14/#22-9 genomic DNA and E14 genomic DNA at a weight ratio of 1:9) as counted from the right. The DNA molecular weights are shown at the left side and chimerism in the chimeric mice at the right side (-: 0%, +: <10%, and ++: 10-30%).

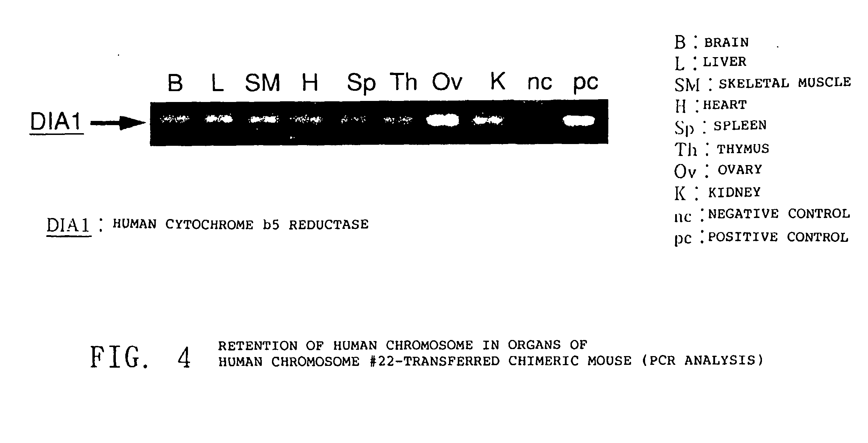

With respect to the chimeric mouse (K22-7) having about 5% contribution to coat color, genomic DNA was obtained from the brain, liver, muscle, heart, spleen, thymus, ovary and kidney with an ISOGEN (Nippon Gene Co.). For each tissue, PCR analysis was conducted with MB and D1A1 selected from the primers for the genes used in Example 2. As a result, both primers gave expected amplification products in all the tissues. The results of PCR analysis using D1A1 primer are shown in Fig. 4. The PCR products were electrophoresed on a 2% agarose gel and stained with ethidium bromide for detection. The lanes in Fig. 4 represent the following from the left: B, brain; L, liver; SM, skeletal muscle; H, heart; Sp, Spleen; Th, thymus; Ov, ovary; K, kidney; nc, non-chimeric mouse tail-derived DNA (negative control); pc, human fibroblast cell (HFL-1) DNA (positive control).

These results show that E14/#22-9 contributed to various normal tissues in the mouse and that it retained human chromosome #22 fragment.

### Example 5

### Expression of the human genes in the chimeric mouse derived from the ES cell retaining human chromosome #22 fragment

The tail of the mouse (K22-7) having about 5% contribution to coat color was frozen with liquid nitrogen and then disrupted for use as a sample for confirming the expression of the human genes. The sample was a mixture of tissues such as skin, bones, muscles, blood and the like. Total RNA was extracted from the sample with an ISOGEN (Nippon Gene Co.) and used in an RT-PCR method to detect mRNAs of human myoglobin (MB) and human cytochrome b5 reductase (D1A1). The RT-PCR was performed in accordance with the method described in Innis et al., "PCR Experiment Manual", published by HBJ Publication Office, 1991. Randam hexamer oligonucleotides (final concentration: 100 pmol, TAKARA SHUZO CO. , LTD.) were used as primers for reverse transcription and Super Script (BRL Co.) as reverse transcriptase. The following primers were used for amplification using cDNA as a template.
MB: 5'-TTAAGGGTCACCCAGAGACT (SEQ ID NO:19), 5'-TGTAGTTGGAGGCCATGTCC (SEQ ID NO:20)
DIA1: 5'-CAAAAAGTCCAACCCTATCA (SEQ ID NO: 21), 5'-GCCCTCAGAAGACGAAGCAG (SEQ ID NO:22)

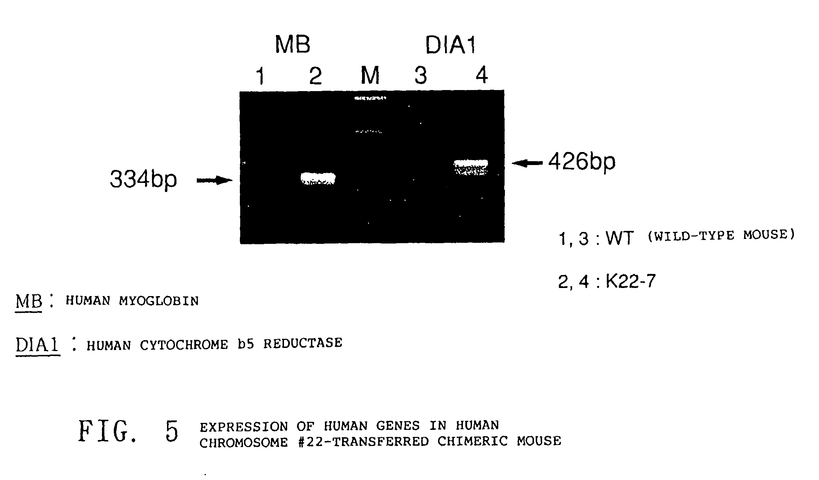

As a result, amplification products specific to mRNAs of both genes were detected (Fig. 5). The RT-PCR products were electrophoresed on a 2% agarose gel and stained with ethidium bromide for detection. In Fig. 5, M is a marker (HindIII digested λ DNA + HaeIII digested φ X174DNA, TAKARA SHUZO CO., LTD.); MB, human myoglobin; D1A1, human cytochrome b5 reductase; and WT, a wild-type C3H mouse.

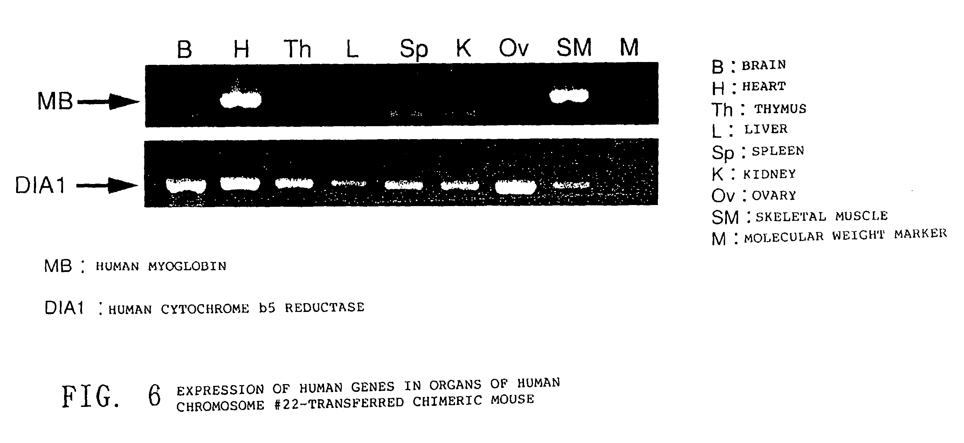

With respect to the same individual (K22-7), total RNA was extracted from the brain, heart, thymus, liver, spleen, kidney, ovary and skeletal muscle with an ISOGEN and RT-PCR was performed on each organ with the above two primers. As a result, expected products of amplification with D1A1 were observed in all the organs and those with MB were observed only in the heart and skeletal muscle (Fig. 6). Myoglobin is known to be expressed specifically in muscle cells (Bassel-Duby et al., MCB, 12:5024, 1992). Hence, the above results show that the gene on the transferred human chromosome fragment can be subjected to the normal tissue-specific regulation in the mouse. The PCR products were electrophoresed on a 2% agarose gel and stained with ethidium bromide for detection. In Fig. 6, the lanes represent the following from the left: B, brain; H, heart; Th, thymus; L, liver; Sp, spleen; K, kidney; Ov, ovary; SM, skeletal muscle; and M, marker (supra). The lower band observed in the results of MB are believed to represent non-specific products.

These results show that the transferred human chromosome #22 fragment can function in normal tissues of the chimeric mice.

### Example 6

### Transfer of human chromosome #4 fragments into ES cells

The mouse A9 cell clone retaining human chromosome #4 (hereinafter referred to as "A9/#4") from Example 1 was used as a chromosome donor cell. Mouse ES cell line E14 (see Example 2) was used as a chromosome recipient cell. The microcell fusion and the selection of G418 resistant clones were conducted by the same procedures as in Example 2. The frequency of the appearance of the drug resistant clones was 1-2 per 10⁷ of E14 cells. The drug resistant clones were stored frozen and genomic DNA were prepared by the same procedures as in Example 2. The retention of the transferred human chromosome #4 or fragments thereof in the drug resistant clones E14/#4-4, E14/#4-7 and E14/#4-11 was confirmed by the methods described in (1)-(3) below.

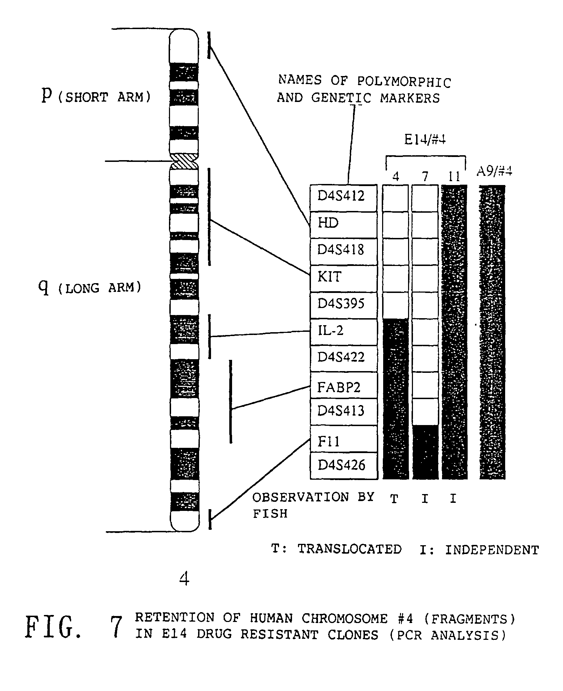

### (1) PCR analysis (Fig. 7)

The presence of the gene on human chromosome #4 (O'Brien, Genetic Maps, 6th edition, Book 5, Cold Spring Harbor Laboratory Press, 1993) and polymorphic markers (Polymorphic STS Primer Pairs: D4S395, D4S412, D4S422, D4S413, D4S418, D4S426 and F11, BIOS Laboratories, Inc.;, Nature 359:794, 1992) was detected by a PCR method. The sequences of oligonucleotide primers for the genes prepared on the basis of nucleotide sequences obtained from data bases such as GenBank, EMBL and the like will be described below.
HD (huntington disease) : 5'-TCGTTCCTGTCGAGGATGAA (SEQ ID NO:23), 5'-TCACTCCGAAGCTGCCTTTC (SEQ ID NO:24)
IL-2 (interleukin-2) : 5'-ATGTACAGGATGCAACTCCTG (SEQ ID NO:25), 5'-TCATCTGTAAATCCAGCAGT (SEQ ID NO:26)
KIT (c-kit) : 5'-GATCCCATCGCAGCTACCGC (SEQ ID NO:27), 5'-TTCGCCGAGTAGTCGCACGG (SEQ ID NO:28)
FABP2 (fatty acid binding protein 2, intestinal), 5'-GATGAACTAGTCCAGGT GAGTT (SEQ ID NO:29), 5'-CCTTTTGGCTTCTACTCCTTCA (SEQ ID NO:30)

PCR amplification was conducted with the above 11 kinds of the primers. As a result, the amplification products having expected lengths were detected with all or part of the primers in all the three clones. In the E14/#4-4 and E14/#4-7 clones, the deletion of partial regions was observed. The results are shown in Fig. 7. In Fig. 7, a schematic chromosome map based on the G bands of human chromosome #4 and the location of some markers on bands are shown at the left side (see Example 2). The arrangement of the genetic and polymorphic markers shows approximate positional relationships on the basis of the presently available information (see Example 2) and the order is not necessarily correct. With respect to the three kinds of the G418 resistant E14 cell clones, the markers for which the expected amplification products were detected are shown by ■ and the markers for which the expected amplification products were not detected are shown by □. The results of the observation by FISH analysis are shown at the lower side. A9/#4 is a chromosome donor cell.

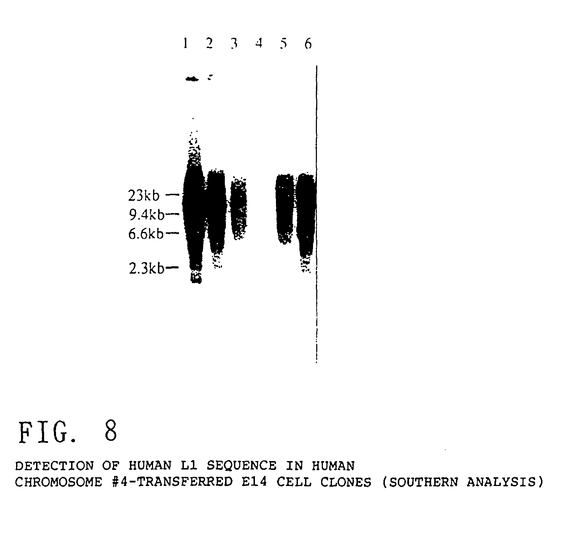

### (2) Southern blot analysis (Fig. 8)

Southern blot analysis was conducted by the same procedure as in Example 2 using human L1 sequence as a probe with genomic DNAs obtained from E14/#4-4 and E14/#4-7. As a result, a large number of bands hybridized with the human L1 sequence were detected in DNAs of both drug resistant clones. The total signal intensity correlated with the degree of the deletion confirmed by the PCR analysis, as compared with that of A9/#4. In Fig. 8, 2*µ* g of genomic DNA digested with BglII was used in each lane. Human L1 sequence labeled with ³²P was used as a probe and the signals were detected with an Image Analyzer (BAS 2000, Fuji Photo Film Co., Ltd.). In Fig. 8, the lanes represent the following as counted from the left: 1, A9/#4 (chromosome donor cell); 2, A9/#4+A9 (1:2); 3, A9/#4+A9 (1:9); 4, A9; 5, E14/#4-7; and 6, E14/#4-4. Lanes 2 and 3 represent mixtures of two kinds of DNAs at the ratios shown in parentheses. The molecular weights of DNAs are shown at the left side.

### (3) Fluorescence in situ hybridization (FISH)

FISH analysis was conducted with probes specific to human chromosomes #4 (CHROMOSOME PAINTING SYSTEM, Cambio Ltd.) by the same procedure as in Example 2. As a result, in almost all of the observed metaphase spreads of the three clones used, human chromosome #4 or partial fragments thereof were detected in the form of translocation to the mouse chromosome with respect to E14/#4-4 and in the form of an independent chromosome with respect to the two other clones. The relative sizes of the observed human chromosome were consistent with those presumed from the results of the PCR analysis.

The results of the above experiments demonstrate that the obtained G418 resistant clones retained the whole human chromosome #4 or partial fragments thereof.

### Example 7

### Production of chimeric mice from the ES cells retaining human chromosome #4 fragments

The cells in frozen stocks of the G418 resistant ES cell clones E14/#4-4 and E14/#4-7 which were confirmed to retain partial fragments of human chromosome #4 were thawed, started to culture, and injected into blastcyst stage embryos obtained by the same method as in Example 3; the injection rate was 10-15 cells per embryo. Two and half days after a foster mother [ICR or MCH(ICR)] mouse (CREA JAPAN, INC.) was subjected to a pseudopregnant treatment, about ten of the ES cell-injected embryos were transplanted to each side of the uterus of the foster mother. The results are shown in Table 2.

**Table 2. Production of chimeric mice from the E14 cell clones retaining human chromosome #4 fragments**

| ES cell clone/human chromosome | G418 resistant clone No. | Number of ES cell-injected blastocyst stage embryos | Number of offspring mice | Number of chimeric mice | Contribution to coat color | | |
|---|---|---|---|---|---|---|---|
| | | | | | <10% | 10-30% | 30%< |
| E14/#4 | 4 | 160 | 8 | 5 | 5 | - | - |
| | 7 | 80 | 5 | 2 | 1 | 1 | - |

As a result of the transplantation of a total of 240 injected embryos, 13 offspring mice were born. Chimerism in the offsprings can be determined by the extent of E14 cell-derived pale gray coat color in the host embryo-derived agouti coat color (dark brown). Out of the 13 offsprings, 7 mice were recognized to have partial pale gray coat color, indicating the contribution of the E14 cells. The maximum contribution was about 15% in one individual derived from E14/#4-7.

These results show that the mouse ES cell clones E14/#4-4 and E14/#4-7 which retain fragments of human chromosome #4 maintain the ability to produce chimera, that is, the ability to differentiate into normal tissues of mouse.

### Example 8

### Confirmation of retention of human chromosomal DNA in the chimeric mice derived from the ES cells retaining partial fragments of human chromosome #4 and expression of the G418 resistance gene

### (1) PCR analysis

Using the chimeric mice produced in Example 7, genomic DNAs were prepared from the tails of one individual derived from E14/#4-7 (K#4-7-1: about 5% chimerism) and one individual derived from E14/#4-4 (K#4-4-41: about 5% chimerism) by the same procedure as in Example 4. These DNAs were used as templates to conduct PCR analysis using polymorphic marker F11 for chromosome #4 analysis (see Example 6) which was detected in E14/#4-7 and E14/#4-4. As a result, expected amplification products were detected in both mice.

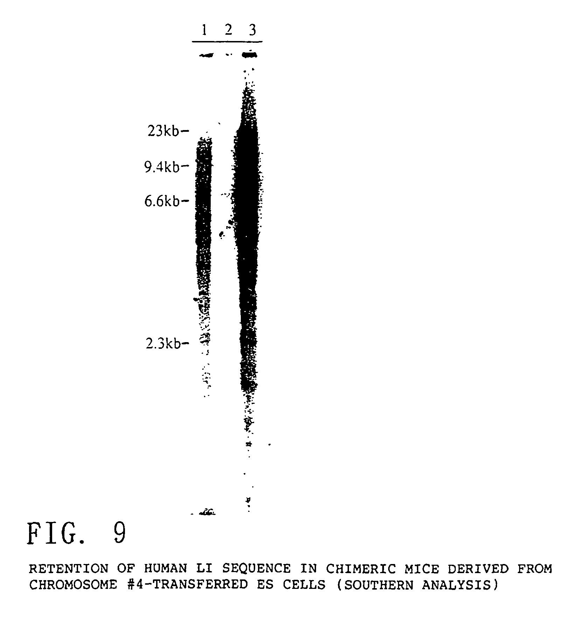

### (2) Southern analysis (Fig. 9)

Southern analysis was conducted in the same manner as in Example 2 by using human L1 sequence as a probe with 2*µ*g of the genomic DNA derived from the tail of one individual derived from E14/#4-7 (K#4-7-1: about 5% chimerism). As a result, the presence of a large number of human L1 sequence was observed and their patterns were similar to those of E14/#4-7. The quantitative ratio to mouse genome was about 10% of that of E14/#4-7 at maximum. In Fig. 9, 2*µ*g of genomic DNA digested with BglII was used in each lane. Human L1 sequence labeled with ³²P was used as a probe and signals were detected with Image Analyzer BAS2000 (Fuji Photo Film Co., Ltd.). The molecular weights of DNAs are shown at the left side. The lanes represent the following as counted from the left: 1, K#4-7-1; 2, blank; and 3, E14/#4-7.

### (3) Test on the tail-derived fibroblast cells for G418 resistance

Fibroblast cells were prepared from the tails of one individual derived from E14/#4-7 (K#4-7-1: about 5% chimerism) and one individual derived from E14/#4-4 (K#4-4-41: about 5% chimerism). In the same procedure as in Example 4, the tail of each mouse was cut at a length of 5-10 mm and washed several times with PBS/1mM EDTA, followed by notching of the tail with a knife. The outer skin layer was removed and the inner tissues were cut into fine pieces. The fine pieces of tissues were transferred into a tube containing 5 ml of PBS/1 mM EDTA and left to stand for 30 minutes to 1 hour at room temperature. Subsequently, the supernatant was removed leaving a 1 ml portion of the PBS/EDTA behind, and 1 ml of 0.25% trypsin/PBS was added. The tissues were dispersed thoroughly by tapping or pipetting at room temperature for 5-10 minutes. After centrifugation at 1,000 rpm for 10 minutes, the precipitate was suspended in 2 ml of DMEM (10% FCS) and inoculated into a 35 mm plate. After cultivation for 7-10 days, the cells were treated with trypsin and about 10⁴ cells per plate were inoculated into two 35 mm plates. G418 was added to the medium in one plate at a final concentration of 400 µg/ml. The cells were cultured for 5-7 days and the appearance of viable cells in each plate were examined. Under these conditions, 100% of the wild-type ICR mouse-derived fibroblast cells were killed in the presence of G418. As a result, G418 resistant fibroblast cells was present in both mice.

These results show that E14/#4-7 and E14/#4-4 contributed to various normal tissues in the mouse and that they retained partial fragments of human chromosome #4.

### Example 9

### Transfer of human chromosome #14 or fragments thereof into mouse ES cells

The mouse A9 cell clone retaining human chromosome #14 (hereinafter referred to as "A9/#14") from Example 1 was used as a chromosome donor cell. Mouse ES cell line TT2 (purchased from Lifetech Oriental Co., Yagi et al., Analytical Biochem., 214:70, 1993) was used as a chromosome recipient cell. The TT2 cells were cultured in accordance with the method described in Aizawa Shinichi, "Biomanual Series 8, Gene Targeting", published by Yodosha, 1995 and G418 resistant primary culture cells (purchased from Lifetech Oriental Co.) treated with mitomycin C (Sigma) were used as feeder cells. The microcell fusion and the selection of G418 resistant clones were conducted by the same procedures as in Example 2. The frequency of the appearance of the drug resistant clones was 3-6 per 10⁷ of TT2 cells. The drug resistant clones were stored frozen and genomic DNA was prepared by the same procedures as in Example 2.

Human chromosome #14 was fragmented by irradiating the microcells with γ-rays (Koi et al., Science, 260:361, 1993). The microcells obtained from about 10⁸ cells of A9/#14 were suspended in 5 ml of DMEM and irradiated with γ -rays of 30 Gy on ice with a Gammacell 40 (Canadian Atomic Energy Public Corporation) at 1.2 Gy/min for 25 minutes. The fusion of γ ray-irradiated microcells and the selection of drug resistant clones were conducted by the same procedure as in the case of the unirradiated micorcells. As a result, the frequency of the appearance of the drug resistant clones was 3 per 10⁷ of TT2 cells. The drug resistant clones were frozen stored and DNA was prepared by the same procedure as in Example 2.

The retention of human chromosome #14 or partial fragments thereof in the unirradiated microcell-transferred G418 resistant clones 1-4 and 1-5, and in the G418 resistant clones 3-1 and 3-2 (a total of 4 clones) into which the γ -ray-irradiated microcell was transferred was confirmed by the methods described in (1) and (2) below.

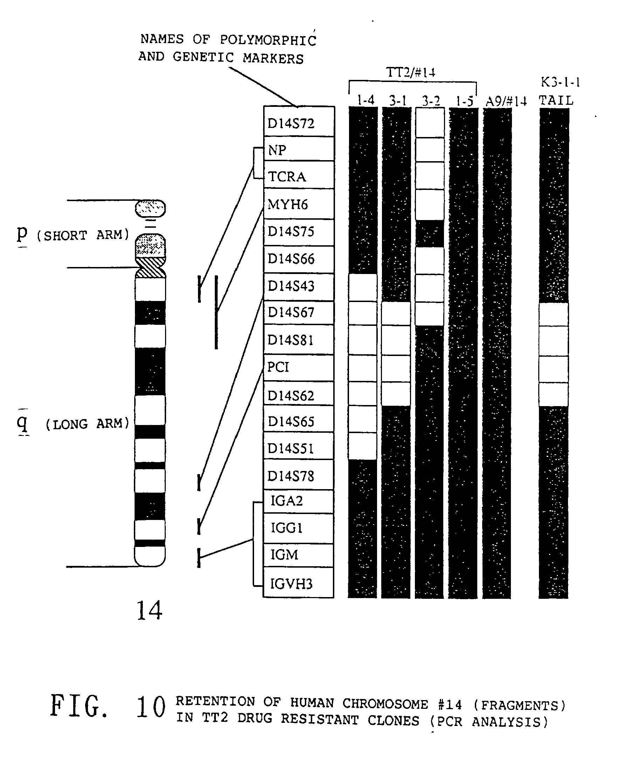

### (1) PCR analysis (Fig. 10)

The presence of the gene on human chromosome #14 (O'Brien, Genetic Maps, 6th edition, Book 5, Cold Spring Harbor Laboratory Press, 1993) and polymorphic markers (Polymorphic STS Primer Pairs: D14S43, D14S51, D14S62, D14S65, D14S66, D14S67, D14S72, D14S75, D14S78, D14S81 and PCI, BIOS Laboratories, Inc.; Nature 359:794, 1992; Nature Genetics, 7:22, 1994) was detected by a PCR method using genomic DNA of the drug resistant clone as a template. The sequences of oligonucleotide primers for the genes prepared on the basis of nucleotide sequences obtained from data bases such as GenBank, EMBL and the like are described below. NP (nucleoside phosphorylase) : 5'-ATAGAGGGTACCCACTCTGG (SEQ ID NO:31), 5'-AACCAGGTAGGTTGATATGG (SEQ ID NO:32)
TCRA (T-cell receptor alpha) : 5'-AAGTTCCTGTGATGTCAAGC (SEQ ID NO:33), 5'-TCATGAGCAGATTAAACCCG (SEQ ID NO:34)
MYH6 (myosin heavy chain cardiac) : 5'-TGTGAAGGAGGACCAGGTGT (SEQ ID NO:35), 5'-TGTAGGGGTTGACAGTGACA (SEQ ID NO:36)
IGA2 (immunoglobulin alpha-2 constant) : 5'-CTGAGAGATGCCTCTGGTGC (SEQ ID NO:37), 5'-GGCGGTTAGTGGGGTCTTCA (SEQ ID NO:38)
IGG1 (immunoglobulin gamma-1 constant) : 5'-GGTGTCGTGGAACTCAGGCG (SEQ ID NO:39), 5'-CTGGTGCAGGACGGTGAGGA (SEQ ID NO:40)
IGM (immunoglobulin mu constant) : 5'-GCATCCTGACCGTGTCCGAA (SEQ ID NO:41), 5'-GGGTCAGTAGCAGGTGCCAG (SEQ ID NO:42)
IGVH3 (immunoglobulin heavy variable-3): 5'-AGTGAGATAAGCAGTGGATG (SEQ ID NO:43), 5'-GTTGTGCTACTCCCATCACT (SEQ ID NO:44)

PCR amplification was conducted using the genomic DNAs of the 4 drug resistant clones as templates with the above 18 kinds of the primers by the same procedure as in Example 2. As a result, expected amplification products were detected with all or part of the primers. In the drug resistant clones 3-1 and 3-2 obtained by using the γ -ray irradiated microcells, a tendency for the deletion of partial regions of chromosome #14 was observed. In the case where the unirradiated microcells were used, deletion was observed as in the case of the 1-4 clone. The results are shown in Fig. 10. In Fig. 10, a schematic chromosome map based on the G bands of human chromosome #14 and the location of some markers on bands are shown at the left side (see Example 2). The arrangement of the genetic and polymorphic markers shows approximate positional relationships on the basis of the presently available information (see Example 2) and the order is not necessarily correct. With respect to four kinds of the G418 resistant TT2 cell clones, the markers for which the expected amplification products were detected are shown by ■ and the markers for which the expected amplification products were not detected are shown by □. A9/#14 is a chromosome donor cell. The results of Example 11 (1) are shown at the right side.

### (2) Fluorescence in situ hybridization (FISH)

FISH analysis was conducted with probes specific to human chromosomes #14 (CHROMOSOME PAINTING SYSTEM, Cambio Ltd.) in accordance with the method described in Matsubara et al., "FISH Experiment Protocol", published by Shujunsha, 1994. As a result, in almost all of the observed metaphase spreads of all the 4 clones, human chromosome #14 or partial fragments thereof were detected in the form of an independent chromosome. The relative sizes of the observed human chromosome were consistent with those presumed from the results of the PCR analysis.

The results of the above experiments demonstrate that the obtained G418 resistant clones 1-4, 1-5, 3-1 and 3-2 retained the whole or partial fragments of human chromosome #14.

### Example 10

### Production of chimeric mice from the ES cells retaining human chromosome #14 fragments

The cells in the frozen stocks of four G418 resistant ES cell clones (1-4, 3-1, 3-2 and 1-5) that were prepared in Example 9 and which were confirmed to retain human chromosome #14 or fragments thereof were thawed, started to culture and injected into 8-cell stage embryos obtained by mating [ICR or MCH(ICR)] male and female mice (CREA JAPAN, INC.); the injection rate was 8-10 cells per embryo. The embryos were cultured in an ES medium overnight to develop to blastocysts. Two and half days after a foster mother ICR mouse (CREA JAPAN, INC.) was subjected to a pseudopregnant treatment, about ten of the injected embryos were transplanted to each side of the uterus of the foster mother. The results are shown in Table 3.

**Table 3. Production of chimeric mice from the TT2 cell clones retaining human chromosome #14 fragments**

| ES cell clone/human chromosome | G418 resistant clone No. | Number of ES cell-injected 8-cell stage embryos | Number of offspring mice | Number of chimeric mice | Contribution to coat color | | |
|---|---|---|---|---|---|---|---|
| | | | | | <20% | 20-50% | 50-80% |
| TT2/#14 | 1-4 | 98 | 20 | 1 | - | - | 1 |
| | 1-5 | 110 | 14 | 2 | 1 | - | 1 |
| | 3-1 | 103 | 11 | 2 | 1 | 1 | - |
| | 3-2 | 183 | 19 | 3 | - | 2 | 1 |

As a result of the transplantation of a total of 494 injected embryos, 64 offspring mice were born. Chimerism in the offsprings can be determined by the extent of TT2 cell-derived agouti coat color (dark brown) in the host embryo-derived albino coat color. Out of the 64 produced offsprings, 8 mice were recognized to have partial agouti coat color, indicating the contribution of the ES cells. The maximum contribution was about 80% in one individual derived from 1-4.

These results show that the G418 resistant ES cell clones (1-4, 1-5, 3-1 and 3-2) retaining human chromosome #14 or fragments thereof maintain the ability to produce chimera, that is, the ability to differentiate into normal tissues of mouse.

### Example 11

### Confirmation of retention of human chromosome #14 fragment DNA in the chimeric mice derived from the ES cells retaining human chromosome #14 fragments

The retention of human chromosome #14 partial fragments in the chimeric mice obtained in Example 10 was confirmed by the methods described in (1)-(3) below.

### (1) PCR analysis using DNAs derived from various tissues

Genomic DNA was extracted from the tail of one individual derived from 3-1 (K3-1-1: about 25% chimerism) by the same procedure as in Example 4. The DNA was used as a template to conduct PCR analysis using all of the 14 primers for chromosome #14 analysis which were detected in 3-1. As a result, expected amplification products were detected with all the 14 primers. (Fig. 10)

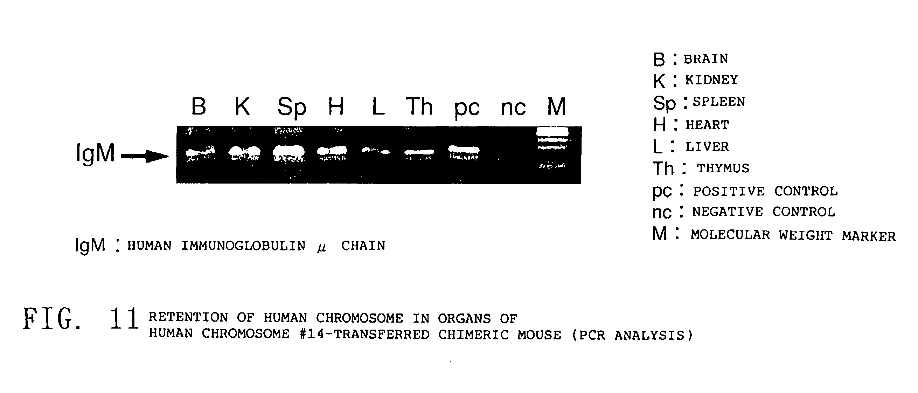

With respect to the same individual (K3-1-1), genomic DNA was obtained from the brain, kidney, spleen, heart, liver and thymus with a Puregene DNA Isolation Kit. For each tissue, PCR analysis was conducted with IGM primers (see Example 9). As a result, expected amplification products were detected in all the tissues (Fig. 11). The PCR products were electrophoresed on a 2% agarose gel and stained with ethidium bromide for detection. In Fig. 11, the lanes represent the following as counted from the left: B, brain; K, kidney; Sp, Spleen; H, heart; L, liver; Th, thymus; pc, human fibroblast cell (HFL-1) DNA (positive control); nc, non-chimeric mouse tail DNA (negative control); and M, marker (HindIII digested λ DNA + HaeIII digested φ X174 DNA, TAKARA SHUZO CO., LTD.).

### (2) Test on the tail-derived fibroblast cells for G418 resistance

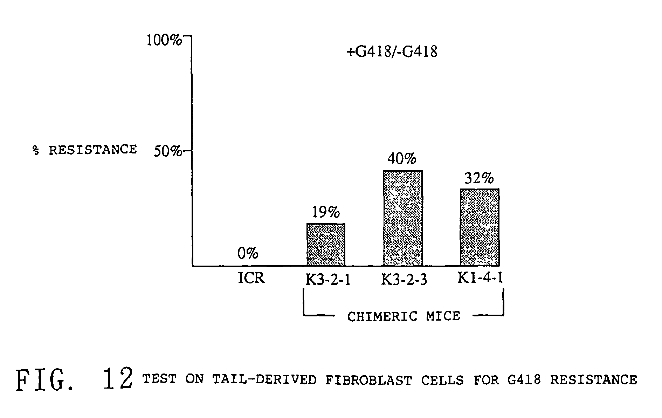

Fibroblast cells were prepared from the tails of two individuals derived from 3-2 (K3-2-1: about 25% chimerism, and K3-2-3: about 50% chimerism) and one individual derived from 1-4 (K1-4-1: about 80% chimerism). In the same procedure as in Example 4, the tail of each chimeric mouse of 3-6 weeks was cut at a length of 5-10 mm and washed several times with PBS/1mM EDTA, followed by notching of the tail with a knife. The outer layer was removed and the inner tissues were cut into fine pieces. The fine pieces of tissues were transferred into a tube containing 5 ml of PBS/1 mM EDTA and left to stand for 30 minutes to 1 hour at room temperature. Subsequently, the supernatant was removed leaving a 1 ml portion of the PBS/EDTA behind, and 1 ml of 0.25% trypsin/PBS was added. The tissues were dispersed thoroughly by tapping or pipetting at room temperature for 5-10 minutes. After centrifugation at 1,000 rpm for 10 minutes, the precipitate was suspended in 2 ml of DMEM (10% FCS) and inoculated into a 35 mm plate. After cultivation for 7-10 days, the cells were treated with trypsin and about 10' cells per plate were inoculated into four 35 mm plates. G418 was added to the medium in two of the plates at a final concentration of 400µ g/ml. The cells were cultured for 5-7 days and the viable cells in each plate were counted. Under these conditions, 100% of the wild-type ICR mouse-derived fibroblast cells were killed in the presence of G418. Assuming the same growth rate of the G418 resistant fibroblast in the non-selective and selective media, the ratio of the viable cells in the selective medium to those in the non-selective medium is believed to reflect the contribution in the fibroblast cell populations of the G418 resistant ES cell-drived fiblablast. As a result, the presence of G418 resistant fibroblast cells was observed in all the three individuals as shown in Fig. 12. In Fig. 12, % resistance is an average of 2 pairs of the selective/non-selective 35 mm plates for each mouse. ICR refers to the wild-type ICR mice.

### (3) FISH analysis of the tail-derived G418 resistant fibroblast cells

FISH analysis of the K3-2-3 and K1-4-1 derived G418 resistant fibroblast cells obtained in (2) was conducted by the same procedure as in Example 2. Total human DNA extracted from the HFL-1 cells (Example 1) was labeled with FITC so that is could be used as a probe (Matsubara et al., "FISH Experimental Protocol", published by Shujunsha, 1994). As a result, in almost all of the observed metaphase spreads of the both individuals, partial fragments of the human chromosome in independent forms were observed.

These results show that the TT2 cell clones retaining fragments of human chromosome #14 contributed to various normal tissues in the mouse individuals and that they retained partial fragments of human chromosome #14.

### Example 12

### Transfer of partial fragments of human chromosome #2 into ES cells

The mouse A9 cell W23 retaining a human chromosome #2 fragment (hereinafter referred to as "A9/#2 W23") from Example 1 was used as a chromosome donor cell. Mouse ES cell line TT2 (see Example 9) was used as a chromosome recipient cell. The microcell fusion and the selection of G418 resistant clones were conducted by the same procedures as in Example 2. The frequency of the appearance of the drug resistant clones was 1-3 per 10⁷ of TT2 cells. The drug resistant clones were stored frozen and genomic DNA was prepared by the same procedures as in Example 2. The retention of partial fragments of human chromosome #2 in drug resistant clones 5-1, 5-2 and 5-3 was confirmed by the methods described in (1) and (2) below.

### (1) PCR analysis

The presence of C_{κ} and FABP1 that are the genes on human chromosome #2 (Genetic Maps, supra) and which were detected in the chromosome donor cell A9/#2 W23 was detected by a PCR method.

As a result of PCR amplification using each primer, expected amplification products were detected with both primers in all of the 3 clones.

### (2) Fluorescence in situ hybridization (FISH)

FISH analysis was conducted with probes specific to human chromosome #2 (CHROMOSOME PAINTING SYSTEM, Cambio Ltd.) by the same method as in Example 2. As a result, in almost all of the observed metaphase spreads of the 3 clones, partial fragments of human chromosome #2 in the form of independent chromosomes were detected. The sizes of the observed human chromosome were the same as those observed in A9/#2 W23.

The results of the above experiments demonstrate that the obtained G418 resistant clones retained partial fragments of human chromosome #2.

### Example 13

### Production of chimeric mice from the ES cells retaining human chromosome #2 fragment

The cells in a frozen stock of the G418 resistant ES cell clone 5-1 that was obtained in Example 12 and which was confirmed to retain human chromosome #2 was thawed, started to culture and injected into 8-cell stage embryos obtained by mating ICR or MCH(ICR) male and female mice (CREA JAPAN, INC.); the injection rate was 10-12 cells per embryo. The embryos were cultured in an ES medium (Example 9) overnight to develop to blastocysts. Two and half days after a foster mother ICR mouse (CREA JAPAN, INC.) was subjected to a pseudopregnant treatment, about ten of the injected embryos were transplanted to each side of the uterus of the foster mother. The results are shown in Table 4.

**Table 4. Production of chimeric mice from the TT2 cell clone retaining human chromosome #2 fragments**

| ES cell clone/human chromosome | G418 resistant clone No. | Number of ES cell-injected 8 cell stage embryos | Number of offspring mice | Number of chimeric mice | Contribution to coat color | | |
|---|---|---|---|---|---|---|---|
| | | | | | <20% | 20-50% | 50-80% |
| TT2/#2 | 5-1 | 264 | 51 | 18 | 7 | 5 | 6 |
| (W23) | | | | | | | |

As a result of the transplantation of a total of 264 injected embryos, 51 offspring mice were born. Chimerism in the offsprings can be determined by the extent of TT2 cell-derived agouti coat color (dark brown) in the host embryo-derived albino coat color. Out of the 51 produced offsprings, 18 mice were recognized to have partial agouti coat color, indicating the contribution of the ES cells. The maximum contribution was about 80%.

These results show that the G418 resistant ES cell clone (5-1) retaining a fragment of human chromosome #2 maintains the ability to produce chimera, that is, the ability to differentiate into normal tissues of mouse individual.

### Example 14

### Detection of human antibody heavy chain in sera of the human chromosome #14 fragment-transferred chimeric mice

The concentrations of human antibody in the sera were determined by enzyme-linked immunosorbent assay (ELISA). The ELISA for human antibody was performed in accordance with the method described in Toyama and Ando, "Monoclonal Antibody Experiment Manual", published by Kodansha, 1987; Andou and Chiba, "Monoclonal Antibody Experiment Procedure Manual", published by Kodansha Scientific, 1991; Ishikawa, "Super High Sensitivity Enzyme Immuno Assay", published by Gakkai-syuppan center, 1993; Ed Harlow and David Lane, "Antibodies A Laboratory Manual", published by Cold Spring Harbor Laboratory, 1988 and A. Doyle and J. B. Griffiths, "Cell & Tissue Culture: Laboratory Procedures", published by John Wiley & Sons Ltd., 1996. In some assays, the condition of reaction were modified, for example, the reaction was performed at 4°C over night. Antibodies to human-immunogloblin or antigen were diluted to about 0.5-10 µg/ml (100-5000 fold) and ELISA plates were coated with these solutions. PBS supplemented with 5% mouse serum (Sigma, M5905) was used for blocking and dilution of the samples and labeled antibodies. PBS was used for 20-fold dilution of the chimeric mouse sera. After washed, the coated plate was blocked over 1 hour. After plate was washed, sample was added and incubated over a half hour. After washed, Enzyme labeled anti-human immunogloblin antibodies diluted 100-5000 folds were added to the plates and incubated over 1 hour, the plate was washed and then substrate was added. In some assays, the same procedure was applied except that a biotin-labeled antibody was used. After plate was washed, avidin-enzyme complex was added. After plate was washed, substrate was added. Absorbances were measured with a microplate reader (Bio-tek instrument, EL312e). The chimeric mice (Example 10, K3-1-2, K3-2-2 and K3-2-3) which were 29-35 days old were bled and assayed by ELISA. Anti-human IgM mouse monoclonal antibody (Sigma, I6385) was diluted with 50 mM carbonate-bicartonate buffer (pH 9.6) and absorbed to the 96-well microtiter plates. The serum samples diluted with mouse serum (Sigma, M5905) supplemented PBS were added to the plates. Subsequently, peroxidase-labeled anti-human IgM goat antibody (Tago, 2392) was added and the plates were incubated. After ABTS substrate (Kirkegaard & Perry Laboratories Inc., 506200) was added, enzyme activity was determined by absorbance measurement at 405 nm. Purified human IgM antibody (CAPEL, 6001-1590) and IgG (Sigma, I4506) were used as standards. The standards were diluted stepwise with mouse serum-supplemented PBS. In the determination of human IgG concentration, anti-human IgG goat antibody (Sigma, I3382) was absorbed to the plate and the human IgG was detected with peroxidase-labeled anti-human IgG goat antibody (Sigma, A0170). The results are shown in Table 5. Both human IgM and IgG were detected.

**Table 5. Concentrations of Human Antibodies in Chimeric Mouse Sera (ELISA)**

| Chimeric Mouse | IgG (mg/l) | IgM (mg/l) |
|---|---|---|
| K3-1-2 | 0.37 | 3.7 |
| K3-2-2 | 0.33 | 5.9 |
| K3-2-3 | 0.51 | 3.4 |

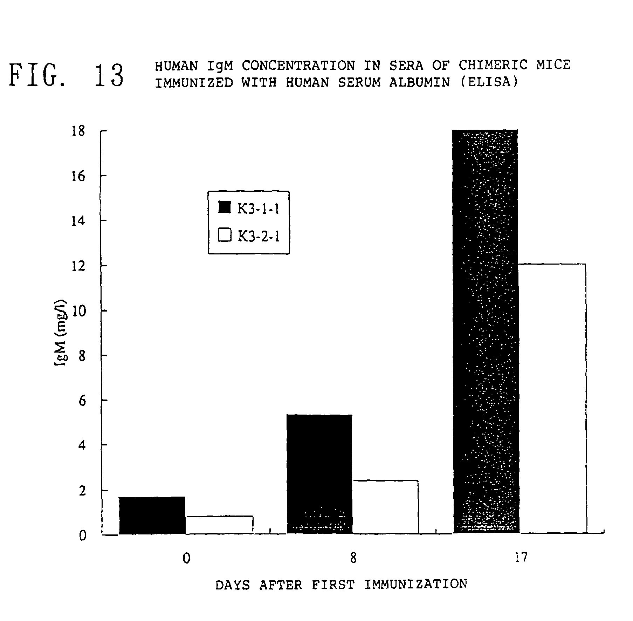

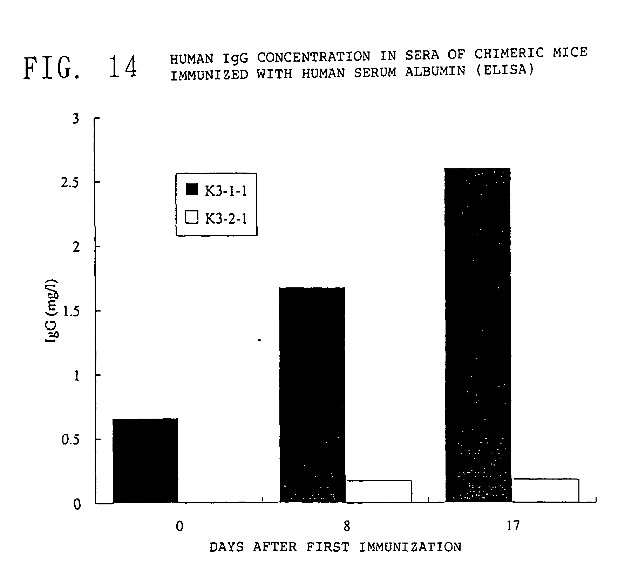

Two milliliters of human serum albumin (HSA, Sigma, A3782) dissolved in PBS was mixed with adjuvant (MPL+TDM Emulsion, RIBI Immunochem Research Inc.) to prepare an antigen solution at a concentration of 0.25 mg/ml. The chimeric mice retaining human chromosome #14 fragment (Example 10, K3-1-1 and K3-2-1) were immunized with 0.2 ml of the antigen solution 3 times at days 27, 34 and 41 after birth. The chimeric mouse sera were assayed by ELISA. The results are shown in Figs. 13 and 14. The human antibody concentration in the sera of the HSA-immunized chimeric mice was increased after the immunization. In the K3-1-1 mouse, 18 µg/ml of human IgM and 2.6 µg/ml of IgG were detected in the serum at day 17 after the immunization. In the serum of the control ICR mouse, the human antibody titer was not significant.

### Example 15

### Production of human antibody heavy chain-producing hybridomas from the human chromosome #14 fragment-transferred chimeric mouse

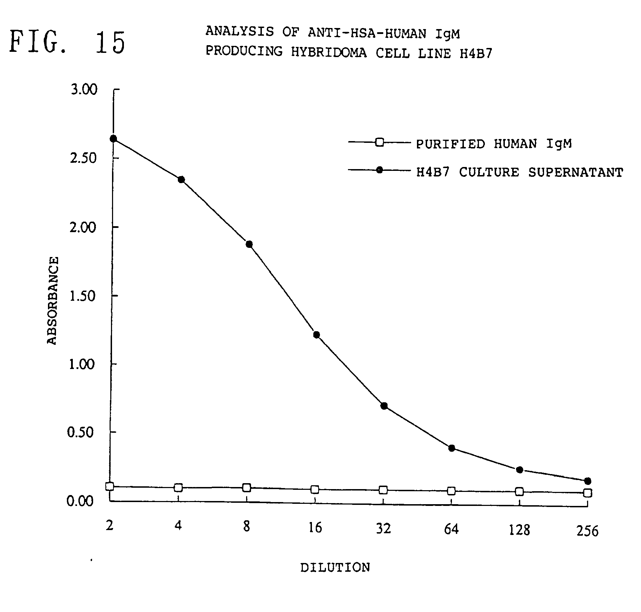

The spleen was removed from the human albumin-immunized chimeric mouse (K3-1-1, Example 14) at day 44 after birth. The spleen cell was fused with a myeloma cell to produce a hybridoma. The hybridoma was produced using a myeloma cell P3X63Ag8.653 (DAINIPPON PHARMACEUTICAL CO., LTD., 05-565) by the method described in Ando and Chiba, "Monoclonal Antibody Experimental Procedure Manual", published by Kodansha Scientific, 1991. The hybridomas were inoculated into ten 96-well plates and cultured for 1 week. The culture supernatant was analyzed by ELISA. The ELISA procedure was conducted by using anti-human IgM mouse monoclonal antibody (Sigma, 16385) immobilized on ELISA plate in the same manner as in Example 14 to give 6 positive clones. HSA (antigen) was dissolved in 50 mM carbonate-bicarbonate buffer (pH 9.6) at a concentration of 5*µ*g/ml and the antigen solution was dispensed in 100*µ*l portions into all the wells of the ELISA plates. After the addition of the supernqtant, peroxidase-labeled anti-human IgA+IgG+IgM goat antibodies (Kierkegaard & Perry Laboratories Inc., 04-10-17) were used for detection of HSA-specific human antibody. One positive clone was confirmed in the ten plates. This clone was one of the 6 human IgM positive clones. The clone (H4B7) was further cultured and the culture supernatant was diluted, followed by ELISA analysis using HSA as an antigen with peroxidase-labeled anti-human IgM goat antibody (Tago, 2392) in the same manner as described above. As a result, the absorbance decreased with the increase in the dilution of the culture solution. Serial twofold dilutions of 2*µ*g/ml human IgM (CAPEL, 6001-1590) showed low absorbance regardless of dilution ratios. This suggests that the antibody produced by hybridoma H4B7 had a specificity to HSA (Fig. 15). In Fig. 15, the dilution of the culture supernatant samples is plotted on the horizontal axis and the absorbance at 405 nm is plotted on the vertical axis.

### Example 16

### Re-marking of the G418 resistance-marked human chromosome #2 fragment with puromycin resistance

The A9 cells retaining the G418 resistance-marked human chromosome #2 fragment (W23) (see Example 1, Fig. 1) were cultured in a G418 (800 *µ*g/ml) containing selective medium (10% FBS, DMEM) in a 100 mm plate. Plasmid pPGKPuro (provided by Dr. Peter W. Laird (WHITEHEAD INSTITUTE)) containing puromycin resistance gene was linearized with restriction enzyme SalI (TAKARA SHUZO CO., LTD.) before transfection. The cells were treated with trypsin and suspended in Dulbecco's phosphate buffered saline (PBS) at a concentration of 5 x 10⁶ cells/ml, followed by electroporation using a Gene Pulser (Bio-Rad Laboratories, Inc.) in the presence of 10*µ* g of DNA in the same manner as in Example 1. A voltage of 1000 V was applied at a capacitance of 25*µ*F with an Electroporation Cell of 4 mm in length (Example 1) at room temperature. The electroporated cells were inoculated into media in 3-6 plates of 100 mm φ. After one day, the medium was replaced with a double-selective medium containing 10 *µ* g/ml of puromycin (Sigma, P-7255) and 800 *µ* g/ml of G418. The colonies formed after 2-3 weeks were collected in groups each consisting of about 200 colonies. The cells of each of the three groups were cultured in two or three 25 cm² flasks to form microcells. The mouse A9 cells were cultured in a 25 cm² flask and fused with the microcells by the same procedure as in Example 1. The fused cells were transferred into two 100 mm plates and cultured in the double-selective medium containing G418 and puromycin. One of the three groups gave two double-drug resistant clones. In these clones, it was most likely that puromycin resistance marker had been introduced into human chromosome #2 fragment.

### Example 17

### Duplication of transferred human chromosome in the human chromosome transferred ES cells

The ES cell clone retaining the G418 resistance marked human chromosome #14 fragment (E14/#14-36) was cultured in a medium containing G418 at a high concentration to give ES cell clones in which the human chromosome was duplicated ("Biomanual Series 8, Gene Targeting", published by Yodosha, 1995). G418 resistant mouse primary cells (purchased from Lifetech Oriental) were inoculated into a 100 mm plate without treating with mitomycin C and used as feeder cells. The E14/#14-36 cells were inoculated into the 100 mm plate and after half a day, the medium was replaced with a medium containing G418 at a concentration of 16 mg/ml. The medium was replaced every 1-2 days. The G418 concentration was changed to 10 mg/ml one week later and the cultivation was continued. Among the colonies formed, 15 were picked up and cultured, followed by FISH analysis of chromosome using human chromosome #14 specific probes (see Example 9). As a result, human chromosome #14 fragment was found to have duplicated in the 8 clones.

### Example 18

### Preparation of Mouse ES cells retaining both human chromosome #2 partial fragments and human chromosome #14 partial fragments.

In a microcell transfer experiment using the double-drug resistant clone PG-1 from Example 16 as a microcell donor cell and a wild-type A9 cell as a recipient cell, it was confirmed that the human chromosome #2 partial fragment retained in PG-1 was marked with a puromycin resistance gene. The preparation of microcells and the fusion with the A9 cells was carried out by the same methods as in Example 1. As a result, 10 days after the microcell fusion, a total of fifty nine G418 resistant colonies appeared. After the medium for these colonies was changed to one containing 8 *µ* g/ml puromycin, the colonies were cultured for 3 days to give 45 viable colonies (76%). In many cases of microcell fusion, only one or few chromosomes are transferred into a recipient cell. Hence, cotransfer of both the resistance genes at a high frequency shows that the G418 resistance-labeled chromosome #2 partial fragment retained in the PG1 clone was also marked with the puromycin resistance gene. In addition, for the detection of the respective marker genes on the human chromosome #2 partial fragment, FISH analysis was conducted by using pSTneoB (see Example 1) as a probe in the case of the A9/#2 W23 clone having only G418 resistance (see Example 16) and by using pPGKPuro (see Example 16) as a probe in the case of the PG1 clone in accordance with the method described in Matsubara et al., "FISH Experiment Protocol", published by Shujunsha, 1994. As a result, in the case of the A9/#2 W23 clone, one signal was observed in each of the sister chromatids of the human chromosome #2 partial fragment observed in Example 12 (2 signals in total). This indicated the insertion of pSTneoB into the human chromosome #2 partial fragment at one site. In the case of the PG1 clone, a total of 4 signals were observed on a chromosome fragment of the same size as in A9/#2 W23. Since pSTneoB and pPGKPuro had identical sequences in their vector portions, the pSTneoB could be detected by the pPGKPuro probe. Hence, it is believed that out of the four signals observed in the PG1 clone, two were from the pSTneoB and the other two were from the pPGKPuro. These results show that the human chromosome #2 partial fragment retained in the PG1 was marked with both the G418 and puromycin resistances.

The PG1 cell clone was used as a chromosome donor cell to prepare a mouse ES cell retaining both a human chromosome #2 partial fragment and a human chromosome #14 partial fragment. The G418 resistant TT2 cell clone 1-4 already retaining the human chromosome #14 partial fragment (see Example 9) was used as a chromosome recipient cell. The microcell fusion and the selection of puromycin resistant cells were carried out by the same methods as in the selection of the G418 resistant clones in Example 9 except that the concentration of puromycin was 0.75 *µ*g/ml. The frequency of the appearance of the resulting puromycin resistant clones was 3-7 per 10' of 1-4 cells. The presence of G418 resistance in these puromycin resistant clones was confirmed from the fact that they were grown in the presence of 300 µ g/ml of G418. The double-drug resistant clones were stored frozen and genomic DNA was prepared by the same methods as in Example 2. The retention of the human chromosome #2 partial fragment and human chromosome #14 partial fragment was confirmed by the method described in (1) in the case of double-drug resistant clones PG5, PG15 and PG16 and by the method described in (2) in the case of the clone PG15.

### (1) PCR analysis

Genomic DNAs of the double-drug resistant clones were used as templates in the PCR amplifications. Among the markers on human chromosomes #2 and #14 (Genetic Maps, supra), the primers whose presence in the A9/#2 W23 clone was confirmed in Example 12 and those whose presence in the TT2/#14 1-4 clone was confirmed in Example 9 were used. All the primers gave expected amplification products in all the three clones.

### (2) Fluorescence in situ hybridization (FISH)

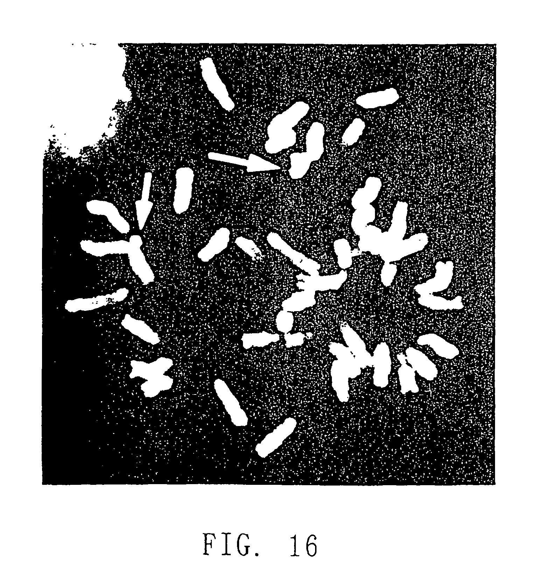

FISH analysis was conducted by using FITC-labeled human total DNA as a probe in the same manner as in Example 11. As a result, in almost all of the metaphase spreads, two (large and small) human chromosome fragments were detected. The large fragment had the same size as that of the partial fragment detected by using the human chromosome #14 specific probes in the case of the TT2/#14 1-4 clone in Example 9 and the small fragment had the same size as that of the partial fragment detected by using the human chromosome #2 specific probes in the case of the TT2/#2 5-1 in Example 12. The results are shown in Fig. 16. In Fig. 16, the less bright chromosome was derived from the mouse. The two (large and small) chromosome fragments of high brightness due to FITC fluorescence as shown by arrows were derived from the human, which are believed to correspond to the human chromosome #14 and #2 partial fragments.

These results show that the obtained double-drug resistant ES clones retained both the human chromosome #2 partial fragment and the human chromosome #14 partial fragment.

### Example 19

### Production of chimeric mice from the mouse ES cell clones retaining both human chromosome #2 partial fragments and human chromosome #14 partial fragments

The cells in frozen stocks of the G418 and puromycin double-resistant TT2 cell clones PG5, PG15 and PG16 from Example 18 which were confirmed to retain human chromosome #2 partial fragments and human chromosome #14 partial fragments were thawed, started to culture and injected into 8-cell stage embryos obtained by mating ICR or MCH(ICR) male and female mice (CREA JAPAN, INC.); the injection rate was 10-12 cells per embryo. The embryos were cultured in a medium for ES cells (see Example 9) overnight to develop to blastocysts. Two and a half day after a foster mother ICR mouse was subjected to a pseudopregnant treatment, about ten of the injected embryos were transplanted to each side of the uterus of the foster mother. The results are shown in Table 6.

**Table 6. Production of chimeric mice from the mouse ES cell clones retaining both human chromosome #2 partial fragments and human chromosome #14 partial fragments**

| ES cell clone/human chromosome | Double-drug resistant clone No. | Number of ES cell-injected 8-cell stage embryos | Number of offspring mice | Number of chimeric mice | Contribution to coat color | | |
|---|---|---|---|---|---|---|---|
| | | | | | <10% | 10-50% | 50%< |
| TT2/#14+#2 | PG5 | 160 | 26 | 8 | 7 | 1 | - |
| | PG15 | 168 | 15 | 3 | 1 | 2 | - |
| | PG16 | 223 | 32 | 12 | 3 | 6 | 3 |

As a result of the transplantation of a total of 551 injected embryos, 73 offspring mice were born. Chimerism in the offsprings can be determined by the extent of TT2 cell-derived agouti coat color (dark brown) in the host embryo-derived albino coat color. Out of the 73 produced offsprings, 23 mice were recognized to have a partial agouti coat color, indicating the contribution of the ES cells.

These results show that the ES cell clones PG5, PG15 and PG16 retaining human chromosome #2 partial fragments and human chromosome #14 partial fragments maintain the ability to produce chimera, that is, the ability to differentiate into normal tissues of mouse.

### Example 20

### Detection of human antibody in sera of the chimeric mice derived from the ES cells retaining both human chromosome #2 partial fragments and human chromosome #14 partial fragments

The two KPG-15 (9 weeks old; derived from the PG-5 clone, 10% chimerism) and KPG-18 (5 weeks old; derived from the PG-5 clone, 10% chimerism) chimeric mice from Example 19 were immunized with 0.2 ml of a solution of human serum albumin (HSA, Sigma, A3782) and adjuvant (MPL+TDM Emulsion, RIBI Immunochem Research Inc.) at a HSA concentration of 0.25 mg/ml. The chimeric mice were bled just before the immunization and 8 days after that and the concentrations of human antibody *µ* and κ chains in the sera were determined by ELISA (see Example 14). Ninety six-well microtiter plates were coated with anti-human antibody κ chain goat antibody (VECTOR LABORATORIES INC., AI-3060) diluted with 50 mM carbonate-bicarbonate buffer (pH 9.6) and then a serum sample diluted with mouse serum (Sigma, M5905)-containing PBS was added. Subsequently, biotin-labeled anti-human antibody κ chain goat antibody (VECTOR LABORATORIES INC., BA-3060) was added to the plates and incubated. A complex of biotinylated horseradish peroxidase and avidin DH (VECTOR LABORATORIES, INC., Vectastain ABC Kit, PK4000) was added and incubated. After 3,3',5,5'-tetramethylbenzidine (TMBZ, Sumitomo Bakelite, ML-1120T) was added as a peroxidase substrate, enzyme activity was determined by absorbance measurement at 450 nm. Purified human IgG antibody having κ chain (Sigma, I-3889) was used as standard. The standard was diluted stepwise with mouse serum-supplemented PBS. In the case of µ chain, 96-well microtiter plates were coated with anti-human antibody µ chain mouse monoclonal antibody (Sigma, I-6385) diluted with 50 mM carbonate-bicarbonate buffer (pH 9.6) and then a serum sample was added. Subsequently, peroxidase-labeled anti-human antibody *µ* chain mouse antibody (The Binding Site Limited, MP008) was added to the plates and incubated. After TMBZ (Sumitomo Bakelite, ML-1120T) was added, enzyme activity was determined by absorbance measurement at 450 nm. Purified human IgM antibody having *µ* chain (CAPPEL, 6001-1590) was used as standard. The standard was diluted stepwise with mouse serum-supplemented PBS. As a result, both the human antibody µ and κ chains were detected in both individuals. The concentrations of these human antibodies in the sera increased after the immunization (Tables 7 and 8).

**Table 7. Concentrations of Human Antibodies in Chimeric Mouse KPG15 (ELISA)**

| | IgM (mg/l) | Igκ (mg/l) |
|---|---|---|
| Before Immunization | 0.19 | 1.6 |
| 8 Days After Immunization | 0.75 | 1.7 |

**Table 8. Concentrations of Human Antibodies in Chimeric Mouse KPG18 (ELISA)**

| | IgM (mg/l) | Igκ (mg/l) |
|---|---|---|
| Before Immunization | 0.29 | 0.57 |
| 8 Days After Immunization | 3.4 | 0.87 |

These results show that human antibody heavy and light chain genes can function in the chimeric mice derived from the ES cells retaining both human chromosome #2 partial fragments and human chromosome #14 partial fragments.

### Example 21

### Detection of anti-HSA human antibody γ chain in sera of the human chromosome #14 fragments transferred chimeric mice

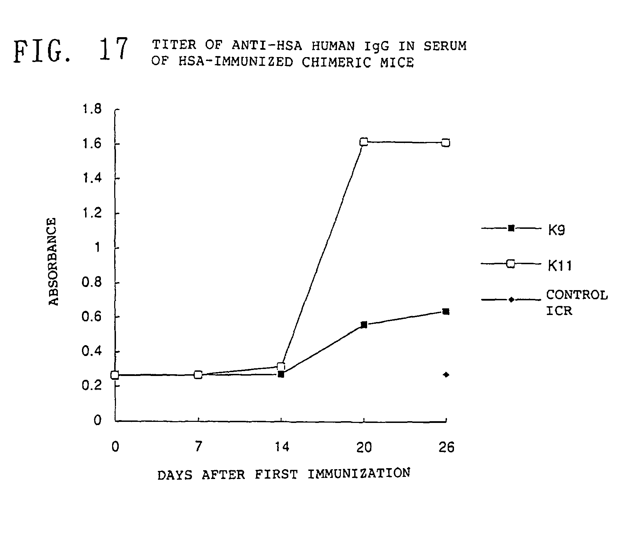

The chimeric mice retaining human chromosome #14 fragments which were produced by the same method as in Example 10 (K9 and K11: both were derived from the TT2 cell clone 3-2, with chimerisms of 50% and 30%, respectively) were immunized with HSA either 4 times at days 79, 93, 107 and 133 after birth (K9) or 3 times at days 74, 88 and 111 after birth (K11) by the same method as in Example 20. Antibodies including human γ chain against human serum albumin in the sera of the chimeric mice were detected by ELISA. Ninety six-well microtiter plates were coated with HSA (Sigma, A 3782) diluted with 50 mM carbonate-bicarbonate buffer (pH 9.6) and then a sample diluted with PBS was added. Subsequently, peroxidase-labeled anti-human IgG mouse antibody (Pharmingen, 08007E) was added to the plates and incubated. After O-phenylenediamine (OPD, Sumitomo Bakelite, ML-11300) was added as a peroxidase substrate, enzyme activity was determined by absorbance measurement at 490 nm. The titer of the anti-HSA human IgG in the sera of the chimeric mice immunized with HSA increased after the immunization. On the other hand, control ICR mouse gave a background level of the anti-HSA human IgG titer after the immunization with HSA. The results are shown in Fig. 17. In Fig. 17, the number of days after the first immunization of the chimeric mice with HSA is plotted on the horizontal axis and the absorbance at 490 nm is plotted on the vertical axis. These results show that the antibody titer of the antigen specific human IgG was increased by stimulation with the HSA antigen in the chimeric mice retaining human chromosome #14 fragments.

### Example 22

### Detection of human antibody λ chain in a serum of the human chromosome #22 fragment transferred chimeric mouse

The chimeric mouse K22-7 from Example 3 (9 weeks old; 10% chimerism) was bled and human antibody λ chain in the serum was detected by ELISA (see Example 14). Ninety six-well microtiter plates were coated with anti-human antibody λ chain goat antibody (VECTOR LABORATORIES INC., AI-3070) diluted with 50 mM carbonate-bicarbonate buffer (pH 9.6) and then a serum sample was added. Subsequently, biotin-labeled anti-human antibody λ chain goat antibody (VECTOR LABORATORIES INC., BA-3070) was added to the plates and incubated. A complex of biotinylated horseradish peroxidase and avidin DH (VECTOR LABORATORIES, INC., Vectastain ABC Kit) was added and incubated. After TMBZ (Sumitomo Bakelite, ML-1120T) was added as a peroxidase substrate, enzyme activity was determined by absorbance measurement at 450 nm. Purified human IgG antibody having λ chain (Sigma, I-4014) was used as standard. The standard was diluted stepwise with mouse serum-supplemented PBS. As a result, human antibody λ chain was detected in the chimeric mouse at a concentration corresponding to 180 ng/ml of human IgG. These results show that human antibody λ chain gene can function in the chimeric mouse retaining a human chromosome #22 fragment.

### Example 23

### Detection of human antibody κ chain in sera of the human chromosome #2 fragment transferred chimeric mice

The chimeric mouse K2-8 from Example 13 (5 weeks old; 70% chimerism) and the chimeric mice K2-3, K2-4 and K2-12 from Example 13 (9 weeks old; chimerisms was 50%, 20% and 80%, respectively) were bled and human antibody κ chain in the sera was detected by ELISA (see Example 14). Ninety six-well microtiter plates were coated with anti-human antibody κ chain goat antibody (VECTOR LABORATORIES INC., AI-3060) diluted with 50 mM carbonate-bicarbonate buffer (pH 9.6) and then a serum sample was added. Subsequently, biotin-labeled anti-human antibody κ chain goat antibody (VECTOR LABORATORIES INC., BA-3060) was added to the plates and incubated. A complex of biotinylated horseradish peroxidase and avidin DH (VECTOR LABORATORIES, INC., Vectastain ABC Kit) was added and incubated. After TMBZ (Sumitomo Bakelite, ML-1120T) was added, enzyme activity was determined by absorbance measurement at 450 nm. Purified human IgG antibody having κ chain (Sigma, I-3889) was used as standard. The standard was diluted stepwise with mouse serum-supplemented PBS. The results are shown in Table 9.

**Table 9. Concentration of Human Antibody κ Chain in Chimeric Mouse (ELISA)**

| Chimeric Mouse | Ig κ (mg/l) |
|---|---|
| K2-3 | 124 |
| K2-4 | 85 |
| K2-8 | 25 |
| K2-12 | 56 |

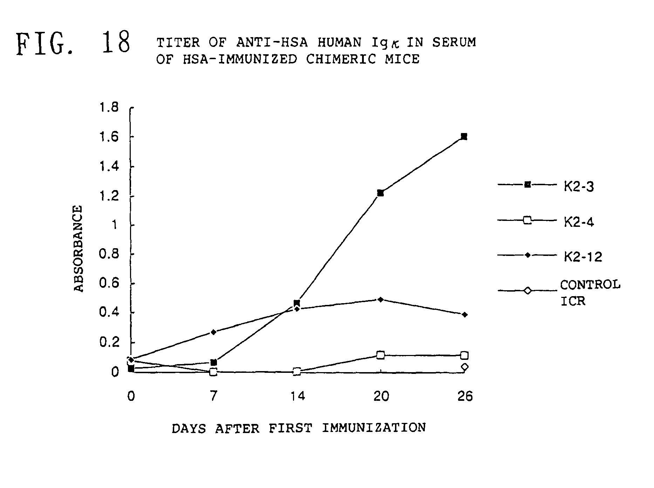

The chimeric mice K2-3 and K2-4 retaining human chromosome #2 fragments from Example 13 were immunized with HSA, 3 times at days 66, 80 and 102 after birth by the same method as in Example 20. The chimeric mouse K2-12 was immunized with HSA, 4 times at days 63, 77, 91 and 116 after birth by the same method as in Example 20. Human antibody κ chain against HSA in the sera of the chimeric mice was detected by ELISA (see Example 14). Ninety six-well microtiter plates were coated with HSA (Sigma, A 3782) diluted with 50 mM carbonate-bicarbonate buffer (pH 9.6) and then a sample was added. Subsequently, biotin-labeled anti-human antibody κ chain goat antibody (VECTOR LABORATORIES, INC., BA-3060) was added to the plates and incubated. A complex of biotinylated horseradish peroxidase and avidin DH (VECTOR LABORATORIES, INC., Vectastain ABC Kit) was added and incubated. After OPD (Sumitomo Bakelite, ML-11300) was added as a peroxidase substrate, enzyme activity was determined by absorbance measurement at 490 nm. The titer of the anti-HSA human κ chain in the sera of the chimeric mice immunized with HSA increased after the immunization. On the other hand, control ICR mouse gave a background level of the anti-HSA human κ chain titer after the immunization. The results are shown in Fig. 18. In Fig. 18, the number of days after the first immunization of the chimeric mice with HSA is plotted on the horizontal axis and the absorbance at 490 nm is plotted on the vertical axis. These results show that human antibody κ chain gene can function in the chimeric mice retaining human chromosome #2 fragments and that the antibody titer of the antigen specific human Igκ was increased by stimulation with the HSA antigen in the chimeric mice.

### Example 24

### Preparation of human antibody heavy chain (µ chain or γ chain)-producing hybridomas from the human chromosome #14 fragment-transferred chimeric mouse

The spleen was removed from the HSA-immunized chimeric mouse K9 (see Example 21) at day 136 after birth. A spleen cell was fused with a myeloma cell to produce a hybridoma. The hybridoma was produced using a myeloma cell Sp-2/0-Ag14 (Dainippon Pharmaceutical Co., Ltd., 05-554) by the method described in Toyama and Ando, "Monoclonal Antibody Experiment Procedure Manual", published by Kodansha Scientific, 1991. The cells were inoculated into a medium containing 10% ORIGEN Hybridoma Cloning Factor (HCF, Bokusui Brown) in eight 96-well plates and G418 was added after 3 days at a concentration of 1 mg/ml, followed by cultivation for 1-3 weeks. The culture supernatant was analyzed by ELISA. Ninety six-well microtiter plates were coated with anti-human *µ* chain mouse monoclonal antibody (Sigma, I-6385) diluted with 50 mM carbonate-bicarbonate buffer (pH 9.6) and a sample diluted with PBS was added. Subsequently, peroxidase-labeled anti-human µ chain mouse antibody (The Binding Site LIMITED, MP008) was added to the plates and incubated. 2,2'-Azino-di-(3-ethyl-benzothiazoline-6-sulfonate) diammonium salt (ABTS, Kirkegaard & Perry Laboratories Inc., 04-10-17) was used as a substrate to detect seven positive clones. In the detection of γ chain-producing clones, 96-well microtiter plates were coated with anti-human γ chain mouse monoclonal antibody (Sigma, I-6260) and a sample diluted with PBS was added. Subsequently, peroxidase-labeled anti-human γ chain mouse antibody (Pharmingen, 08007E) was added to the plates and incubated. ABTS (Kirkegaard & Perry Laboratories Inc., 04-10-17) was used as a substrate and two human antibody γ chain-positive clones were obtained.

### Example 25

### Preparation of human antibody light chain-producing hybridomas from the human chromosome #2 fragment-transferred chimeric mouse

The spleen was removed from the HSA-immunized chimeric mouse K2-3 (see Example 23) at day 105 after birth. A spleen cell was fused with a myeloma cell to produce a hybridoma. The hybridoma was produced using a myeloma cell P3X63Ag8.653 (Dainippon Pharmaceutical Co., Ltd., 05-565) by the method described in Toyama and Ando, "Monoclonal Antibody Experiment Procedure Manual", published by Kodansha Scientific, 1991. The cells were inoculated into a medium containing 10% HCF (Bokusui Brown) in ten 96-well plates and G418 was added after 3 days at a concentration of 1 mg/ml, followed by cultivation for 1-3 weeks. The culture supernatant was assayed by ELISA. The ELISA analysis was conducted by the same method as in Example 23 and two human antibody κ chain-positive clones were obtained.

### Example 26

### Re-marking of the G418 resistance-marked human chromosome #22 with puromycin resistance

The A9 cells retaining the G418 resistance-marked human chromosome #22 (A9/#22 γ 2) from Example 1 were re-marked with puromycin resistance by the same method as in Example 16. About 200 colonies of double-drug resistant clones obtained by electroporation of the γ 2 cells with pPGKPuro were collected as one group and three such groups (P1, P2 and P3) were used as donor cells to perform microcell transfer into wild-type mouse A9 cells. As a result, 6, 1 and 3 of double-drug resistant clones were obtained from the groups P1, P2 and P3, respectively. The clone 6-1 from group P3 was used as a microcell donor cell and a wild-type A9 cell as a recipient cell to perform a microcell transfer experiment (see Example 18). As a result, the human chromosome #22 was confirmed to have been further marked with a puromycin resistance gene. The preparation of microcells and the fusion with A9 cells were conducted by the same methods as in Example 1. As a result, twenty eight G418 resistant colonies appeared 11 days after the microcell transfer. After the medium for these colonies was changed to one containing 8 µg/ml puromycin, these colonies were cultured for 3 days to give 21 (75%) viable colonies. In many cases of microcell fusion, only one or few chromosomes are transferred into a recipient cell. Hence, cotransfer of both the resistance genes at a high frequency shows that the G418 resistance-labeled chromosome #22 retained in the 6-1 clone was marked with the puromycin resistance gene.

### Example 27

### Preparation and Sequencing of cDNA of a human antibody heavy chain variable region from the human antibody heavy chain-producing hybridoma

Among the human antibody heavy chain (IgM)-producing hybridomas obtained in Example 15, H4B7 (HSA-specific) and H8F9 (non-specific) hybridomas were selected. Total RNAs were obtained from these hybridomas using ISOGEN (Nippon Gene). The synthesis of cDNA from 5*µ*g each of the total RNAs was conducted with a Ready-To-Go T-primed 1st strand Kit (Pharmacia Co.). Using the resulting cDNA and the following primers prepared with reference to Larrick et al., BIO/TECHNOLOGY, 7, 934-, 1989; Word et al., Int. Immunol., 1, 296-, 1989, PCR was performed to amplify a human antibody heavy chain variable region.
CM1 (human IgM constant region) : 5'-TTGTATTTCCAGGAGAAAGTG (SEQ ID NO: 45)
CM2 (ditto) : 5'-GGAGACGAGGGGGAAAAGGG (SEQ ID NO:46)
HS1 (human heavy chain variable region) : 5'-ATGGACTGGACCTGGAGG(AG) TC(CT)TCT(GT)C (SEQ ID NO:47) (a mixture of 8 sequences)
HS2 (ditto) : 5'-ATGGAG(CT)TTGGGCTGA(GC)CTGG(GC)TTT(CT)T (SEQ ID NO:48) (a mixture of 16 sequences)
HS3 (ditto) : 5'-ATG(AG)A(AC)(AC)(AT)ACT(GT)TG(GT)(AT)(GCT)C(AT)(CT) (GC)CT(CT)CTG (SEQ ID NO:49) (a mixture of 6144 sequences)
* ( ) means that any one of the bases therein should be selected.

In both cases of the H4B7 and H8F9 hybridomas, the first run of PCR was performed by using three kinds of primer combinations of HS1xCM1, HS2xCM1 and HS3xCM1 in 40 cycles at 94 °C for 1 minute, 50 °C for 2 minutes and 72 °C for 3 minutes with a Thermal Cycler 140 (Perkin-Elmer Corp.). The PCR products were amplified again under the same temperature conditions in 30 cycles using HS1xCM2, HS2xCM2 and HS3xCM2 primers, respectively. The amplification products were electrophoresed on a 1.5% agarose gel and detected by staining with ethidium bromide. As a result, an amplification product of about 490 bp was detected with the HS3xCM2 primer in the case of the H4B7 hybridoma. In the case of the H8F9 hybridoma, a slight band was detected at the same site with the HS3xCM2 primer. The band in the case of H8F9 was amplified again with the HS3xCM2 primer in 30 cycles under the same temperature conditions as above. As a result, the amplification product was detected as a very intensive signal. These PCR products were cloned into a pBlueScriptII SK+ (Stratagene Ltd.) at a SmaI site in accordance with the method described in Ishida et al., "Gene Expression Experiment Manual", published by Kodansha Scientific, 1995. Among the amplification product-inserted plasmids, plasmids #2, #3, #4(H4B7), #11, #13 and #14 (H8F9) were selected and the nucleotide sequences of the amplification products were determined with a Fluorescence Autosequencer (Applied Biosystems Inc.). As a result of the comparison of the obtained nucleotide sequences or deduced amino acid sequences with those of known human antibody VH region (Marks et al., Eur. J. Immunol. 21, 985-, 1991) and JH region (Ravetch et al., Cell, 27, 583-, 1981), it was revealed that both the H4B7 and H8F9 hybridomas contained a combination of genes for VH4 family and JH2. These results show that the chimeric mouse retaining human chromosome #14 partial fragment produced a complete functional human antibody heavy chain protein.

### Example 28

### Preparation and Sequencing of cDNA of human antibody κ chain from the spleen of the human antibody κ chain-expressing chimeric mouse

In the same manner as in Example 5, cDNA was prepared from the spleen of the chimeric mouse K2-8 from Example 13 which was confirmed to express human antibody κ chain in Example 23. Using the resulting cDNA and the following primers prepared with reference to Larrick et al. , BIO/TECHNOLOGY, 7, 934-, 1989; Whitehurst et al., Nucleic Acids Res., 20, 4929-, 1992, PCR was performed to amplify human antibody κ chain variable region. cDNA from the liver of the chimeric mouse K2-8 and cDNA from the spleen of the chimeric mouse K3-2-2 derived from the TT2/#14 3-2 clone (see Example 10) were used as negative controls.
KC2 (human Ig κ chain constant region) : 5'-CAGAGGCAGTTCCAGATTTC (SEQ ID NO:50)
KC3 (ditto) : 5'-TGGGATAGAAGTTATTCAGC (SEQ ID NO:51)
KVMIX (human Ig κ chain variable region) :
5'-ATGGACATG(AG)(AG)(AG)(AGT)(CT)CC(ACT)(ACG)G(CT)(GT)CA(CG)CTT (SEQ ID NO:52) (a mixture of 3456 sequences)
* ( ) means that any one of the bases therein should be selected.

PCR was performed by using primer combinations of KVMIXxKC2 and KVMIXxKC3 in 40 cycles at 94 °C for 15 seconds, 55°C for 15 seconds and 72°C for 20 seconds with a Thermal Cycler 9600 (Perkin-Elmer Corp.). The amplification products were electrophoresed on a 1.5% agarose gel and detected by staining with ethidium bromide. As a result, expected amplification products of about 420 bp (KC2) and about 450 bp (KC3) were detected. In the case of the two negative controls, no specific amplification product was detected. These amplification products were cloned into a pBlueScriptII SK⁺ (Stratagene Ltd.) at a SmaI or EcoRI site in accordance with the method described in Ishida et al., "Gene Expression Experiment Manual", published by Kodansha Scientific, 1995. Among the amplification product-inserted plasmids, VK-#1 clone derived from the KVMIXxKC2 primers was selected and the nucleotide sequence of the amplification product was determined with a Fluorescence Autosequencer (Applied Biosystems Inc.). Since the obtained nucleotide sequence did not contain a termination codon at any site between an initiation codon and a constant region of human Igκ chain, the cloned amplification products are believed to encode a variable region of functional human Ig κ chain. As a result of the comparison of the obtained nucleotide sequences with those of known human antibody V_{κ} region (Klein et al., Eur. J. Immunol. 23, 3248-, 1993) and J_{κ} region (Whitehurst et al., supra), it was revealed that the VK-#1 clone contained a combination of genes for V_{κ} 3 family and J_{κ} 4. These results show that the chimeric mouse retaining human chromosome #2 partial fragment produced a complete functional human antibody κ chain protein.

### Example 29

### Detection and quantitation of human antibody γ chain subclasses and µ chain in sera of the chimeric mice retaining human chromosome #14 fragment

The chimeric mice K15A and K16A from Example 10 (derived from the 1-4 clone, with chimerism of 70% and 50%, respectively) of 11 weeks after birth were bled and human antibody γ chain subclasses and µ chain in the sera were detected by the same ELISA method as in Example 14.

### Quantative Determination of human IgG1

Ninety six-well microtiter plates were coated with anti-human IgG antibody (Sigma, I-6260) diluted with PBS. A serum sample was added. Subsequently, peroxidase-labeled anti-human IgG1 antibody (Pharmingen, 08027E) was added to the plates and incubated. After TMBZ (Sumitomo Bakelite, ML-1120T) was added, enzyme activity was determined by absorbance measurement at 450 nm. Purified human IgG1 antibody (Sigma, I-3889) was used as standard. The standard was diluted stepwise with mouse serum-supplemented PBS.

### Quantative Determination of human IgG2

Ninety six-well microtiter plates were coated with anti-human IgG2 antibody (Sigma, I-9513) diluted with PBS. A serum sample was added. Subsequently, peroxidase-labeled anti-human IgG antibody (Sigma, A-0170) was added to the plates and incubated. After TMBZ (Sumitomo Bakelite, ML-1120T) was added, enzyme activity was determined by absorbance measurement at 450 nm. Purified human IgG2 antibody (Sigma, I-4139) was used as standard. The standard was diluted stepwise with mouse serum-supplemented PBS.

### Quantative Determination of human IgG3

Anti-human IgG3 antibody (Sigma, I-7260) was diluted with 100 mM glycine-HCl buffer (pH 2.5) and incubated for 5 minutes at room temperature, followed by 10-fold dilution with 100 mM phosphate buffer (pH 7.0). Ninety six-well microtiter plates were coated with the anti-human IgG3 antibody solution. A serum sample was added. Subsequently, peroxidase-labeled anti-human IgG antibody (Pharmingen, 08007E) was added to the plates and incubated. After TMBZ (Sumitomo Bakelite, ML-1120T) was added, enzyme activity was determined by absorbance measurement at 450 nm. Purified human IgG3 antibody (Sigma, I-4389) was used as standard. The standard was diluted stepwise with mouse serum-supplemented PBS.

### Quantative Determination of human IgG4

Anti-human IgG4 antibody (Sigma, I-7635) was diluted with 100 mM glycine-HCl buffer (pH 2.5) and incubated for 5 minutes at room temperature, followed by 10-fold dilution with 100 mM phosphate buffer (pH 7.0). Ninety six-well microtiter plates were coated with the anti-human IgG3 antibody solution. A serum sample was added. Subsequently, peroxidase-labeled anti-human IgG antibody (Pharmingen, 08007E) was added to the plates and incubated. After TMBZ (Sumitomo Bakelite, ML-1120T) was added, enzyme activity was determined by absorbance measurement at 450 nm. Purified human IgG4 antibody (Sigma, I-4639) was used as standard. The standard was diluted stepwise with mouse serum-supplemented PBS.

### Quantative Determination of human IgM

Ninety six-well microtiter plates were coated with anti-human µ chain mouse monoclonal antibody (Sigma, I-6385) diluted with PBS. A serum sample was added. Subsequently, peroxidase-labeled anti-human µ chain mouse antibody (The Binding Site Limited, MP008) diluted with mouse serum (Sigma, M5905)-supplemented PBS was added to the plates and incubated. After TMBZ (Sumitomo Bakelite, ML-1120T) was added as a peroxidase substrate, enzyme activity was determined by absorbance measurement at 450 nm. Purified human IgM having *µ* chain (CAPPEL, 6001-1590) was used as standard. The standard was diluted stepwise with mouse serum (Sigma, M5905)-supplemented PBS.

The results are shown in Table 10. All the subclasses IgG1, IgG2, IgG3 and IgG4, and IgM were detected in the two chimeric mice K15A and K16A.

**Table 10. Concentrations of Human antibody IgG Subclasses and IgM in the Chimeric Mice (ELISA)**

| Chimeric mouse | IgG1 | IgG2 | IgG3 | IgG4 | IgM (mg/l) |
|---|---|---|---|---|---|
| K15A | 2.25 | 1.96 | 0.17 | 0.43 | 7.09 |
| K16A | 0.30 | 0.69 | 0.10 | 0.07 | 0.87 |

### Example 30

### Preparation of mouse ES cell clones (TT2) retaining human chromosome #22

The cell clone 6-1 (A9/#22, G418 and puromycin resistant) from Example 26 was used as a chromosome donor cell for the preparation of mouse ES cell (TT2) retaining human chromosome #22. A wild-type TT2 cell line (see Example 9) was used as a chromosome recipient cell. The microcell fusion and the selection of puromycin resistant clones were conducted by the same procedures as in the selection of G418 resistant clones in Example 9 except that the concentration of puromycin was 0.75 *µ*g/ml. The frequency of the appearance of the puromycin resistant clones was 1-2 per 10⁷ of TT2 cells. The puromycin resistant clones were stored frozen and genomic DNA was prepared by the same methods as in Example 2. The retention of human chromosome #22 in the puromycin resistant clone PG22-1 was confirmed by the methods described in (1) and (2) below.

### (1) PCR analysis

Genomic DNA of the puromycin resistant clone was used as a template in PCR amplification. Among the genes on human chromosome #22 (Genetic Maps, supra), ten primers whose presence in the A9/#22 clone was confirmed in Example 2 were used in the PCR amplification. All the markers which existed in the A9/#22 clone (see Example 2) were detected.

### (2) Southern blot analysis

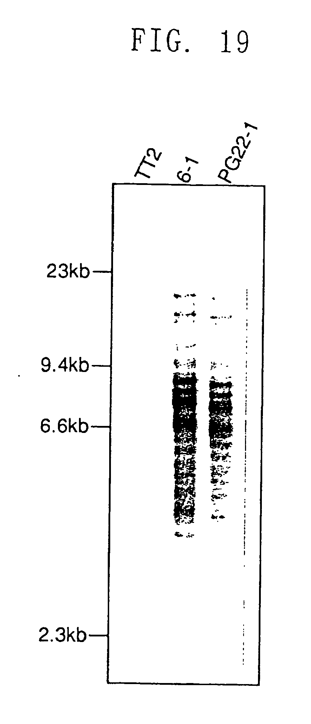

In accordance with the same method as described in Example 2 using human L1 sequence as a probe, Southern blot analysis was conducted with genomic DNAs obtained from wild-type TT2 (negative control), the chromosome donor cell 6-1 and the puromycin resistant TT2 cell clone PG22-1. The results are shown in Fig. 19. In Fig. 19, the molecular weights of DNAs are shown at the left side. The band pattern of the PG22-1 clone was equivalent to that of the 6-1 cell and the signal intensities were the same. Hence, it was confirmed that chromosome #22 in the 6-1 cell had been transferred certainly into the PG22-1 clone.

These experiments demonstrate that the puromycin resistant TT2 cell clone PG22-1 retained the whole or the most part of human chromosome #22.

### Example 31

### Production of chimeric mice from the mouse ES cells (TT2) retaining human chromosome #22

The cells in a frozen stock of the puromycin resistant TT2 cell clone PG22-1 from Example 30 which was confirmed to retain human chromosome #22 were thawed, started to culture and injected into 8-cell stage embryos obtained by mating ICR or MCH(ICR) male and female mice (CREA JAPAN, INC.); the injection rate was 10-12 cells per embryo. The embryos were cultured in a medium for ES cells (see Example 9) overnight to develop to blastocysts. Two and a half day after a foster mother ICR mouse was subjected to a pseudopregnant treatment, about ten of the injected embryos were transplanted to each side of the uterus of the foster mother. The results are shown in Table 11.

**Table 11. Production of chimeric mice from the TT2 cell clone retaining human chromosome #22**

| ES cell clone/human chromosome | Puromycin resistant clone No. | Number of ES cell-injected 8-cell stage embryos | Number of offspring mice | Number chimeric mice | Contribution to coat color | | |
|---|---|---|---|---|---|---|---|
| | | | | | <20% | 20-50% | 50-80% |
| TT2/#22 | PG22-1 | 266 | 36 | 8 | 4 | 1 | 3 |

As a result of the transplantation of a total of 266 injected embryos, 36 offspring mice were born. Chimerism in the offsprings can be determined by the extent of TT2 cell-derived agouti coat color (dark brown) in the host embryo-derived albino coat color. Out of the 36 produced offsprings, 8 mice were recognized to have a partial agouti coat color, indicating the contribution of the ES cells.

These results show that the ES cell clone (derived from TT2, PG22-1) retaining human chromosome #22 maintain the ability to produce chimera, that is, the ability to differentiate into normal tissues of mouse.

### Example 32

### Detection and quantitation of human antibody λ chain in sera of the chimeric mice retaining human chromosome #22

The concentration of human antibody λ in the sera of the chimeric mice KPG22-1, 2 and 3 from Example 31 was determined by ELISA in accordance with the same procedure as in Example 14. The chimeric mice of 2 months after birth were bled and human antibody λ chain in the sera was detected by ELISA. Ninety six-well microtiter plates were coated with anti-human immunoglobulin λ chain antibody (VECTOR LABORATORIES INC., IA-3070) diluted with PBS and then a serum sample was added. Subsequently, biotin-labeled anti-human immunoglobulin λ chain antibody (VECTOR LABORATORIES INC., BA-3070) was added to the plates and incubated. A complex of biotinylated horseradish peroxidase and avidin DH (VECTOR LABORATORIES, INC., Vectastain ABC Kit) was added and incubated. After TMBZ (Sumitomo Bakelite, ML-1120T) was added, enzyme activity was determined by absorbance measurement at 450 nm. Purified human IgM antibody having λ chain (Dainippon Pharmaceutical Co., Ltd., U13200) was used as standard. The standard was diluted stepwise with mouse serum-supplemented PBS. The results are shown in Table 12. These results show that human antibody λ chain gene can function in the chimeric mice retaining human chromosome #22.

**Table 12. Concentration of Human Antibody λ Chain in Chimeric Mice (ELISA)**

| Chimeric Mouse | %Chimerism | Igλ (mg/l) |
|---|---|---|
| KPG22-1 | 50 | 12 |
| KPG22-2 | 50 | 18 |
| KPG22-3 | 20 | 24 |

### Example 33

### Detection of anti-human HSA human antibody λ chain in a serum of the human chromosome #22 transferred chimeric mouse

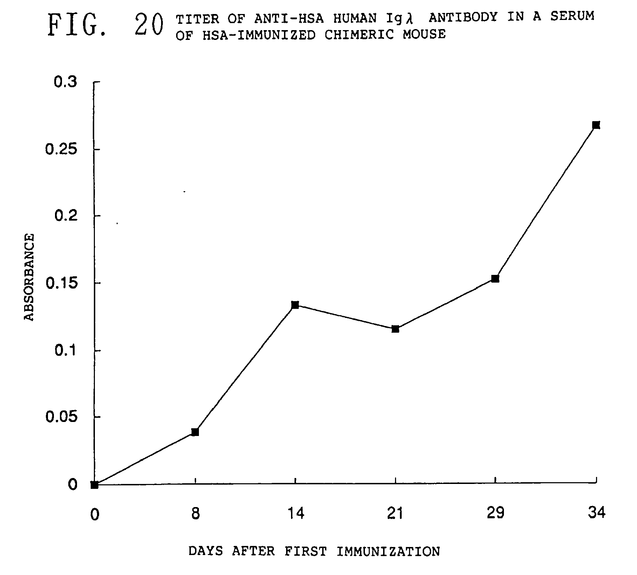

The chimeric mouse KPG22-3 from Example 31 was immunized with HSA, 3 times at days 79, 94 and 110 after birth by the same method as in Example 20. Human antibody λ chain in the serum of the chimeric mouse was detected by ELISA in accordance with the same procedure as in Example 14. Ninety six-well microtiter plates were coated with HSA (Sigma, A 3782) diluted with 50 mM carbonate-bicarbonate buffer (pH 9.6) to a concentration of 5 *µ* g/ml and a serum sample was added. Biotinylated anti-human Ig λ antibody (VECTOR LABORATORIES INC., BA-3070) was added. Subsequently, a complex of biotinylated-horseradish peroxidase and avidin DH (VECTOR LABORATORIES, INC., Vectastain ABC Kit) was added to the plates and incubated. After TMBZ (Sumitomo Bakelite, ML-1120T) was added, enzyme activity was determined by absorbance measurement at 450 nm. The titer of the anti-HSA human λ chain in the serum of the chimeric mouse increased after the immunization. On the other hand, control ICR mouse gave a background level of the anti-HSA human λ chain titer after the immunization with HSA. The results are shown in Fig. 20. In Fig. 20, the number of days after the first immunization of the chimeric mouse with HSA is plotted on the horizontal axis and the absorbance at 450 nm is plotted on the vertical axis. These results show that human antibody λ chain gene can function in the chimeric mouse retaining human chromosome #22 and that the antibody titer of the antigen specific human Igλ was increased by stimulation with the HSA antigen.

### Example 34

### Preparation of human antibody light chain-producing hybridomas from the human chromosome #22 transferred chimeric mouse

The spleen was removed from the mouse KPG22-3 (see Example 33) at day 113 after birth by the same method as in Example 25. A spleen cell was fused with a myeloma cell to produce a hybridoma. The hybridoma was produced using a myeloma cell SP-2/0-Ag14 (Dainippon Pharmaceutical Co., Ltd., 05-554) by the method described in Toyama and Ando, "Monoclonal Antibody Experiment Manual", published by Kodansha Scientific, 1991. The cells were inoculated into a medium containing 10% HCF (Air Brown) in five 96-well plates and cultured for 1-3 weeks. The supernatant of the culture solution in colony-positive wells was analyzed by ELISA. The ELISA analysis was conducted by the same method as in Example 33 and four human antibody λ chain-positive clones were obtained.

### Example 35

### Preparation of mouse ES cell clones retaining both a human chromosome #22 partial fragment and a human chromosome #14 partial fragment

The 6-1 cell clone from Example 26 (A9/#22, G418 and puromycin resistant) was used as a chromosome donor cell for the preparation of mouse ES cells retaining both a human chromosome #22 partial fragment and a human chromosome #14 partial fragment. The G418 resistant TT2 cell clone 1-4 retaining a human chromosome #14 partial fragment from Example 9 was used as a chromosome recipient cell. The experiment of microcell fusion and the selection of puromycin resistant cells were carried out by the same methods as in the selection of the G418 resistant clones in Example 9 except that the concentration of puromycin was 0.75 *µ*g/ml. As a result, the frequency of the appearance of the puromycin resistant clones was 1-2 per 10⁷ of 1-4 cells. The retention of G418 resistance in the puromycin resistant clones was confirmed from the fact that these clones were grown in the presence of 300 *µ*g/ml G418. The double-drug resistant clones were stored frozen and genomic DNAs were prepared by the same methods as in Example 2. The retention of human chromosome #22 and a human chromosome #14 partial fragment in the double-drug resistant clone PG22-5 was confirmed by PCR analysis. With genomic DNA of the double-drug resistant clone used as a template, PCR amplification was conducted using primers whose presence on chromosome #22 was confirmed in Example 2 (A9/#22) and primers whose presence on chromosome #14 was confirmed in Example 9 (TT2/#14 1-4); as a result, three markers (D22S275, D22S315 and Igλ) of the ten markers on chromosome #22 and all of the markers on chromosome #14 in the TT2/#14 1-4 clone were detected.

These experiments demonstrate that the obtained double-drug resistant TT2 cell clone retained both a human chromosome #22 partial fragment and a human chromosome #14 partial fragment.

### Example 36

### Production of the chimeric mouse from the mouse ES cell clone retaining both a human chromosome #22 partial fragment and a human chromosome #14 partial fragment

The cells in a frozen stock of the G418 and puromycin double-resistant TT2 cell clone PG22-5 from Example 35 which was confirmed to retain a human chromosome #22 partial fragment and a human chromosome #14 partial fragment were thawed, started to culture and injected into 8-cell stage embryos obtained by mating ICR or MCH(ICR) male and female mice (CREA JAPAN, INC.); the injection rate was 10-12 cells per embryo. The embryos were cultured in a medium for ES cells (see Example 9) overnight to develop to blastocysts. Two and a half day after a foster mother ICR mouse was subjected to a pseudopregnant treatment, about ten of the injected embryos were transplanted to each side of the uterus of the foster mother. The results are shown in Table 13.

**Table 13. Production of the chimeric mouse from the mouse ES cell clone retaining both a human chromosome #22 partial fragment and a human chromosome #14 partial fragment**

| ES cell clone/human chromosome | Double-drug resistant clone No. | Number of ES cell-injected 8-cell stage embryos | Number of offspring mice | Number of chimeric mice | Contribution to coat color | | |
|---|---|---|---|---|---|---|---|
| | | | | | <20% | 20-50% | 50-80% |
| TT2/#22+#14 | PG22-5 | 302 | 16 | 5 | 3 | 2 | 0 |

As a result of the transplantation of a total of 302 injected embryos, 16 offspring mice were born. Chimerism in the offsprings can be determined by the extent of TT2 cell-derived agouti coat color (dark brown) in the host embryo-derived albino coat color. Out of the 16 produced offsprings, 5 mice were recognized to have a partial agouti coat color, indicating the contribution of the ES cell.

These results show that the ES cell clone PG22-5 retaining a human chromosome #22 partial fragment and a human chromosome #14 partial fragment maintains the ability to produce chimera, that is, the ability to differentiate into normal tissues of mouse.

### Example 37

### Detection of human antibody λ chain and µ chain in sera of the chimeric mice derived from the ES cells retaining both a human chromosome #22 partial fragment and a human chromosome #14 partial fragment

The chimeric mice KPG22-9, 10 and 12 from Example 36 were immunized with HSA. The chimeric mice KPG22-9 and 10 were immunized 11 weeks after birth and bled 2 weeks after the immunization. The chimeric mouse KPG22-12 was immunized twice at 7 and 11 weeks after birth and bled 2 weeks after the second immunization.

A serum human antibody µ chain, a serum human antibody λ chain, and a serum antibody having both human antibody λ and µ chains were detected by ELISA in accordance with Example 14.

For the detection of complete human antibody molecules, 96-well microtiter plates were coated with anti-human immunoglobulin λ chain antibody (Kirkegaard & Perry Laboratories Inc., 01-10-11) diluted with PBS and a serum sample was added. Subsequently, peroxidase-labeled anti-human immunoglobulin µ chain antibody (The Binding Site Limited, MP008) was added to the plates and incubated. After TMBZ (Sumitomo Bakelite, ML-1120T) was added as a peroxidase substrate, enzyme activity was determined by absorbance measurement at 450 nm. Purified human IgM antibody having λ chain (Dainippon Pharmaceutical Co., Ltd., U13200) was used as standard. The standard was diluted stepwise with mouse serum-supplemented PBS. Human antibody µ and λ chains were detected and determined quantitatively by ELISA in the same manner as in Examples 29 and 32. The results are shown in Table 14.

**Table 14. Concentrations of Human Antibodies in Chimeric Mice (ELISA)**

| ES clone | Chimeric mouse | Chimerism (%) | IgM (mg/l) | Igλ (mg/l) | IgM, λ (mg/l) |
|---|---|---|---|---|---|
| PG22-5 | KPG22-9 | 30 | 2.54 | 9.9 | 0.043 |
| PG22-5 | KPG22-10 | 5 | 4.96 | 21.5 | 0.333 |
| PG22-5 | KPG22-12 | 40 | 3.71 | 7.0 | 0.048 |
| 3-2 | K9 | 50 | 6.66 | - | < 0.003 |
| PG22-1 | KPG22-2 | 50 | - | 17.6 | < 0.003 |

Both λ and µ chains were detected in the chimeric mice. An antibody molecule having both human antibody µ and λ chains was detected. These results show: the human antibody λ chain gene and human antibody µ chain gene can function at the same time in the chimeric mice derived from the ES cells retaining human chromosome #22 partial fragments and human chromosome #14 partial fragments; and a complete antibody containing both human heavy and light chains was produced in part of the B cells.

The control mice, that is, the chimeric mouse K9 retaining only human chromosome #14 from Example 10 and the chimeric mouse KG22-2 retaining only human chromosome #22 from Example 31, gave background levels of an antibody having both human antibody λ and µ chains in the sera. It was confirmed that in these detection systems, only a complete antibody molecule having human λ and *µ* chains was detected.

### Example 38

### Detection of human antibody having human κ and µ chains in sera of the chimeric mice derived from the ES cells retaining both human chromosome #2 partial fragments and human chromosome #14 partial fragments

The chimeric mouse KPG-15 (derived from the TT2ES clone PG5, 10% chimerism) was immunized during 2-3 months after birth 3 times with 0.2 ml of a solution of human serum albumin (HSA, Sigma, A3782) and adjuvant (MPL+TDM Emulsion, RIBI Immunochem Research Inc.) in PBS at a HSA concentration of 0.25 mg/ml and bled (see Example 15). The chimeric mouse KPG-26 (derived from the TT2ES clone PG6, 40% chimerism) of 6 weeks after birth was bled. The concentration of a complete human antibody molecule in the sera was determined by ELISA in accordance with Example 14. Ninety six-well microtiter plates were coated with anti-human immunoglobulin κ chain antibody (Kirkegaard & Perry Laboratories Inc., 01-10-10) diluted with PBS, and a serum sample was added. Subsequently, peroxidase-labeled anti-human immunoglobulin µ chain antibody (The Binding Site Limited, MP008) was added to the plates and incubated. After TMBZ (Sumitomo Bakelite, ML-1120T) was added as a peroxidase substrate, enzyme activity was determined by absorbance measurement at 450 nm. Purified human IgM antibody having κ chain (CAPPEL, 6001-1590) was used as standard. The standard was diluted stepwise with mouse serum-supplemented PBS. The concentrations of κ chain and µ chain were determined by the same method as in Example 20. The results are shown in Table 15.

**Table 15. Concentrations of Human Antibodies in Chimeric Mice (ELISA)**

| ES clone | Chimeric mouse | Chimerism (%) | IgM (mg/l) | Ig κ (mg/l) | IgM, κ (mg/l) |
|---|---|---|---|---|---|
| PG-5 | KPG15 | 10 | 0.18 | 1.01 | 0.075 |
| PG-6 | KPG26 | 40 | 1.52 | 1.26 | 0.018 |
| 3-2 | K9 | 50 | 6.66 | - | < 0.002 |
| 5-1 | K2-9 | 40 | - | 135 | < 0.002 |

A antibody molecule having both human antibody µ and κ chains was detected. The control mice, that is, the chimeric mouse K9 retaining only human chromosome #14 from Example 10 and the chimeric mouse K2-9 retaining only human chromosome #2 from Example 13, gave background levels ( < 0.002 mg/ml) of an antibody having human antibody κ and µ chains in the sera. These results show: the human antibody κ chain gene and human antibody *µ* chain gene can function at the same time in the chimeric mice derived from the ES cells retaining both human chromosome #2 partial fragments and human chromosome #14 partial fragments; and a complete antibody molecule containing both human heavy and light chains was produced in part of the B cells.

### Example 39

### Preparation of mouse ES cell clone (TT2F, XO) retaining a human chromosome #2 partial fragment

The cell clone PG1 from Example 16 was used as a chromosome donor cell for the preparation of a mouse ES cell (XO) retaining a human chromosome #2 partial fragment. A TT2F cell (purchased from Lifetec Oriental Co.) having a karyotype of (39, XO), which was reported to differentiate efficiently into an oocyte in chimeric mice (Shinichi Aizawa, "Biomanual Series 8, Gene Targeting" published by Yodosha, 1995), was used as a chromosome recipient cell. The experiment of microcell fusion and the selection of puromycin resistant cells were carried out by the same methods as in the selection of the G418 resistant clones in Example 9 except that the concentration of puromycin was 0.75*µ* g/ml. The frequency of the appearance of the puromycin resistant clones was 5 per 10' of TT2F cells. The puromycin resistant clones were stored frozen and genomic DNAs were prepared from the clones by the same methods as in Example 2. The retention of human chromosome #2 partial fragments in the drug resistant clones P-20 and P-21 was confirmed by PCR analysis. As a result of PCR amplification using genomic DNAs of the drug resistant clones as templates and three kinds of primers Cκ, FABP1 and Vκ 1-2 whose presence in the A9/#2 W23 clone was confirmed in Example 1, all of the three primers gave expected amplification products in both of the two clones.

These experiments demonstrate that the obtained puromycin resistant ES cell clone (TT2F, XO) retained a human chromosome #2 partial fragment.

### Example 40

### Production of the chimeric mice from the mouse ES cell clone (TT2F, XO) retaining a human chromosome #2 partial fragment

The cells in a frozen stock of the puromycin resistant TT2F cell clone P-21 from Example 39 which was confirmed to retain a human chromosome #2 partial fragment were thawed, started to culture and injected into 8-cell stage embryos obtained by mating ICR or MCH(ICR) male and female mice (CREA JAPAN, INC.); the injection rate was 10-12 cells per embryo. The embryos were cultured in a medium for ES cells (see Example 9) overnight to develop to blastocysts. Two and a half day after a foster mother ICR mouse was subjected to a pseudopregnant treatment, about ten of the injected embryos were transplanted to each side of the uterus of the foster mother. The results are shown in Table 16.

**Table 16. Production of the chimeric mice from the TT2F cell clone retaining a human chromosome #2 partial fragment**

| ES cell clone/human chromosome | Puromycin resistant clone No. | Number of ES cell- injected 8-cell stage embryos | Number of offspring mice | Number of chimeric mice | Contribution to coat color | | | |
|---|---|---|---|---|---|---|---|---|
| | | | | | <20% | 20-50% | 50-90% | 100% |
| TT2F/#2fg. | P-21 | 141 | 20 | 9 | 0 | 2 | 3 | 4 |

As a result of the transplantation of a total of 141 injected embryos, 20 offspring mice were born. Chimerism in the offsprings can be determined by the extent of TT2F cell-derived agouti coat color (dark brown) in the host embryo-derived albino coat color. Out of the 20 produced offsprings, 9 mice were recognized to have a partial agouti coat color, indicating the contribution of the ES cell. Four of the 9 mice were chimeric mice having a full agouti coat color from the ES cells.

These results show that the ES cell clone P-21 retaining a human chromosome #2 partial fragment maintains the ability to produce chimera, that is, the ability to differentiate into normal tissues of mouse.

### Example 41

### Detection and quantitative determination of human antibody κ chain in sera of the chimeric mice derived from the TT2F clone retaining a human chromosome #2 partial fragment

The chimeric mice K2-1F, 2F, 3F and 4F (derived from the P-21 clone, 100% chimerism) from Example 40 of about 1 month after birth were bled and the concentration of human antibody κ chain in the sera was determined quantitatively by ELISA in the same manner as in Example 20.

The results are shown in Table 17. It was confirmed that the human antibody κ chain gene could function in the chimeric mice when the TT2F was used as an ES cell.

**Table 17. Concentration of Human Antibody κ Chain in Chimeric Mice (ELISA)**

| Chimeric mouse | %Chimerism | Ig κ (mg/l) |
|---|---|---|
| K2-1F | 100 | 66 |
| K2-2F | 100 | 156 |
| K2-3F | 100 | 99 |
| K2-4F | 100 | 20 |

### Example 42

### Confirmation of the retention of human chromosome in progenies of the chimeric mice derived from the mouse ES cell (TT2F, XO) retaining a human chromosome #2 partial fragment

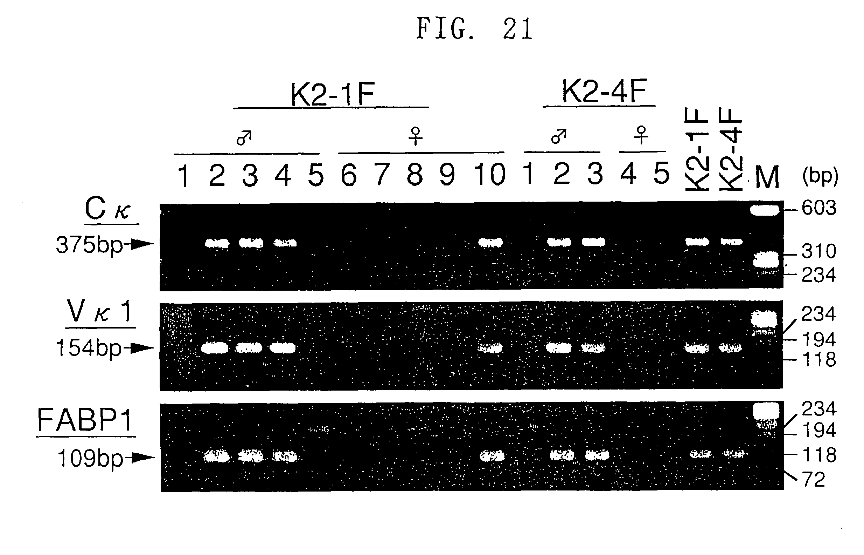

Examination was made as to whether ES cell-derived progenies would be reproduced by mating the female chimeric mice K2-1F and K2-4F (both were of 100% chimerism in coat color) from Example 40 with ICR male mice. In such a mating, offspring mice of an agouti coat color should be reproduced from the oocytes derived from the TT2F cell (agouti coat color, dominant) in the chimeric mice and offspring mice of an albino coat color should be reproduced from oocytes derived from ICR if the oocytes are fertilized with the sperms from ICR male mice (albino, recessive). All the viable offspring mice (K2-1F, 10 mice and K2-4F, 5 mice) obtained by one mating of the respective combinations had an agouti coat color which derived from the ES cells. The retention of human chromosome fragments in genomic DNAs prepared from the tails of the offspring mice was examined by a PCR method. As a result of the PCR amplification using three kinds of primers whose presence in the P-21 clone (see Example 39) was confirmed, the presence of these three markers was confirmed in 4 out of the ten mice from K2-1F and in 2 out of the five mice from K2-4F. The results of the PCR of these 15 offspring mice are shown in Fig. 21. In Fig. 21, markers (φ) X174/HaeIII fragment, Nippongene) and the DNA molecular weights of main bands are shown at the right side and the lengths of expected products of amplification with the respective primers are shown by arrows at the left side. At the right side, the results with tail-derived DNA of the mother chimeric mice K2-1F and K2-4F (positive controls) are shown. These results show that the TT2 cell clone P-21 differentiated into functional oocyte in the chimeric mice and that a human chromosome #2 partial fragment was transmitted to offsprings through oocyte.

### Example 43

### Confirmation of the retention of human chromosome in progenies of the chimeric mice derived from the mouse ES cell (TT2, XY) retaining a human chromosome #2 partial fragment

Examination was made as to whether ES cell-contributed offspring mice would be produced by mating K2-18 (70% chimeric male mouse from Example 13) with K2-19 (60% chimeric female mouse of Example 13) or non-chimeric female littermates. Since TT2 cell has the karyotype of (40, XY), it may differentiate into a functional sperm in the male chimeric mouse K2-18. If this is the case, offspring mice of an agouti coat color should be reproduced from ICR (albino, recessive)-derived oocytes fertilized with sperms from TT2 cell (agouti color, dominant) in the chimeric mice. While a total of 110 viable offspring mice were obtained by the mating, ten had an agouti coat color which derived from the ES cells. The retention of human chromosome fragments in genomic DNAs prepared from the tails of 7 out of the ten offspring mice of an agouti coat color was examined by a PCR method. As a result of PCR amplification using two kinds of primers Cκ and FABP1 whose presence in the 5-1 clone (TT2/#2fg.. Example 12) was confirmed and primer V κ 1-2 which was shown in Example 1, the presence of all of the three markers was confirmed in 2 out of the seven mice. These results show that the TT2 cell clone 5-1 retaining a human chromosome #2 partial fragment differentiated into functional sperms in the chimeric mice and that the human chromosome #2 partial fragment was transmitted to offsprings through the sperms.

### Example 44

### Detection and quantitative determination of human antibody κ chain in sera of offspring mice of the chimeric mice

The concentration of human antibody κ chain in the sera of the offspring mice K2-1F-1~10 and K2-4F-1~5 from Example 42 was determined quantitatively by ELISA. The mice of about 4-6 months after birth were bled and the concentration of human antibody κ chain in the sera was determined by ELISA in the same manner as in Example 20.

The results are shown in Table 18 together with the data obtained in Example 42 on the retention of chromosome. It was confirmed that the human antibody κ chain gene can function in the offspring mice reproduced from the chimeric mice.

**Table 18. Concentration of Human Antibody κ Chain in Offspring Mice (ELISA)**

| Mother mouse | Number of mouse | Presence of human chromosome #2 fragments | Ig κ (mg/l) |
|---|---|---|---|
| K2-1F | #1 | - | 0.58 |
| K2-1F | #2 | + | 84.1 |
| K2-1F | #3 | + | 12.8 |
| K2-1F | #4 | + | 15.1 |
| K2-1F | #5 | - | 0.52 |
| K2-1F | #6 | - | 0.58 |
| K2-1F | #7 | - | 1.30 |
| K2-1F | #8 | - | 0.90 |
| K2-1F | #9 | - | 0.56 |
| K2-1F | #10 | + | 28.8 |
| K2-4F | #1 | - | < 0.04 |
| K2-4F | #2 | + | 23.3 |
| K2-4F | #3 | + | 11.8 |
| K2-4F | #4 | - | 0.08 |
| K2-4F | #5 | - | 0.06 |

### Example 45

### Analysis of spleen cells from the human chromosome #14 partial fragment transferred chimeric mice

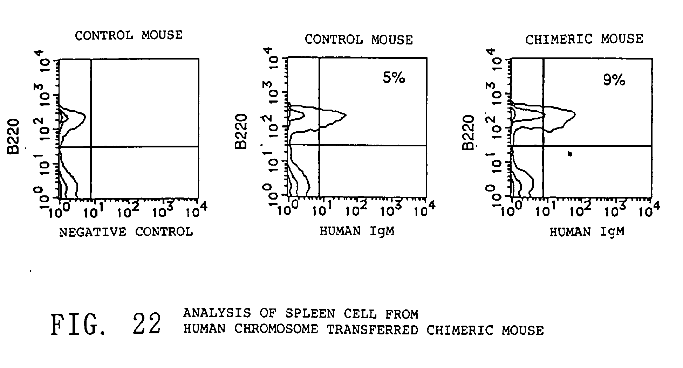

Flow cytometry analysis was accordance with the method described in "New Biochemical Experiment Lecture 12, molecular immunology I-Immunocells · Cytokines-", edited by the Japanese Biochemical Society, 1989, published by Tokyo Kagaku Dojin; "Cell Technology Separated Volume 8, New Cell Technology Experiment Protocol", edited by the University of Tokyo, Medical Science Institute, Anti-cancer Laboratory, 1991, published by Shujunsha; and A.Doyle and J.B.Griffiths, "Cell & Tissue Culture: Laboratory Procedures", published by John Wiley & Sons Ltd., 1996. The spleen was removed from the chimeric mouse KPG06 (derived from the PG16 clone, 30% chimerism) from Example 19 of six months after birth and treated with an aqueous solution of ammonium chloride. The spleen cells were stained with fluorescein isothiocyanate (FITC)-labeled anti-mouse CD45R (B220) antibody (Pharmingen, 01124A) in PBS containing 1% rat serum. After being washed, the cells were reacted with 0.1 µ g of biotin-labeled anti-human IgM antibody (Pharmingen, 08072D) or a control biotin-labeled anti-human λ chain antibody (Pharmingen, 08152D) in PBS containing 5% mouse serum and stained with 0.1 µ g of streptoavidin-phycoerythrin (Pharmingen, 13025D), followed by analysis with a flowcytometer (Becton Dickinson Immunocytometry Systems, FACSort). An ICR mouse retaining no human chromosome was used as a control for analysis by the same method. The results are shown in Fig. 22. In Fig. 22, the human IgM is plotted on the horizontal axis and the CD45R (B220) is plotted on the vertical axis. A population of cells positive to both B cell marker CD45R (FITC) and human IgM (PE) increased by 4%, indicating that cells expressing human antibody µ chain on the cell surfaces were present in the chimeric mice.

### Example 46

### Cloning and sequencing of variable regions of human antibody genes from cDNA derived from the spleen of the chimeric mice expressing human antibody heavy chain, κ and λ chains, respectively

In the same manner as in Example 5, cDNAs were synthesized from RNAs extracted from the spleens of the chimeric mice K15A (derived from the 1-4 clone, prepared by the method described in Example 10), K2-8 prepared in Example 13 and KPG22-2 prepared in Example 31, all of which were confirmed to express human antibody heavy chain, κ and λ chains in Examples 29, 23 and 32, respectively. PCR was performed using the respective cDNAs and the following primers to amplify the variable regions of respective human antibody. cDNA derived from the spleen of a non-chimeric mouse ICR was used as a negative control. Those primers set forth below without indication of reference literature were designed on the basis of the nucleotide sequences obtained from data bases such as Genebank and the like.
· K15A (heavy chain)
   For constant region:
   HIGMEX1-2; 5'-CCAAGCTTCAGGAGAAAGTGATGGAGTC (SEQ ID NO:53)
   HIGMEX1-1: 5'-CCAAGCTTAGGCAGCCAACGGCCACGCT (used in 2nd PCR of VH3BACK) (SEQ ID NO:54)
   For variable region:
   VH1/5BACK (59°C, 35 cycles, Marks et al., Eur. J. Immnol., 21, 985-, 1991).
   VH4BACK (59°C, 35 cycles, Marks et al., supra), and
   VH3BACK (1st PCR:59 °C , 35 cycles; 2nd PCR:59°C, 35 cycles, Marks et al., supra)
· K2-8 (light chain κ)
   For constant region:
   KC2H: 5'-CCAAGCTTCAGAGGCAGTTCCAGATTTC (SEQ ID NO:55)
   For variable region:
   Vkl/4BACK (55°C, 40 cycles, Marks et al., Eur. J. Immnol., 21, 985-, 1991),
   Vk2BACK (55°C, 40 cycles, Marks et al., supra), and
   Vk3BACK (55°C, 40 cycles, Marks et al., supra)
· KPG22-2 (light chain λ)
   For constant region:
   Cλ MIX (a mixture of the following three kinds of primers at an equal molar ratio)
   IGL1-CR : 5'-GGGAATTCGGGTAGAAGTCACTGATCAG (SEQ ID NO:56)
   IGL2-CR : 5'-GGGAATTCGGGTAGAAGTCACTTATGAG (SEQ ID NO:57)
   IGL7-CR : 5'-GGGAATTCGGGTAGAAGTCACTTACGAG (SEQ ID NO:58)
   For variable region:
   V λ 1LEA1 (55°C, 40 cycles, Williams et al., Eur. J. Immunol., 23, 1456-, 1993),
   Vλ 2MIX (55°C, 40 cycles, a mixture of Vλ 2 LEA1 and Vλ 2 JLEAD (Williams et al. (supra)) at an equal molar ratio)
   Vλ 3MIX (55°C, 40 cycles, a mixture of Vλ 3LEA1, Vλ 3JLEAD and Vλ 3BACK4, which were reported in Williams et al. (supra) at an equal molar ratio.

   The PCR was performed with combinations of the primers for constant regions with those for variable regions (3 primer pairs each for heavy chain, κ and λ chains) at 94 °C for 15 seconds, at the annealing temperatures shown with respect to the respective primers for variable region for 15 seconds, at 72°C for 20 seconds in the cycle numbers shown with respect to the respective primers for variable region using a Thermal Cycler 9600 (Perkin-Elmer Corp.). In the second run of PCR using VH3BACK, the amplification products of the first run of PCR were amplified again with a combination of the two primers H1GMEX1-1 and VH3BACK. All of the amplification products were electrophoresed on a 1.5% agarose gel and stained with ethiduim bromide for detection. As a result, the amplification products having expected lengths (heavy chain, about 470 bp; light chain κ, about 400 bp; and light chain λ , about 510 bp) were detected in all of the combinations. In the negative control, specific amplification product was not detected at the same position in any of the combinations. The obtained amplification products were extracted from the agarose gel using prep.A.gene (Bio-Rad Laboratories, Inc.), treated with restriction enzymes (heavy chain, HindIII and PstI; light chain κ, HindIII and PvuII; and light chain λ, HindIII and EcoRI), and cloned into pUC119 (TAKARA SHUZO CO., LTD.) at the sites of HindIII/PstI (heavy chain), HindIII/HincII (κ chain) and HindIII/EcoRI (λ chain). The nucleotide sequences of the products that were amplified with the following primers and which were cloned into the plasmids were determined with a Fluorescence Autosequencer (Applied Biosystems Inc.).
· HIGMEX1-2× VH1/5BACK: 10 clones
· HIGMEX1-2× VH4BACK: 8 clones
· HIGMEX1-2 (2nd PCR, HIGMEX1-1)× VH3BACK: 5 clones
· KC2HX Vκ 1/4BACK: 6 clones
· KC2H× Vκ 2BACK: 7 clones
· KC2H× Vκ 3BACK: 4 clones
· Cλ MIX× Vλ 1LEA1: 5 clones
· Cλ MIX× Vλ 2MIX: 6 clones
· Cλ MIX× Vλ 3MIX: 5 clones

The obtained nucleotide sequences were analyzed with DNASIS (Hitachi Software Engineering Co., Ltd.). The results show that all of the sequences were derived from human and that they were functional sequences which did not contain a termination codon at any site between an initiation codon and a constant region: this was true with all of the κ and λ chains and with 21 out of a total of 23 heavy chains. When the same sequences were removed from the determined sequences, unique variable region sequences were identified. as follows: 17 heavy chains, 11κ chains, and 12 λ chains.

### Example 47

### Analysis of the nucleotide sequences of variable region of human antibody genes from cDNA derived from the spleen of the chimeric mouse expressing human antibody heavy chain, κ and λ chains, respectively

The nucleotide sequences determined in Example 46 (heavy chain, 17 clones; κ chain, 11 clones; and λ chain, 12 clones) were analyzed in the following points.
1. Identification of known germ line V gene segments used in the respective variable regions
2. Identification of known germ line J gene segments used in the respective variable regions
3. Identification of known germ line D gene segments used in the heavy chain variable regions
4. Identification of the addition of N region in the heavy chain variable regions on the basis of the results of 1, 2 and 3
5. Determination of the amino acid sequences deduced from the nucleotide sequences of the respective variable regions

The results are shown in Table 19. For the identification in points of 1 and 2, search for homology with germ line V and J segments registered in Genbank and the like was conducted with DNASIS. The VH segments, Vκ segments and Vλ segments are shown in Table 19 together with the family names of the respective V fragments in accordance with the conventions described in Cook et al., Nature genetics, 7, 162-, 1994 (VH fragments), Klein et al., Eur. J. Immunol, 23, 3248-, 1993 (V κ fragments) and Williams et al. (supra) (V λ fragments), respectively. For the identification in point 3, search for homology with germ line D fragments reported in Ichihara et al., The EMBO J., 7, 13, 4141-, 1988 was conducted with DNASIS. Assignment was based on at least 8 bp identity and the results are shown in Table 19. DN1* is believed to be the new DN family segment reported in Green et al., Nature Genetics, 7, 13-, 1994. For the identification in point 4, the nucleotide sequences which did not appear in any germline sequences were determined to be N regions on the basis of the results for 1(V), 2(J) and 3 (D). As a result, N region was observed in 11 of the 13 sequences in which D segment was identified and its average length was 8.7 bp. For the determination in point 5, the respective sequences were converted by DNASIS to amino acid sequences which were expressed with one letter symbols. In Table 19, only CDR3 region is shown. At the right side of Table 19, the names of the primers used in cloning of the respective variable regions and the names of clones are shown.

**Table 19**

| V family | V segment | CDR3 | J (D) | V primer | Clone |
|---|---|---|---|---|---|
| K15A | | | | | |
| VH1 | VH1-8 | VRSSSWYEYYYYGMDV | J6 (DN1) | VH4BACK | H4-10 |
| | VHI-18 | GGITMVRGLHTDWYFDL | J2 (DXP'1) | VHI/53ACK | H1-7 |
| | VH1-24 | APYSGRFDY | J4 (DKI) | VH1/5BACK | H1-6 |
| | VH1-46 | ERYYGSGSYQDYYYYYGMDV | J6 (DXP'1) | VH1/5BACK | H1-2 |
| | VH1-46 | GGYSGYEDYYYYGMDV | J6 (DK1) | VH1/5BACK | H1-10 |
| VH2 | VH2-5 | SYFDWPDFDY | J4 (DXP1) | VH4BACK | H4-14 |
| VH3 | VH3-21 | EGCSGGSCLPGYYYYGMDV | J6 (DLR2) | VH1/5BACK | H1-4 |
| | VH3-23 | AHGDPYFDY | J4 | VH1/5BACK | H1-3 |
| | VH3-23 | DADAFDI | J3 | VH1/5BACK | H1-8 |
| | VH3-23 | SGWDY | J4 (DN1*) | VH3BACK | H3-3 |
| | VH3-23 | TGFDL | 12 | VH4BACK | H4-4 |
| | VH3-33 | EGGYGSVGDYYYYGMDV | J6 (DXP'1) | VH1/5BACK | H1-9 |
| | VH3-33 | GGYSYGYDYYYYGMDV | J6(DXP'1) | VH3BACK | H3-5 |
| | VH3-33 | GYSSGWYDY | J4 (DN1*) | VH4BACK | H4-9 |
| VH4 | VH4-34 | RYSSGWYYFDY | J4 (DN1*) | VH4BACK | H4-15 |
| | VH4-59 | GRIAVASFDY | J4 (DN1*) | VH4BACK | H4-2 |
| | VH4-59 | GSGSYFHFDY | J4 | VH4BACK | H4-6 |
| K2-8 | | | | | |
| V κ 1 | O18-8 | QQHDNLPFT | J3 | V κ 1BACK | K1-1 |
| | 018-8 | QQYDNLPIT | J5 | V κ 1BACK | K1-3 |
| | O18-8 | QQHDNLPFA | J3 | V κ 2BACK | K2-2 |
| | L1 | QQYNSYPLT | J4 | V κ 1BACK | K1-6 |
| V κ 2 | A17 | MQGTHLLT | J4 | V κ 2BACK | K2-1 |
| | A17 | MQGTHWIT | J5 | V κ 2BACK | K2-5 |
| V κ 3 | A27 | QQYGSSPTWT | J1 | V κ 3BACK | K3-1 |
| | A27 | QQYGSSPFT | J3 | V κ 3BACK | K3-4 |
| | A27 | QQYGSSPLWT | J1 | V κ 3BACK | K3-5 |
| | A27 | QQYGSSPPWT | J1 | V κ 3BACK | K3-6 |
| Vκ6 | A26-10 | HQSSSLPQT | J1 | V κ 2BACK | K2-4 |
| KPG22-2 | | | | | |
| Vλ1 | DPL3 | AAWDDSLDVV | JC3 | V λ 1LEA1 | L1-3 |
| | DPL5 | GTWDSSLSAGV | JC2 | V λ 1LEA1 | L1-4 |
| | DPL5 | GTWDSSLSAGW | JC3 | V λ 1LEA1 | L1-6 |
| | DPL5 | GTWDSSLSAVV | JC2 | Vλ1LEA1 | L1-9 |
| | DPL8 | QSYDSSLSGVV | JC3 | Vλ1LEA1 | L1-8 |
| V λ 2 | DPL10 | CSYAGSSTLV | JC2 | V λ 2MIX | L2-4 |
| | DPL11 | SSYTSSSTVV | JC2 | V λ 2MIX | L2-1 |
| | DPL11 | SSYTSSSTLV | JC2 | V λ 2MIX | L2-3 |
| | DPL11 | CSYTSSSTFV | JC2 | V λ2MIX | L2-7 |
| | DPL12 | SSYAGSNNLV | JC3 | V λ 2MIX | L2-5 |
| | DPL12 | SSYAGSNNFVV | JC3 | V λ 2MIX | L2-6 |
| V λ 3 | DPL16 | NSRDSSGNLV | JC2 | V λ 3MIX | L3-1 |

### Example 48

### Preparation of a targeting vector for knocking out antibody genes (heavy-chain and light-chain κ genes) in TT2 (or TT2F) ES cells

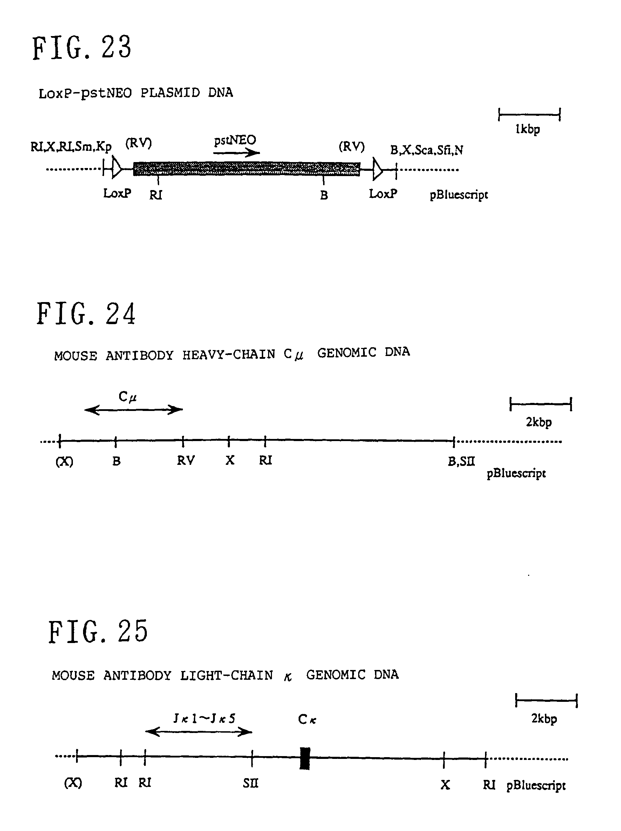

It becomes possible to transfer a human chromosome #14 fragment marked with a G418 resistance gene (Example 9) and human chromosome #2 (Example 18) or #22 (Example 35) marked with a puromycin resistance gene into TT2 (or TT2F) cells in which mouse antibody genes (heavy-chain, light-chain κ) are disrupted. Those chimeric mice which are produced from these human chromosomes #14+#2 or #14+#22-transferred, mouse antibody genes (heavy-chain, light-chain κ)-disrupted TT2 (or TT2F) ES cells according to the method of Example 19 (heavy-chain + κ chain) or Example 36 (heavy-chain + λ chain) are expected to produce antibodies both heavy- and light-chains of which are mainly derived from humans. The abbreviations of the restriction enzymes, etc. appearing in Figures 23-27 are as follows.
Restriction enzymes: Kp: KpnI, B: BamHI, RI: EcoRI, RV: EcoRV, N: NotI, SII: ScaII, Sca: ScaI, Sfi: SfiI, Sm: SmaI, X: XhoI, SI: SalI, dKp: deletion of KpnI, (X): XhoI restriction site from λ vector
Dotted portion: pBluescript SKII(+) plasmid DNA
◁▷: LoxP sequence

### 1. Preparation of plasmid LoxP-pstNEO in which LoxP sequence is inserted at both the ends of a G418 resistance gene

For the deletion of a G418 resistance gene after knocking out an antibody gene of TT2 or (TT2F) cells, it is necessary to insert LoxP sequence (Sauer et al., Proc. Natl. Acad. Sci. USA, 85, 5166-, 1988) which is the recognition sequence of Cre recombinase (Sauer et al., supra) at both the ends of the G418 resistance gene (Example 1) in the same direction. Briefly, pstNEO gene was cut out from pSTneoB plasmid DNA (Example 1) with restriction enzyme XhoI. The DNA fragment was purified by agarose gel electrophoresis and then blunted with T4-DNA polymerase (Blunting End Kit from Takara Shuzo). LoxP sequence-containing plasmid DNA pBS246 (Plasmid pBS246, loxP2 Cassette Vector, U. S. Patent 4959317) was purchased from GIBCO BRL. XhoI linker DNAs were inserted into the EcoRI and SpeI restriction sites of this plasmid. The pstNEO DNA fragment described above was inserted into the EcoRV restriction site of the thus modified pBS246 to give plasmid LoxP-pstNEO (Fig. 23).

### 2. Isolation of genomic DNA clones containing C57BL/6-derived antibody heavy-chain Cµ (IgM constant region) or light-chain Jκ -Cκ (Igκ joint region and constant region)

Since TT2 (or TT2F) cells were derived from F1 mice between C56BL/6 mice and CBA mice, the inventors have decided to prepare vectors for antibody gene knockout using genomic DNA clones derived from a C57BL/6 mouse. As a genomic DNA library, an adult C57BL/6N male liver-derived λ DNA library from Clontech was used. As a probe for screening, the following synthetic DNA sequences (60 mers) were used.
Heavy-chain C µ probe: 5'-ACC TTC ATC GTC CTC TTC CTC CTG AGC CTC TTC TAC AGC ACC ACC GTC ACC CTG TTC AAG-3'(SEQ ID NO: 59)
Light-chain κ probe: 5'-TGA TGC TGC ACC AAC TGT ATC CAT CTT CCC ACC ATC CAG TGA GCA GTT AAC ATC TGG AGG-3'(SEQ ID NO: 60)

The λ clones were isolated and analyzed to subclone those DNA fragments containing heavy-chain C µ or light-chain Jκ -Cκ into plasmid pBluescript SKII(+) (Stratagene) (heavy-chain C µ : Fig. 24; light-chain Jκ -Cκ: Fig. 25). These DNA fragments were used to prepare targeting vectors for disrupting mouse antibody genes in TT2 (or TT2F) cells as described below.

### 3. Preparation of a vector plasmid for disrupting a mouse antibody heavy-chain gene

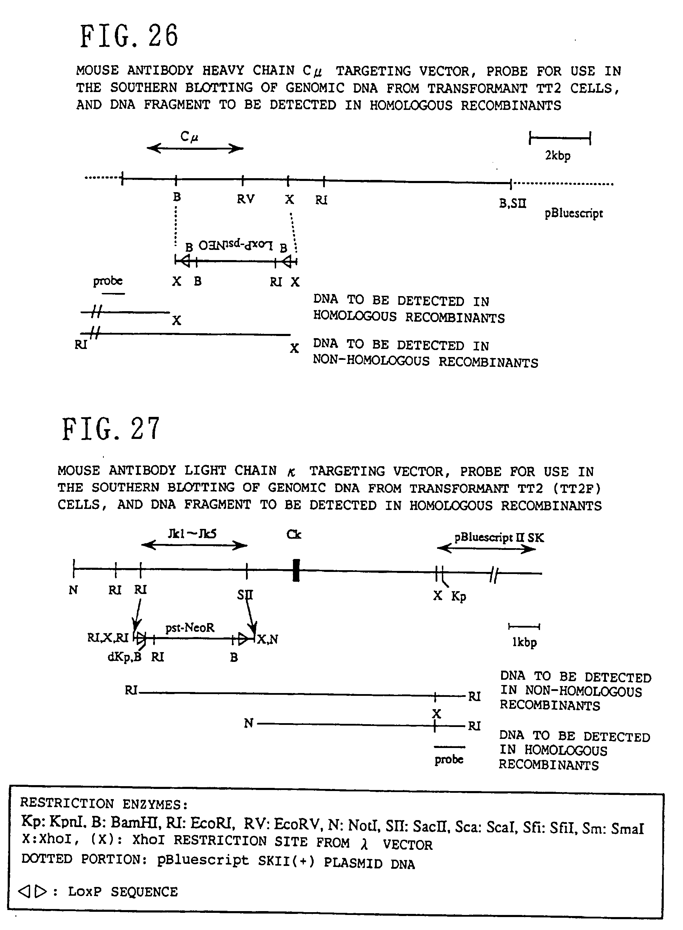

In the C µ -encoding region in the genomic DNA fragment containing a mouse antibody heavy-chain constant region which was prepared in 2 above, a DNA fragment containing the 2nd to 4th exons (BamHI-XhoI) was replaced with the LoxP-pstNEO gene prepared in 1 above (Fig. 26). The direction of transcription of pstNEO was opposite to the direction of transcription of the antibody gene. This plasmid DNA was amplified using *E*. *coli* JM109 and purified by cesium chloride equilibrium centrifugation ("Introduction to Cell Technology Experimental Operations", published by Kodansha, 1992). The purified plasmid DNA was cleaved at one site with restriction enzyme SacII and used for transfection of TT2 (or TT2F) ES cells. As a probe for Southern blot analysis of transformant genomic DNA to detect from transformant TT2 (or TT2F) ES cells those clones in which homologous recombination has taken place in the antibody heavy-chain portion with the targeting vector, a DNA fragment (about 500 bp) of the switch region located upstream of C µ -encoding region. This DNA fragment was obtained by amplifying 129 mouse genomic DNAs by PCR under the following conditions.
Sense primer: 5'-CTG GGG TGA GCC GGA TGT TTT G-3'(SEQ ID NO: 61)
Antisense primer: 5'-CCA ACC CAG CTC AGC CCA GTT C-3'(SEQ ID NO: 62)
Template DNA: 1 *µ* g of EcoRI-digested 129 mouse genomic DNAs
The reaction buffer, deoxynucleotide mix and Taq DNA polymerase used were from Takara Shuzo.
Reaction conditions: 94°C , 3 min, 1 cycle → 94°C, 1 min; 55°C, 2 min; 72°C, 2 min; 3 cycles → 94°C , 45 sec; 55°C , 1 min; 72°C, 1 min; 36 cycles

After it was confirmed that amplified DNA fragment can be cleaved at one site with restriction enzyme HindIII as indicated in the Genbank database, this DNA fragment was subcloned into the EcoRV restriction site of plasmid pBluescript. This plasmid DNA (S8) was digested with restriction enzymes BamHI and XhoI. A PCR fragment (about 550 bp) was purified by agarose gel electrophoresis to give a probe. Genomic DNA from those TT2 (or TT2F) ES cells transformed with the targeting vector is digested with restriction enzymes EcoRI and XhoI, and separated by agarose gel electrophoresis. Then, Southern blotting is performed using the above probe.

### 4. Preparation of a vector for disrupting the mouse antibody light-chain κ gene

The genomic DNA fragment prepared in 2 above contains the J region and constant region of mouse antibody light-chain κ. A DNA fragment (EcoRI-SacII) containing the J region (J1-J5) was replaced with the LoxP-pstNEO gene prepared in 1 above (Fig. 27). The direction of transcription of pstNEO was the same as that of the antibody gene. This plasmid DNA was amplified using *E*. *coli* JM109 and purified by cesium chloride equilibrium centrifugation. The purified plasmid DNA was cleaved at one site with restriction enzyme KpnI and used for transfection of TT2 (or TT2F) ES cells. As a probe for Southern blot analysis of transformant genomic DNA to detect from transformant TT2 (or TT2F) ES cells those clones in which homologous recombination has taken place in the antibody heavy-chain portion with the targeting vector, a DNA fragment at the 3' end of the light-chain J κ -Cκ genomic DNA fragment (see Fig. 25) (XhoI-EcoRI; about 1.4 kbp) was used. Genomic DNA from those TT2 (or TT2F) ES cells transformed with the targeting vector is digested with restriction enzymes EcoRI and NotI, and separated by agarose gel electrophoresis. Then, Southern blotting is performed using the above probe.

### Example 49

### Production of a mouse ES cell antibody heavy-chain gene-disrupted clone

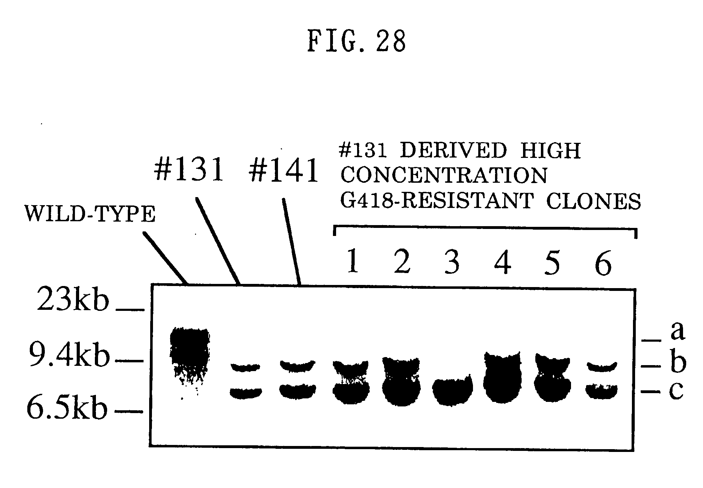

In order to obtain a recombinant in which an antibody heavy-chain gene has been disrupted by homologous recombination (hereinafter, referred to as an "antibody heavy-chain homologous recombinant"), the antibody heavy-chain targeting vector prepared in Section 3, Example 48 was linearized with restriction enzyme SacII (Takara Shuzo), and transferred into mouse TT2F ES cells according to the method described by Shinichi Aizawa, "Biomanual Series 8, Gene Targeting", published by Yodosha, 1995. The TT2F cells were treated with trypsin and suspended in HBS at a concentration of 2.5x10⁷ cells/ml. To the cell suspension, 5 µg of DNA was added. Then, electroporation was performed with a gene pulser (Bio-Rad Laboratories, Inc.; resistor unit not connected). A voltage of 250 V was applied at a capacitance of 960 µF using an electroporation cell of 4 mm in length at room temperature. The electroporated cells were suspended in 20 ml of an ES medium and inoculated into two tissue culture plastic plates (Corning) of 100 mm into which feeder cells were seeded preliminarily. Similarly, experiments using 10 and 15 µ g of DNA were also conducted. After one day, the medium was replaced with a medium containing 300 *µ* g/ml of G418 (GENETICIN; Sigma). Seven to nine days thereafter, a total of 176 colonies formed were picked up. Each colony was grown up to confluence in a 12-well plate, and then four fifths of the culture was suspended in 0.2 ml of a preservation medium [ES medium + 10% DMSO (Sigma)] and stored frozen at -80°C. The remaining one fifth was inoculated into a 12-well gelatin coated plate and cultured for 2 days. Then, genomic DNA was obtained by the method described in Example 2. These genomic DNAs from G418 resistant TT2F cells were digested with restriction enzymes EcoRI and XhoI (Takara Shuzo) and separated by agarose gel electrophoresis. Then, Southern blotting was performed to detect homologous recombinants with the probe described in Section 3, Example 48. As a result, 3 clones out of the 176 clones were homologous recombinants. The results of Southern blot analysis of wild-type TT2F cells and homologous recombinants #131 and #141 are shown in the left-side three lanes in Fig. 28. In wild-type TT2F cells, two bands (a and b) are detected which were obtained by the EcoRI and XhoI digestion. In the homologous recombinants, it is expected that one of these bands disappears and that a new band (c) will appear at the lower part of the lane. Actually, band (a) has disappeared in #131 and #141 in Fig. 28 and a new band (c) has appeared. The size of DNA is shown at the left side of the Figure. These results show that one allele of an antibody heavy-chain gene in these recombinant clones has been disrupted by homologous recombination.

### Example 50

### Production of chimeric mice from antibody heavy-chain homologous recombinant ES cells

The cells in a frozen stock of the antibody heavy-chain homologous recombinant TT2F cell clone #131 from Example 49 were thawed, started to culture and injected into 8-cell stage embryos obtained by mating a male and a female mouse of ICR or MCH(ICR) (CREA JAPAN, INC.); the injection rate was 10-12 cells per embryo. After the embryos were cultured overnight in the medium for ES cells (see Example 9) to develop into blastocysts, about ten of the TT2F cell-injected embryos were transplanted to each side of the uterus of a foster mother ICR mouse (CREA JAPAN, INC.; 2.5 days after pseudopregnant treatment). As a result of transplantation of a total of 94 injected embryos, 22 offspring mice were born. Chimerism in the offsprings can be determined by the extent of TT2F cell-derived agouti coat color (dark brown) in the host embryo (ICR)-derived albino coat color (white). Out of the 22 offsprings, 18 mice were recognized to have partial agouti coat color, indicating the contribution of the ES cells. Out of the 18 mice, 16 mice were female chimeric mice in which more than 80% of their coat color was agouti (i.e. ES cell-derived). From these results, it was confirmed that the antibody heavy-chain homologous recombinant ES cell clone #131 retains the ability to produce chimera. Since a large number of the resultant chimeric mice are female mice exhibiting extremely high contribution, it is very likely that the ES cells have differentiated into functional germ cells (oocytes). Two female chimeric mice exhibiting 100% contribution were mated with MCH(ICR) male mice. As a result, all of the offspring mice exhibited agouti coat color. These offsprings are derived from #131 (see Example 42), and thus it is considered that a disrupted antibody heavy-chain allele was transmitted to them at a rate of 50%.

### Example 51

### Production of a double knockout clone from the antibody heavy-chain homologous recombinant

It has been reported that a clone in which both alleles are disrupted can be obtained by disrupting one allele by insertion of a G418 resistance gene, culturing an ES cell clone in a medium with an increased G418 concentration and screening the resultant high concentration G418 resistant clones (Shinichi Aizawa, "Biomanual Series 8, Gene Targeting", published by Yodosha, 1995). Based on this technique, the inventors have conducted the following experiments in order to obtain both alleles-disrupted clones from the TT2F antibody heavy-chain homologous recombinants #131 and #141. First, in order to determine the lethal concentration of G418 for both #131 and #141 clones, each clone was inoculated into ten 35 mm plates at a rate of about 100 cells per plate (in this Example, G418 resistant primary culture cells which were not treated with mitomycin were used as feeder cells)(see Example 9). The cells were cultured in an ES medium containing 0, 0.5, 1, 2, 3, 5, 8, 10, 15 and 20 mg/ml of G418 (GENETICIN, Sigma) for 10 days. As a result, definite colonies were observed at a concentration of up to 3 mg/ml, but no colony formation was observed at 5 mg/ml. Based on these results, the minimum lethal concentration was decided to be 5 mg/ml. Then, high concentration G418 resistant clones were selected at concentrations of 4, 5, 6, 7 and 8 mg/ml. For each of #131 and #141, cells were inoculated into ten 100 mm plates at a rate of about 10⁶ cells per plate and cultured in an ES medium containing G418 at each of the concentrations described above (5 grades; two plates for each concentration). Twelve days after the start of culture, definite colonies (#131: 12 clones; #141: 10 clones) were picked up from plates of 7 mg/ml and 8 mg/ml in G418 concentration. These clones were stored frozen and genomic DNA was prepared by the same procedures as in Example 49. The genomic DNAs from these high concentration G418 resistant clones were digested with restriction enzymes EcoRI and XhoI (Takara Shuzo) and separated by agarose gel electrophoresis. Then, Southern blotting was performed to detect with the probe from Section 3, Example 48 those clones in which both alleles have been disrupted. As a result, one clone derived from #131 (#131-3) was found to be both alleles-distrupted clone. The results of Southern blot analysis of 6 clones derived from #131 are shown in Fig. 28. In wild-type TT2F cells, two wild-type bands (a, b) are detected after the EcoRI and XhoI digestion. In one allele homologous recombinants (#131, #141), the upper band (a) has disappeared and a new band (c) has appeared (Example 49). Furthermore, it is expected that due to the disruption of both alleles, another wild-type band (b) disappears and that the disruption-type band (c) remains alone. In Fig. 28, this band pattern is observed in clone No. 3 (#131-3). This demonstrates that both alleles of an antibody heavy-chain gene have been disrupted in this clone.

### Example 52

### Removal of a G418 resistance marker gene from the antibody heavy-chain-deficient homozygote TT2F clone

The G418 resistance marker gene in the antibody heavy-chain both alleles-disrupted clone (high concentration G418 resistant clone #131-3) from Example 51 was removed by the following procedures. An expression vector, pBS185 (BRL), containing Cre recombinase gene which causes a site-specific recombination between the two LoxP sequences inserted at both the ends of the G418 resistance gene was transferred into #131-3 clone according to the methods described in Shinichi Aizawa, "Biomanual Series 8, Gene Targeting", published by Yodosha, 1995 and Seiji Takatsu et al., "Experimental Medicine (extra number): Basic Technologies in Immunological Researches", p. 255-, published by Yodosha, 1995). Briefly, #131-3 cells were treated with trypsin and suspended in HBS to give a concentration of 2.5x10⁷ cells/ml. To the cell suspension, 30 µ g of pBS185 DNA was added. Then, electroporation was performed with a gene pulser (Bio-Rad Laboratories, Inc.; resistor unit not connected). A voltage of 250 V was applied at a capacitance of 960 *µ* F using an electroporation cell of 4 mm in length (see Example 1). The electroporated cells were suspended in 5 ml of an ES medium and inoculated into a tissue culture plastic plate (Corning) of 60 mm in which feeder cells were seeded preliminarily. After two days, the cells were treated with trypsin and reinoculated into three 100 mm plates (preliminarily seeded with feeder cells) such that the three plates have 100, 200 and 300 cells, respectively. A similar experiment was also conducted under the same conditions except that the setting of the gene pulser was changed (resistor unit connected; resistance value infinite). After seven days, a total of 96 colonies formed were picked up and treated with trypsin. Then, the colonies were divided into two groups; one was inoculated into a 48-well plate preliminarily seeded with feeder cells and the other was inoculated into a 48-well plate coated with gelatin alone. The latter was cultured in a medium containing 300*µ* g/ml of G418 (GENETICIN, Sigma) for three days. Then, G418 resistance was judged from the survival ratio. As a result, 6 clones died in the presence of G418. These G418 sensitive clones were grown to confluence in 35 mm plates, and four fifths of the resultant culture was suspended in 0.5 ml of a preservation medium [ES medium + 10% DMSO (Sigma)] and stored frozen at -80°C. The remaining one fifth was inoculated into a 12-well gelatin coated plate and cultured for two days. Thereafter, genomic DNA was prepared by the same procedures as in Example 2. These genomic DNAs from G418 sensitive TT2F clones were digested with restriction enzyme EcoRI (Takara Shuzo) and separated by agarose gel electrophoresis. Then, Southern blotting was performed to confirm the removal of the G418 resistance gene using a 3.2 kb XhoI fragment (Probe A) from G418 resistance gene-containing pSTneoB. As a result, bands observed in #131-3 clone which hybridize with Probe A were not detected at all in the sensitive clones. From these results, it was confirmed that the G418 resistance marker gene had been surely removed in the G418 sensitive clones obtained. Additionally, as a result of Southern blot analysis performed in the same manner using Probe B obtained by digesting pBS185 DNA with EcoRI, no specific band which hybridizes with Probe B was detected in these G418 sensitive clones. Thus, it is believed that Cre recombinase-containing pBS185 is not inserted into the chromosomes of the sensitive clones. In other words, these sensitive clones can be transformed with the vector for knocking out an antibody light-chain (vector having a loxP sequence at both the ends of a G418 resistance gene) described in Section 4, Example 48.

### Example 53

### Transfer of human chromosome #14 fragment (containing antibody heavy-chain gene) into the antibody heavy-chain-deficient ES cell clone

Human chromosome #14 (containing an antibody heavy-chain gene) marked with a G418 resistance gene is transferred by microcell fusion as described in Example 9 into the mouse ES cell clone (from TT2F, G418 sensitive) obtained in Example 52 which is deficient in an endogenous antibody heavy-chain. In the resultant G418 resistant clone, the retention of human chromosome #14 fragment containing a human antibody heavy-chain gene is confirmed by PCR analysis or the like (see Example 9).

### Example 54

### Transfer of human chromosome #2 fragment or human chromosome #22 fragment into the antibody heavy-chain-deficient ES cell clone retaining human chromosome #14 fragment

A human chromosome #2 fragment (containing the antibody heavy-chain κ gene) or human chromosome #22 (containing the antibody heavy-chain λ gene) marked with a puromycin resistance gene is transferred into the antibody heavy-chain-deficient mouse ES cell clone retaining a human chromosome #14 partial fragment (G418 resistant) from Example 53 by microcell fusion as described in Examples 18 and 35. In the resultant puromycin and G418 double drug-resistant clone, the retention of the human chromosome #14 fragment and human chromosome #2 fragment or #22 fragment is confirmed by PCR analysis or the like (see Examples 18 and 35).

### Example 55

### Production of chimeric mice from the endogenous antibody heavy-chain-deficient mouse ES cells retaining human chromosome #14 fragment containing a human antibody heavy-chain gene

Chimeric mice from the endogenous antibody heavy-chain gene-deficient mouse ES cell clone obtained in Example 53 retaining human chromosome #14 fragment containing a human antibody heavy-chain gene are produced by the same procedures as in Example 10. In the resultant chimeric mice, a human antibody heavy-chain produced in the ES cell clone-derived B cells is detected by the method described in Example 14. Since antibody heavy-chain genes functional in the ES cell clone-derived B cells are only the human-derived gene on the transferred chromosome, many of the ES cell clone-derived B cells produce human antibody heavy-chain.

### Example 56

### Production of chimeric mice from the endogenous antibody heavy-chain-deficient mouse ES cells retaining human chromosomes #14+#2 fragments or #14+#22 fragments

Chimeric mice are produced by the same procedures as in Examples 19, 36, etc from the endogenous antibody heavy-chain gene-deficient mouse ES cell clone retaining human chromosomes #14+#2 fragments or #14+#22 fragments obtained in Example 54. In the resultant chimeric mice, human antibody heavy-chain and light-chain κ or λ are detected in the ES cell clone-derived B cells according to the method described in Examples 14, 23 and 32. As in Example 55, antibody heavy-chain genes functional in the ES cell clone-derived B cells are only the human-derived gene on the transferred chromosome. Thus, many of the ES cell clone-derived B cells produce human heavy-chains. Furthermore, complete human antibody molecules both heavy and light-chains which are derived from humans are also detected by the method described in Examples 37 and 38.

### Example 57

### Production of human antibody-producing hybridomas from the chimeric mice derived from the endogenous antibody heavy-chain-deficient mouse ES cells retaining human chromosomes #14+#2 fragments or #14+#22 fragments

The chimeric mice from Example 56 are immunized with an antigen of interest in the same manner as in Examples 15, 25 and 34. The spleen is isolated from each mice and the spleen cells are fused with myeloma cells to produce hybridomas. After cultivation for 1-3 weeks, the culture supernatant is analyzed by ELISA. The ELISA is performed by the method described in Examples 14, 15, 21, 24, 25, 33, 34, 37 and 38. As a result, human antibody positive clones and clones which are human antibody positive and specific to the antigen used in the immunization are obtained.

### Example 58

### Production of an antibody light-chain gene-disrupted clone from the antibody heavy-chain-deficient homozygote mouse ES cells

A homologous recombinant, which has further disruption in an antibody light-chain gene in the antibody heavy-chain-deficient homozygote TT2F cell clone (G418 sensitive) obtained in Example 52 is produced by the following procedures. Briefly, the antibody light-chain targeting vector prepared in Section 4, Example 48 is linearized with restriction enzyme KpnI (Takara Shuzo), and transferred into the above TT2F cell clone (G418 sensitive) according to the method described in Shinichi Aizawa, "Biomanual Series 8: Gene Targeting", published by Yodosha, 1995. After 7-9 days, colonies formed are picked up. They are stored frozen and genomic DNA is prepared in the same manner as in Example 49. Genomic DNAs from G418 resistant clones are digested with restriction enzymes EcoRI and NotI (Takara Shuzo) and separated by agarose gel electrophoresis. Then, Southern blot analysis is performed to detect homologous recombinants with the probe described in Section 4, Example 48.

### Example 59

### Production of an double knockout clone from the antibody light-chain homologous recombinant

A clone in which both alleles of a light-chain gene are disrupted is prepared from the TT2F antibody light-chain homologous recombinant (and antibody heavy-chain-deficient homozygote) clone from Example 58 by the procedures described below. Briefly, a high concentration G418 resistant clone is prepared and stored frozen, and DNA is prepared in the same manner as in Example 51. Genomic DNA from the high concentration G418 resistant clone is digested with restriction enzymes EcoRI and NotI (Takara Shuzo) and separated by agarose gel electrophoresis. Then, Southern blot analysis is performed to detect those clones in which both alleles have been disrupted, with the probe from Section 4, Example 48.

### Example 60

### Removal of the G418 resistance gene from the antibody light-chain-deficient homozygote (antibody heavy-chain-deficient homozygote) TT2F cell clone

The G418 resistance marker gene in the antibody light-chain both alleles-disrupted clone (high concentration G418 resistant clone) obtained in Example 59 is removed by the same procedures as in Example 52. Briefly, an expression vector, pBS185 (BRL), containing Cre recombinase gene which causes a site-specific recombination between the two loxP sequences inserted at both the ends of the G418 resistance gene (Section 1, Example 48) was transferred into the above clone according to the method described in Example 52. The resultant G418 sensitive clones are grown to confluence in 35 mm plates, and 4/5 of the resultant culture was suspended in 0.5 ml of a preservation medium [ES medium + 10% DMSO (Sigma)] and stored frozen at -80°C by the same procedures as in Example 52. The remaining 1/5 was inoculated into a 12-well gelatin coated plate. After cultivation for two days, genomic DNA is prepared by the method described in Example 2. These genomic DNAs from G418 sensitive TT2F clones are digested with restriction enzyme EcoRI (Takara Shuzo) and separated by agarose gel electrophoresis. Then, Southern blotting is performed to confirm the removal of the G418 resistance gene using a 3.2 kb XhoI fragment from G418 resistance gene-containing pSTneoB as a probe.

### Example 61

### Transfer of human chromosome #14 fragment (containing antibody heavy-chain gene) into the antibody heavy-chain & light-chain-deficient ES cell clone

Human chromosome #14 (containing a human antibody heavy-chain gene) marked with a G418 resistance gene is transferred by microcell fusion as described in Example 9 into the mouse ES cell clone (from TT2F, G418 sensitive) obtained in Example 60 which is deficient in both endogenous antibody heavy-chain and light-chain. In the resultant G418 resistant clones, the retention of human chromosome #14 fragment containing a human antibody heavy-chain gene is confirmed by PCR analysis, etc. (see Example 9).

### Example 62

### Transfer of human chromosome #2 fragment (containing light-chain κ gene) into the antibody heavy-chain & light-chain-deficient ES cell clone retaining human chromosome #14 fragment (containing antibody heavy-chain gene)

A partial fragment of human chromosome #2 (containing human antibody light-chain κ gene) marked with a puromycin resistance gene is transferred by microcell fusion as described in Example 18 into the mouse ES cell clone (from TT2F, G418 resistant) obtained in Example 61 which is deficient in endogenous antibody heavy-chain and light-chain and which retains human chromosome #14. In the resultant puromycin and G418 double drug-resistant clones, the retention of the human chromosome #14 fragment and human chromosome #2 fragment is confirmed by PCR analysis etc. (see Example 18).

### Example 63

### Transfer of human chromosome #22 fragment (containing light-chain λ gene) into the antibody heavy-chain & light-chain-deficient ES cell clone retaining human chromosome #14 fragment (containing antibody heavy-chain gene)

Human chromosome #22 (containing human antibody light-chain λ gene) marked with a puromycin resistance gene is transferred by microcell fusion as described in Example 35 into the mouse ES cell clone (from TT2F, G418 resistant) obtained in Example 61 which is deficient in endogenous antibody heavy-chain and light-chain and which retains human chromosome #14. In the resultant puromycin and G418 double drug-resistant clones, the retention of human chromosome #14 fragment and human chromosome #22 fragment is confirmed by PCR analysis etc. (see Example 35).

### Example 64

### Production of endogenous antibody heavy-chain & light-chain-deficient mouse ES cells retaining fragments of the three human chromosomes of #2 (containing antibody light-chain κ gene), #14 (containing antibody heavy-chain gene) and #22 (containing antibody λ chain gene)

In order to obtain mouse ES cells retaining three kinds of human chromosomes, human chromosome #2 or #22 is marked by inserting therein a marker gene such as blasticidin resistance (Izumi et al., Exp. Cell. Res., 197:229, 1991), hygromycin resistance (Wind et al., Cell, 82:321-, 1995), etc. This marking is performed according to the method described in Examples 16 and 26. Human chromosome #22 (containing human antibody light-chain λ gene) marked with blasticidin resistance, hygromycin resistance, etc. is transferred into the mouse ES cell clone (from TT2F, G418 resistant, puromycin resistant) obtained in Example 62 which is deficient in endogenous antibody heavy-chain & light-chain and which retains both human chromosome #14 fragment and human chromosome #2 partial fragment, according to the method described in Example 9. As feeder cells for culturing ES cells, appropriate cells are selected depending on the selection marker used. When a hygromycin resistance marker is used, primary culture fibroblasts obtained from a transgenic mouse strain which retains and expresses the marker (Johnson et al., Nucleic Acids Research, vol. 23, No. 7, 1273-, 1995) are used. It is confirmed by PCR analysis, etc. (see Examples 9, 18 and 35) that the resultant G418, puromycin and hygromycin (or blasticidin) triple drug-resistant clones retain the three kinds of human chromosomes (fragments) described above. In the same manner, human chromosome #2 (fragment) marked with a hygromycin or blasticidin resistance gene is transferred into the mouse ES cell clone (from TT2F, G418 resistant, puromycin resistant) obtained in Example 63 which is deficient in endogenous antibody heavy-chain & light-chain and which retains both human chromosome #14 fragment and human chromosome #22 partial fragment.

### Example 65

### Production of chimeric mice from the endogenous antibody heavy-chain & light-chain genes-deficient mouse ES cells retaining human chromosomes fragments containing human antibody genes for heavy-chain and light-chain

Chimeric mice are produced by the same procedures as in Example 10, etc from the endogenous antibody heavy-chain & light-chain genes-deficient mouse ES cell clones retaining human chromosomes fragments containing human antibody genes obtained in Examples 61, 62, 63 and 64. In the resultant chimeric mice, mouse antibodies produced in host embryo-derived B cells and human antibodies produced mainly in ES cell clone-derived B cells are detected by the method described in Examples 14, 23 and 32. Since antibody heavy-chain genes and light-chain κ genes functional in the ES cell clone-derived B cells are only human-derived genes on the transferred chromosomes, many of the ES cell clone-derived B cells produce human antibody heavy-chain and light-chain κ (Lonberg et al., Nature, 368:856-, 1994). Furthermore, complete human antibody molecules both heavy and light-chains which are derived from humans are also detected by the method described in Examples 37 and 38.

### Example 66

### Production of human antibody-producing hybridomas from chimeric mice derived from the endogenous antibody heavy-chain & light-chain genes-deficient mouse ES cells retaining human chromosomes fragments containing human antibody genes for heavy-chain and light-chain genes

The chimeric mice from Example 65 are immunized with an antigen of interest in the same manner as in Example 25. The spleen is isolated from each mice and the spleen cells are fused with myeloma cells to produce hybridomas. After cultivation for 1-3 weeks, the culture supernatant is analyzed by ELISA. The ELISA is performed by the method described in Examples 14, 15, 21, 22, 23, 24, 25, 33, 34, 37 and 38. As a result, human antibody positive clones and clones which are human antibody positive and specific to the antigen used in the immunization are obtained.

### Example 67

### Production of chimeric mice with heavy-chain gene-deficient host embryos

From those mice exhibiting agouti coat color among the progeny of the endogenous antibody heavy-chain one allele-disrupted TT2F cell clone-derived chimeric mice produced in Example 49, mice retaining the disrupted allele are selected by Southern blot analysis, PCR (Example 49) or the like (the expected possibility is 1/2). Offsprings born by the mating of those antibody heavy-chain-deficient heterozygous male and female mice are subjected to Southern blot analysis (see Example 49) and analysis of *µ* chain expression on the surface of lymphocytes (Kitamura et al., Nature, 350:423-, 1991), etc. As a result, antibody heavy-chain-deficient homozygotes can be obtained which hardly produce functional antibodies of their own (the expected possibility is 1/4; for the results in membrane-type µ chain-deficient mice, see Kitamura et al., Nature, 350:423-, 1991). Embryos obtained by mating the homozygous male and female mice bred in a clean environment may be used as hosts for producing chimeric mice. In this case, most of B cells functional in the resultant chimeric mice are derived from the injected ES cells. Other mouse strains which cannot produce their own functional B cells, such as RAG-2-deficient mouse (Sinkai et al., Cell, 68:855-, 1992), may also be used for this purpose. In this system, chimeric mice are produced by the same procedures as in Example 10, etc. using the mouse ES cell clone from Examples 62, 63 or 64 which is deficient in endogenous antibody heavy-chain & light-chain and which retains human chromosomes #14+#2, #14+#22 or #14+#2+#22 fragments. The resultant chimeric mice produce antibodies consisting mainly of human heavy-chain and light-chain by the expression of human antibody heavy-chain (on chromosome #14), light-chain κ (on chromosome #2) and light-chain λ (on chromosome #22) genes functional in ES cell-derived B cells.

### Example 68

### Retention of the human chromosome in offsprings of human chromosome #14 fragment-transferred ES cell-derived chimeric mice

Human chromosome #14 fragment retaining chimeric mice obtained by the method described in Example 9 or a modified method thereof in which mouse ES cells in Example 9 are replaced with TT2F (39, XO, see Example 39) are mixed and mated with wild-type ICR mice (albino, CREA JAPAN INC. ) to produce offsprings. Genomic DNA is prepared from the tail of those offspring mice exhibiting agouti coat color. The DNA is subjected to PCR to examine the retention of human chromosome #14 fragment (see Examples 9, 42 and 43). Mouse ES cell clones retaining human chromosome #14 fragment differentiate into functional oocytes or sperms in chimeric mice, as described in Examples 42 and 43, and thus human chromosome #14 fragment can be transmitted to the progenies derived from such oocytes or sperms. This means that it is possible to establish a mouse strain retaining human chromosome #14 fragment containing a human antibody heavy-chain gene which can transmit the transferred chromosome to its progeny. In this Example and the following Examples 69-74, "human chromosome #14 fragment" means one containing a human antibody heavy-chain gene; "human chromosome #2 fragment" means one containing the human antibody κ chain gene; and " human chromosome #22 fragment" means one containing the human antibody λ chain gene.

### Example 69

### Retention of the human chromosome in offsprings of human chromosome #22 fragment-transferred ES cell-derived chimeric mice

Human chromosome #22 fragment-retaining chimeric mice obtained by the method described in Example 30 or a modified method thereof in which mouse ES cells in Example 30 are replaced with TT2F (39, XO) are mixed and mated with ICR mice to produce offsprings. Genomic DNA is prepared from the tail of those offspring mice exhibiting agouti coat color. The DNA is subjected to PCR to examine the retention of human chromosome #22 fragment (see Examples 30, 42 and 43). Mouse ES cell clones retaining human chromosome #22 fragment differentiate into functional oocytes or sperms in chimeric mice, as described in Examples 42 and 43, and thus human chromosome #22 fragment can be transmitted to the progenies derived from such oocytes or sperms. This means that it is possible to establish a mouse strain retaining human chromosome #22 fragment containing the human antibody light-chain λ chain gene which can transmit the transferred chromosome to its progeny.

### Example 70

### Production of mice retaining both human chromosome #2 fragment and #14 fragment by mating

The human chromosome #2 fragment retaining mouse strain from Example 42 or 43 is mated with the human chromosome #14 fragment retaining mouse strain from Example 68 to produce offsprings. Genomic DNA is prepared from tails of the offspring mice. The DNA is analyzed by PCR, etc. (Examples 9, 42 and 43) to confirm those mice which retain both human chromosome #2 partial fragment and human chromosome #14 fragment.

### Example 71 .

### Production of mice retaining both human chromosome #22 (fragment) and #14 fragment by mating

The human chromosome #22 fragment retaining mouse strain from Example 69 is mated with the human chromosome #14 fragment retaining mouse strain from Example 68 to produce offsprings. Genomic DNA is prepared from tails of the offspring mice. The DNA is analyzed by PCR, etc. (Examples 30, 42 and 43) to confirm those mice which retain both human chromosome #22 fragment and #14 fragment.

### Example 72

### Production of mice retaining the three human chromosomes of #2 fragment, #14 fragment and #22 fragment by mating

The mouse strain retaining both human chromosome #2 fragment and #14 fragment obtained in Example 71 is mated with the mouse strain retaining a human chromosome #2 fragment obtained in Example 42 or 43 to produce offsprings. Genomic DNA is prepared from tails of the offspring mice. The DNA is analyzed by PCR, etc. (Examples 9, 30, 42 and 43) to confirm those mice which retain all of the three human chromosomes, # 22 fragment), #14 (fragment and #2 fragment. Alternatively, mice retaining all of the above three human chromosomes may also be obtained by mating the mouse strain retaining both human chromosome #2 fragment and #14 fragment from Example 70, with the mouse strain retaining a human chromosome #22 fragment from Example 69.

### Example 73

### Production of a complete human antibody-producing mouse strain by mating

The mouse strains retaining human chromosomes #2+#14 (Example 70), #14+#22 (Example 71) and #2+#14+#22 (Example 72), respectively, are repeatedly mated with mouse strains deficient in endogenous antibody heavy-chain (Example 67; Kitamura et al., Nature, 350:423-, 1991) or light-chain κ (Zou et al., EMBO J., 12:811-, 1993; Chen et al., EMBO J., 12:821-, 1993) gene. From the resultant offsprings, those mice retaining human chromosomes #2+#14, #14+#22 or #2+#14+#22 are selected by PCR analysis, etc. (Examples 9, 30, 42 and 43) to thereby establish a mouse strain which produces mainly complete human (Green et al., Nature Genetics, 7:13-, 1994; Lonberg et al., Nature, 368:856-, 1994).

### Example 74

### Production of a human antibody-producing hybridoma from a mouse strain which is obtained by mating and which retains human chromosome fragment(s) containing a human antibody gene(s)

The mice retaining human chromosome fragment(s) containing a human antibody gene(s) obtained in Example 42, 43, 68, 69, 70, 71, 72 or 73 are immunized with an antigen of interest in the same manner as in Example 25. The spleen is isolated from each mice and the spleen cells are fused with myeloma cells to produce hybridomas. After cultivation for 1-3 weeks, the culture supernatant is analyzed by ELISA. The ELISA is performed by the method described in Examples 14, 15, 21, 22, 25, 33, 34, 37 and 38. As a result, human antibody positive clones and clones which are human antibody positive and specific to the antigen used in the immunization are obtained.

### Example 75

### Detection and determination of mouse IgM in sera of chimeric mice derived from the mouse antibody heavy-chain both alleles-disrupted TT2F cell clone

From offspring mice born in the same manner as in Example 40, from the mouse antibody heavy-chain both alleles-disrupted TT2F cell clone (#131-3) from Example 51, three mice having chimerisms of 0%, 50% and 99%, respectively, were selected. Mouse IgM in their sera was detected and determined. Briefly, the chimeric mice of about 2 weeks after birth were bled and mouse IgM concentration in the sera was determined by ELIZA by the same procedures as in Example 14. A PBS-diluted anti-mouse IgM antibody (Kirkegaard & Perry Laboratories Inc., 01-18-03) was fixed, and then a PBS-diluted serum sample supplemented with 5% FBS was added thereto. Peroxidase-labeled anti-mouse IgM antibody (Kirkegaard & Perry Laboratories Inc., 074-1803) was added thereto and absorbance at 450 nm was determined using TMBZ as a substrate. Purified mouse IgM (Fermingen, 0308ID) was used as a standard. This standard was diluted stepwise with FBS-supplemented PBS. The results are shown in Table 20. Of the chimeric mice derived from the mouse antibody heavy-chain both alleles-disrupted TT2F cells, the mouse having a chimerism of 99% showed a low mouse IgM concentration. Thus, it was confirmed that the mouse heavy-chain gene from the ES cells hardly functions in this mouse.

**Table 20. Concentration of Mouse IgM in Chimeric Mice (ELISA)**

| Chimerism % | IgM (mg/ℓ) |
|---|---|
| 0 | 12 |
| 50 | 11 |
| 99 | 1.5 |

### INDUSTRIAL APPLICABILITY

According to the present invention, a chimeric mouse retaining foreign human chromosome fragment(s) and expressing the gene(s) on the chromosome fragment(s) is provided. It is possible to produce biologically active substances using the chimeric mouse of the present invention.

According to the present invention, a pluripotent cell retaining a foreign human chromosome fragment(s) and expressing the gene(s) on the chromosome fragment(s) is provided. The pluripotent cell can be used for treatment of hereditary diseases, for example, by bone marrow transplantation.

### SEQUENCE LISTING

SEQ ID NO: 1
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TGGAAGGTGG ATAACGCCCT 20
SEQ ID NO:2
   SEQUENCE LENGTH:22
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TCATTCTCCT CCAACATTAG CA 22
SEQ ID NO:3
   SEQUENCE LENGTH:21
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TGTAGGGGAC CTGGAGCCTT G 21
SEQ ID NO:4
   SEQUENCE LENGTH:21
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TTGACAACTC ACCTGGACTA G 21
SEQ ID NO:5
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CTCTCCTGCA GGGCCAGTCA 20
SEQ ID NO:6
   SEQUENCE LENGTH:22
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TGCTGATGGT GAGAGTGAAC TC 22
SEQ ID NO:7
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      AGTCAGGGCA TTAGCAGTGC 20
SEQ ID NO:8
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GCTGCTGATG GTGAGAGTGA 20
SEQ ID NO:9
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TGGTGGCTGA AAGCTAAGAA 20
SEQ ID NO: 10
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CCAGAAGAAT GGTGTCATTA 20
SEQ ID NO:11
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TCCAGGTTCT GCAGAGCAAG 20
SEQ ID NO:12
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TGTAGTTGGA GGCCATGTCC 20
SEQ ID NO:13
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CCCCACCCAT GATCCAGTAC 20
SEQ ID NO: 14
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GCCCTCAGAA GACGAAGCAG 20
SEQ ID NO: 15
   SEQUENCE LENGTH:22
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GAGAGTTGCA GAAGGGGTGA CT 22
SEQ ID NO:16
   SEQUENCE LENGTH:22
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GGAGACCACC AAACCCTCCA AA 22
SEQ ID NO: 17
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GGCTATGGGG ACCTGGGCTG 20
SEQ ID NO:18
   SEQUENCE LENGTH: 22
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CAGAGACACA GGCACGTAGA AG 22
SEQ ID NO: 19
   SEQUENCE LENGTH :20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TTAAGGGTCA CCCAGAGACT 20
SEQ ID NO: 20
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TGTAGTTGGA GGCCATGTCC 20
SEQ ID NO: 21
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CAAAAAGTCC AACCCTATCA 20
SEQ ID NO:22
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GCCCTCAGAA GACGAAGCAG 20
SEQ ID NO: 23
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TCGTTCCTGT CGAGGATGAA 20
SEQ ID NO:24
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TCACTCCGAA GCTGCCTTTC 20
SEQ ID NO: 25
   SEQUENCE LENGTH:21
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      ATGTACAGGA TGCAACTCCT G 21
SEQ ID NO:26
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TCATCTGTAA ATCCAGCAGT 20
SEQ ID NO:27
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GATCCCATCG CAGCTACCGC 20
SEQ ID NO: 28
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TTCGCCGAGT AGTCGCACGG 20
SEQ ID NO:29
   SEQUENCE LENGTH:2 2
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GATGAACTAG TCCAGGTGAG TT 22
SEQ ID NO:30
   SEQUENCE LENGTH:22
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CCTTTTGGCT TCTACTCCTT CA 22
SEQ ID NO:31
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      ATAGAGGGTA CCCACTCTGG 20
SEQ ID NO: 32
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      AACCAGGTAG GTTGATATGG 20
SEQ ID NO:33
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      AAGTTCCTGT GATGTCAAGC 20
SEQ ID NO: 34
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TCATGAGCAG ATTAAACCCG 20
SEQ ID NO:35
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TGTGAAGGAG GACCAGGTGT 20
SEQ ID NO: 36
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TGTAGGGGTT GACAGTGACA 20
SEQ ID NO: 37
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CTGAGAGATG CCTCTGGTGC 20
SEQ ID NO:38
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GGCGGTTAGT GGGGTCTTCA 20
SEQ ID NO: 39
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GGTGTCGTGG AACTCAGGCG 20
SEQ ID NO:40
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CTGGTGCAGG ACGGTGAGGA 20
SEQ ID NO:41
   SEQUENCE LENGTH: 20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GCATCCTGAC CGTGTCCGAA 20
SEQ ID NO: 42
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GGGTCAGTAG CAGGTGCCAG 20
SEQ ID NO:43
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      AGTGAGATAA GCAGTGGATG 20
SEQ ID NO:44
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GTTGTGCTAC TCCCATCACT 20
SEQ ID NO:45
   SEQUENCE LENGTH:21
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TTGTATTTCC AGGAGAAAGT G 20
SEQ ID NO:46
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GGAGACGAGG GGGAAAAGGG 20
SEQ ID NO: 47
   SEQUENCE LENGTH:27
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      ATGGACTGGA CCTGGAGGRT CYTCTKC 27
SEQ ID NO:48
   SEQUENCE LENGTH:27
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      ATGGAGYTTG GGCTGASCTG GSTTTYT 27
SEQ ID NO:49
   SEQUENCE LENGTH: 27
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      ATGRAMMWAC TKTGKWBCWY SCTYCTG 27
SEQ ID NO: 50
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CAGAGGCAGT TCCAGATTTC 20
SEQ ID NO:51
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TGGGATAGAA GTTATTCAGC 20
SEQ ID NO: 52
   SEQUENCE LENGTH:20
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      ATGGACATGR RRDYCCHVGY KCASCTT 27
SEQ ID NO: 53
   SEQUENCE LENGTH:28
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CCAAGCTTCA GGAGAAAGTG ATGGAGTC
SEQ ID NO: 54
   SEQUENCE LENGTH:28
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CCAAGCTTAG GCAGCCAACG GCCACGCT
SEQ ID NO: 55
   SEQUENCE LENGTH:28
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CCAAGCTTCA GAGGCAGTTC CAGATTTC
SEQ ID NO:56
   SEQUENCE LENGTH:28
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GGGAATTCGG GTAGAAGTCA CTGATCAG
SEQ ID NO:57
   SEQUENCE LENGTH:28
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GGGAATTCGG GTAGAAGTCA CTTATGAG
SEQ ID NO:58
   SEQUENCE LENGTH:28
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      GGGAATTCGG GTAGAAGTCA CTTACGAG
SEQ ID NO:59
   SEQUENCE LENGTH:60
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      ACCTTCATCG TCCTCTTCCT CCTGAGCCTC TTCTACAGCA CCACCGTCAC CCTGTTCAAG
SEQ ID NO:60
   SEQUENCE LENGTH:60
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      TGATGCTGCA CCAACTGTAT CCATCTTCCC ACCATCCAGT GAGCAGTTAA CATCTGGAGG
SEQ ID NO:61
   SEQUENCE LENGTH:22
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CTGGGGTGAG CCGGATGTTT TG
SEQ ID NO:62
   SEQUENCE LENGTH:22
   SEQUENCE TYPE: nucleic acid
   STRANDEDNESS: single
   TOPOLOGY: linear
   MOLECULE TYPE: Other nucleic acid synthetic DNA
   SEQUENCE DESCRIPTION
      CCAACCCAGC TCAGCCCAGT TC

## Claims

1. A method for producing a chimeric mouse retaining from one to three human chromosome fragment(s) selected from the group of chromosome 2, 14 and 22 fragments that have a base length not less than 1 Mb (1 million bp) and contain a gene, or the greater part of the functional sequences thereof, encoding a human antibody, which comprises preparing a microcell containing said chromosome fragment(s) and transferring said chromosome fragment(s) into a pluripotent non-human cell by fusion with the microcell.

2. A method for producing a pluripotent mouse cell retaining from one to three human chromosome fragment(s) selected from the group of chromosome 2, 14 and 22 fragments that have a base length not less than 1 Mb (1 million bp) and contain a gene, or the greater part of the functional sequences thereof, encoding a human antibody, which comprises preparing a microcell containing said chromosome fragment(s) and transferring said chromosome fragment(s) into a pluripotent cell by fusion with the microcell.

3. The method of claim 1 or 2, wherein the human antibody gene is the heavy-chain gene and a fragment of chromosome 14 is retained.

4. The method of any of claims 1-3, wherein the human antibody gene is the light-chain κ gene and a fragment of chromosome 2 is retained.

5. The method of any of claims 1-4, wherein the human antibody gene is the light-chain λ gene and a fragment of chromosome 22 is retained.

6. The method of any of claims 1-5, wherein the human antibody gene is the light- chain κ gene and a fragment of chromosome 2 is retained and the human antibody gene is the light-chain λ gene and a fragment of chromosome 22 is retained.

7. The method of any of claims 1-6, wherein the microcell containing said chromosome fragment(s) is induced from a hybrid cell prepared by the fusion of a cell from which said chromosome fragment(s) is (are) derived, with a cell having a high ability to form a microcell.

8. The method of claim 7, wherein the microcell containing said chromosome fragment(s) is induced from a cell prepared by a further fusion of the microcell induced from said hybrid cell with a cell having a high ability to form a microcell.

9. The method of claim 8, wherein the cell from which said chromosome fragment(s) is (are) derived is a human normal diploid cell.

10. The method of any one of claims 7-9, wherein the cell having a high ability to form a microcell is a mouse A9 cell.

11. The method of any one of claims 1-10, wherein the pluripotent non-human cell is an ES cell.

12. The method of any one of claims 1-11, wherein the pluripotent non-human cell has a disrupted gene homologous to said gene, or the greater part of the functional sequences thereof, encoding a human antibody on said chromosome fragment (s).

13. The method of any one of claims 1-12, wherein said chromosome fragment(s) containing said gene, or the greater part of the functional sequences thereof, encoding a human antibody is (are) transferred into a pluripotent non-human cell having a disrupted gene homologous to said gene, or the greater part of the functional sequences thereof, encoding a human antibody and then, a chimera is produced from the pluripotent cell by using an embryo of a mouse strain deficient in said disrupted gene.

14. The method of any one of claims 1-13, wherein the mouse strain deficient in the gene homologous to said gene, or the greater part of the functional sequences thereof, encoding a human antibody is produced by homologous recombination in gene targeting.

15. The method for producing a chimeric mouse according to any one of claims 1-14, wherein said chimeric mouse expresses said gene, or the greater part of the functional sequences thereof, encoding a human antibody on said chromosome fragment(s).

16. The method for producing a chimeric mouse according to claim 15, wherein said chimeric mouse transmits said chromosome fragment(s) to its progeny.

17. A chimeric mouse obtainable according to the method of any of claims 1-16 or a progeny thereof.

18. A chimeric mouse, or progeny thereof, retaining and expressing a gene, or the greater part of the functional sequences thereof, encoding a human antibody from a fragment of at least one of human chromosome 2, 14 or 22, that has a base length of not less than 1 Mb.

19. A chimeric mouse of claim 17 or 18, or progeny thereof, wherein the human antibody gene is the heavy-chain gene and a fragment of chromosome 14 is retained.

20. A chimeric mouse of any of claims 17-19, or progeny thereof, wherein the human antibody gene is the light-chain κ gene and a fragment of chromosome 2 is retained.

21. A chimeric mouse of any of claims 17-20, or progeny thereof, wherein the human antibody gene is the light-chain λ gene and a fragment of chromosome 22 is retained.

22. A chimeric mouse of any of claims 17-21, or progeny thereof, wherein the human antibody gene is the light-chain κ gene and a fragment of chromosome 2 is retained and the human antibody gene is the light-chain λ gene and a fragment of chromosome 22 is retained.

23. A chimeric mouse of any one of claims 17-22, or progeny thereof, which is deficient in a mouse antibody gene which is homologous to the human antibody gene.

24. A chimeric mouse of claim 23, or progeny thereof, wherein the deficiency of the mouse antibody gene is caused by disrupting said mouse antibody gene by homologous recombination.

25. A chimeric mouse of any of claims 17-24, or progeny thereof, expressing at least one class or subclass of human antibody.

26. A chimeric mouse of claim 25, or progeny thereof, wherein the class or subclass of human antibody is selected from IgM, IgG, IgE, IgA, IgD and their subclasses.

27. A chimeric mouse according to claim 17 or 18 obtainable by mating the chimeric mouse of claim 18, or progeny thereof, with a mouse strain deficient in a gene homologous to said gene, or the greater part of the functional sequences thereof, encoding a human antibody.

28. A chimeric mouse produced by mating the chimeric mouse of any of claims claim 18-27 or progeny thereof.

29. A tissue from the chimeric mouse of any of claims 17-28, or progeny thereof, said tissue retaining and expressing a gene, or the greater part of the functional sequences thereof, encoding a human antibody from one to three human chromosome fragment(s) selected from the group of chromosome 2, 14 or 22 fragments that have a base length of not less than 1 Mb.

30. A cell from the chimeric mouse of any of claims 17-28, or progeny thereof, said cell retaining and expressing a gene, or the greater part of the functional sequences thereof, encoding a human antibody from one to three human chromosome fragment(s) selected from the group of chromosome 2, 14 or 22 fragments that have a base length of not less than 1 Mb.

31. The cell of claim 30, which is a B cell or spleen cell.

32. A hybridoma prepared by the fusion of the cell of claim 31 with a myeloma cell.

33. A pluripotent mouse cell obtainable according to the method of any of claims 2-14.

34. Use of the pluripotent cell of claim 33 for producing a chimeric mouse.

35. A method for producing an antibody, which comprises expressing the gene, or the greater part of the functional sequences thereof, encoding a human antibody in the chimeric mouse of any one of claims 17-28, or progeny thereof, the tissue of claim 29, the cell of claims 30 or 31 or the hybridoma of claim 32, and recovering said antibody as an expression product.

36. A method for preparing an antibody, which comprises extracting a gene, or the greater part of the functional sequences thereof, encoding a human antibody from the chimeric mouse of any of claims 17-28, or progeny thereof, the tissue of claim 29, the cell of claims 30 or 31 or immortalized cells derived therefrom, and transforming said gene, or the greater part of the functional sequences thereof, into an animal or insect cell, culturing the cell under the conditions where the human antibody gene can be expressed, and collecting the antibody as the expression product.

37. A method for preparing an antibody, which comprises creating a cDNA comprising a gene, or the greater part of the functional sequences thereof, encoding a human antibody from the chimeric mouse of any of claims 17-28, or progeny thereof, the tissue of claim 29, the cell of claims 30 or 31 or immortalized cells derived therefrom, and transforming said cDNA into an animal or insect cell, culturing the cell under the conditions where the human antibody gene can be expressed, and collecting the antibody as the expression product.

38. The method of claim 36 or 37, wherein the animal or insect cell is selected from the group consisting of CHO cells, BHK cells, hepatoma cells, myeloma cells and SF9 cells.

39. A method for preparing a polyclonal antibody, which comprises immunizing the chimeric mouse of any of claims 17-28, or progeny thereof, with an antigen and collecting anti-serum from the blood or ascites of the mouse.

40. A method for preparing anti-serum, which comprises immunizing the chimeric mouse of any of claims 17-28, or progeny thereof, with an antigen and collecting anti-serum from the blood or ascites of the mouse.

41. A method for preparing a monoclonal antibody, which comprises immunizing the chimeric mouse of any of claims 17-28, or progeny thereof, with an antigen and collecting said monoclonal antibody from a hybridoma derived from said mouse.

42. A method for preparing an antibody comprising immunizing a mouse of any one of claims 17 to 28, or progeny thereof, with an antigen, extracting a gene, or the greater part of the functional sequences thereof, encoding a human antibody from said immunized mouse, transforming said gene, or the greater part of the functional sequences thereof, into a cell, culturing said cell and collecting the antibody against the antigen.

43. A method for isolating a DNA encoding a human antibody gene encoded by a fragment of at least one of human chromosome 2, 14 or 22, comprising extracting said DNA from the chimeric mouse of any of claims 17-28, or progeny thereof, the tissue of claim 29, the cell of claims 30 or 31 or immortalized cells derived therefrom.

## Patentansprüche

1. Verfahren zur Herstellung einer chimären Maus, die ein bis drei humane Chromosomenfragment(e), ausgewählt aus der Gruppe aus Chromosom 2-, 14- und 22-Fragmenten, beibehält, die eine Basenlänge von nicht weniger als 1 Mb (1 Million bp) aufweisen, und ein Gen, oder einen Grossteil der funktionellen Sequenzen davon, welches für einen humanen Antikörper codiert, enthalten, umfassend die Herstellung einer Mikrozelle, die das (die) besagte(n) Chromosomenfragment(e) enthält, und die Überführung des (der) besagten Chromosomenfragment(s/e) in eine pluripotente nicht-humane Zelle durch Fusion mit der Mikrozelle.

2. Verfahren zur Herstellung einer pluripotenten Mauszelle, die ein bis drei humane Chromosomenfragment(e), ausgewählt aus der Gruppe aus Chromosom 2-, 14- und 22-Fragmenten, beibehält, die eine Basenlänge von nicht weniger als 1 Mb (1 Million bp) aufweisen, und ein Gen, oder einen Grossteil der funktionellen Sequenzen davon, welches für einen humanen Antikörper codiert, enthalten, umfassend die Herstellung einer Mikrozelle, die das (die) besagte(n) Chromosomenfragment(e) enthält, und die Überführung des (der) besagten Chromosomenfragment (s/e) in eine pluripotente Zelle durch Fusion mit der Mikrozelle.

3. Verfahren nach Anspruch 1 oder 2, worin das Gen des humanen Antikörpers das der schweren Kette ist und ein Fragment des Chromosoms 14 beibehalten wird.

4. Verfahren nach einem der Ansprüche 1 bis 3, worin das Gen des humanen Antikörpers das der leichten Kette κ ist und ein Fragment des Chromosoms 2 beibehalten wird.

5. Verfahren nach einem der Ansprüche 1 bis 4, worin das Gen des humanen Antikörpers das der leichten Kette λ ist und ein Fragment des Chromosoms 22 beibehalten wird.

6. Verfahren nach einem der Ansprüche 1 bis 5, worin das Gen des humanen Antikörpers das der leichten Kette κ ist und ein Fragment des Chromosoms 2 beibehalten wird, und das Gen des humanen Antikörpers das der leichten Kette λ ist und ein Fragment des Chromosoms 22 beibehalten wird.

7. Verfahren nach einem der Ansprüche 1 bis 6, worin die Mikrozelle, die das (die) besagte(n) Chromosomenfragment(e) enthält, aus einer Hybridzelle induziert wird, die durch Fusion einer Zelle, von der das (die) besagte(n) Chromosomenfragment(e) stammt (stammen), mit einer Zelle, die eine hohe Fähigkeit zur Mikrozellenbildung aufweist, hergestellt wird.

8. Verfahren nach Anspruch 7, worin die Mikrozelle, die das (die) besagte(n) Chromosomenfragment(e) enthält, aus einer Zelle induziert wird, die durch weitere Fusion der Mikrozelle, induziert aus besagter Hybridzelle, mit einer Zelle, die eine hohe Fähigkeit zur Mikrozellenbildung aufweist, hergestellt wurde.

9. Verfahren nach Anspruch 8, worin die Zelle, von der das (die) besagte(n) Chromosomenfragment(e) stammt (stammen), eine normale humane, diploide Zelle ist.

10. Verfahren nach einem der Ansprüche 7 bis 9, worin die Zelle, die eine hohe Fähigkeit zur Mikrozellenbildung hat, eine Maus A9-Zelle ist.

11. Verfahren nach einem der Ansprüche 1 bis 10, worin die pluripotente nicht-humane Zelle eine ES-Zelle ist.

12. Verfahren nach einem der Ansprüche 1 bis 11, worin die pluripotente nicht-humane Zelle ein unterbrochenes Genhomolog des besagten Gens, oder des Grossteils der funktionellen Sequenzen davon, das für einen humanen Antikörper auf dem (den) besagten Chromosomenfragment(en) codiert, aufweist.

13. Verfahren nach einem der Ansprüche 1 bis 12, worin das (die) besagte(n) Chromosomenfragment(e), das (die) besagtes Gen, oder den Grossteil der funktionellen Sequenzen davon, welches für einen humanen Antikörper codiert, enthält (enthalten), in eine pluripotente nicht-humane Zelle transferiert wird (werden), die ein unterbrochenes Genhomolog des besagten Gens, oder des Grossteils der funktionellen Sequenzen davon, welches für einen humanen Antikörper codiert, aufweist, und dann eine Chimäre aus der pluripotenten Zelle unter Verwendung eines Embryos eines in besagtem unterbrochenen Gen defizitären Mausstammes erzeugt wird.

14. Verfahren nach einem der Ansprüche 1 bis 13, worin der Mausstamm defizitär in dem Genhomolog des besagten Gens, oder des Grossteils der funktionellen Sequenzen davon, welches für einen humanen Antikörper codiert, durch homologe Rekombination durch Gentargeting erzeugt wird.

15. Verfahren zur Herstellung einer chimären Maus gemäss einem der Ansprüche 1 bis 14, worin besagte chimäre Maus besagtes Gen, oder einen Grossteil der funktionellen Sequenzen davon, welches für einen humanen Antikörper auf besagte(m/n) Chromosomenfragment(en) codiert, exprimiert.

16. Verfahren zur Herstellung einer chimären Maus gemäss Anspruch 15, worin besagte chimäre Maus das (die) besagte(n) Chromosomenfragment(e) auf ihre Nachkommen überträgt.

17. Chimäre Maus, erhältlich nach dem Verfahren nach einem der Ansprüche 1 bis 16, oder ein Nachkomme davon.

18. Chimäre Maus, oder ein Nachkomme davon, die ein Gen oder einen Grossteil der funktionellen Sequenzen davon, welches einen humanen Antikörper codiert, enthalten auf einem Fragment von mindestens einem der humanen Chromosomen 2, 14 oder 22, welches eine Basenlänge von nicht weniger als 1 Mb aufweist, beibehält und exprimiert.

19. Chimäre Maus nach Anspruch 17 oder 18, oder ein Nachkomme davon, worin das Gen des humanen Antikörpers das der schweren Kette ist und ein Fragment des Chromosoms 14 beibehalten wird.

20. Chimäre Maus nach einem der Ansprüche 17 bis 19, oder ein Nachkomme davon, worin das Gen des humanen Antikörpers das der leichten Kette κ ist und ein Fragment des Chromosoms 2 beibehalten wird.

21. Chimäre Maus nach einem der Ansprüche 17 bis 20, oder ein Nachkomme davon, worin das Gen des humanen Antikörpers das der leichten Kette λ ist und ein Fragment des Chromosoms 22 beibehalten wird.

22. Chimäre Maus nach einem der Ansprüche 17 bis 21, oder ein Nachkomme davon, worin das humane Antikörpergen das der leichten Kette κ ist und ein Fragment des Chromosoms 2 beibehalten wird, und das humane Antikörpergen das der leichten Kette λ ist und ein Fragment des Chromosoms 22 beibehalten wird.

23. Chimäre Maus nach einem der Ansprüche 17 bis 22, oder ein Nachkomme davon, die defizitär in einem murinen Antikörpergen ist, welches homolog zum humanen Antikörpergen ist.

24. Chimäre Maus nach Anspruch 23, oder ein Nachkomme davon, worin die Defizienz in dem murinen Antikörpergen durch Unterbrechung des besagten murinen Antikörpergens aufgrund homologer Rekombination verursacht wird.

25. Chimäre Maus nach einem der Ansprüche 17 bis 24, oder ein Nachkomme davon, welche zumindest eine Klasse oder Unterklasse von humanen Antikörpern exprimiert.

26. Chimäre Maus nach Anspruch 25, oder ein Nachkomme davon, worin die Klasse oder Unterklasse des humanen Antikörpers ausgewählt ist aus IgM, IgG, IgE, IgA, IgD und deren Unterklassen.

27. Chimäre Maus gemäss Anspruch 17 oder 18, erhältlich durch Verpaarung der chimären Maus nach Anspruch 18, oder eines Nachkommen davon, mit einem Mausstamm, defizitär in einem Gen, das homolog zu dem besagten Gen ist, oder dem Grossteil der funktionellen Sequenzen davon, welches für einen humanen Antikörper codiert.

28. Chimäre Maus, erzeugt durch Verpaarung der chimären Maus nach einem der Ansprüche 18 bis 27, oder ein Nachkomme davon.

29. Gewebe aus der chimären Maus nach einem der Ansprüche 17 bis 28, oder eines Nachkommen davon, wobei besagtes Gewebe ein Gen, oder ein Grossteil der funktionellen Sequenzen davon, welches für einen humanen Antikörper codiert, von ein bis drei humanen Chromosomenfragment(en), ausgewählt aus der Gruppe der Chromosomen 2-, 14- oder 22-Fragmente, welche eine Basenlänge von nicht weniger als 1 Mb aufweisen behält und exprimiert.

30. Zelle aus der chimären Maus nach einem der Ansprüche 17 bis 28, oder eines Nachkommen davon, wobei besagte Zelle ein Gen, oder ein Grossteil der funktionellen Sequenzen davon, welches für einen humanen Antikörper codiert, von ein bis drei humanen Chromosomenfragment(en), ausgewählt aus der Gruppe der Chromosom 2-, 14- oder 22-Fragmente, welche eine Basenlänge von nicht weniger als 1 Mb aufweisen behält und exprimiert.

31. Zelle nach Anspruch 30, die eine B-Zelle oder eine Milzzelle ist.

32. Hybridom, hergestellt durch Fusion der Zelle von Anspruch 31 mit einer Myelomzelle.

33. Pluripotente Mauszelle, erhältlich nach dem Verfahren gemäss einem der Ansprüche 2 bis 14.

34. Verwendung der pluripotenten Zelle nach Anspruch 33 zur Herstellung einer chimären Maus.

35. Verfahren zur Herstellung eines Antikörpers, bestehend aus dem Exprimieren des Gens, oder des Grossteils der funktionellen Sequenzen davon, das einen humanen Antikörper codiert, in der chimären Maus nach einem der Ansprüche 17 bis 28, oder einem Nachkommen davon, dem Gewebe nach Anspruch 29, der Zelle nach den Ansprüchen 30 oder 31, oder dem Hybridom nach Anspruch 32 und Gewinnung des besagten Antikörpers als ein Expressionsprodukt.

36. Verfahren zur Herstellung eines Antikörpers, bestehend aus der Extraktion eines Gens, oder des Grossteils der funktionellen Sequenzen davon, das einen humanen Antikörper codiert, durch die chimäre Maus nach einem der Ansprüche 17 bis 28, oder einem Nachkommen davon, das Gewebe nach Anspruch 29, die Zelle nach Anspruch 30 oder 31, oder immortalisierte Zellen davon abstammend, und Transformation des besagten Gens, oder des Grossteils der funktionellen Sequenzen davon, in eine Tier- oder Insektenzelle, Kultivierung der Zelle unter den Bedingungen, unter denen das humane Antikörpergen exprimiert werden kann, und Sammeln des Antikörpers als das Expressionsprodukt.

37. Verfahren zur Herstellung eines Antikörpers, umfassend die Erzeugung einer cDNA, welche ein Gen, oder den Grossteil der funktionellen Sequenzen davon umfasst, welches einen humanen Antikörper codiert, durch die chimäre Maus nach einem der Ansprüche 17 bis 28, oder einen Nachkommen davon, das Gewebe nach Anspruch 29, die Zelle nach Ansprüchen 30 oder 31, oder immortalisierte Zellen davon abstammend, und die Transformation besagter cDNA in eine Tier- oder Insektenzelle, Kultivierung der Zelle unter den Bedingungen, unter denen das humane Antikörpergen exprimiert werden kann, und Sammeln des humanen Antikörpers als das Expressionsprodukt.

38. Verfahren nach Anspruch 36 oder 37, worin die Tier- oder Insektenzelle ausgewählt wird aus der Gruppe bestehend aus CHO-Zellen, BHK-Zellen, Hepatomzellen, Myelomzellen und SF9-Zellen.

39. Verfahren zur Herstellung eines polyklonalen Antikörpers, umfassend die Immunisierung der chimären Maus nach einem der Ansprüche 17 bis 28, oder eines Nachkommen davon, mit einem Antigen, und das Sammeln von Antiserum aus dem Blut oder Ascites der Maus.

40. Verfahren zur Herstellung von Antiserum, umfassend die Immunisierung der chimären Maus nach einem der Ansprüche 17 bis 28, oder eines Nachkommen davon, mit einem Antigen, und das Sammeln von Antiserum aus dem Blut oder Ascites der Maus.

41. Verfahren zur Herstellung eines monoklonalen Antikörpers, umfassend die Immunisierung der chimären Maus nach einem der Ansprüche 17 bis 28, oder eines Nachkommen davon, mit einem Antigen, und das Sammeln des besagten monoklonalen Antikörpers aus einer Hybridoma, abgeleitet von besagter Maus.

42. Verfahren zur Herstellung eines Antikörpers, umfassend die Immunisierung einer Maus nach einem der Ansprüche 17 bis 28, oder eines Nachkommen davon, mit einem Antigen, die Extraktion eines Gens, oder des Grossteils der funktionellen Sequenzen davon, welches einen humanen Antikörper codiert, aus besagter immunisierter Maus, Transformation des besagten Gens, oder des Grossteils der funktionellen Sequenzen davon, in eine Zelle, Kultivierung besagter Zelle und Sammeln des gegen das Antigen gerichteten Antikörpers.

43. Verfahren zur Isolierung einer DNA, die ein humanes Antikörpergen codiert, welches durch ein Fragment von mindestens einem der humanen Chromosomen 2, 14 oder 22 codiert wird, umfassend die Extraktion besagter DNA aus der chimären Maus nach einem der Ansprüche 17 bis 28, oder eines Nachkommen davon, dem Gewebe nach Anspruch 29, der Zelle nach Anspruch 30 oder 31, oder immortalisierte Zellen davon abstammend.

## Revendications

1. Procédé destiné à produire une souris chimère conservant de un à trois fragment(s) de chromosome humain choisi(s) dans le groupe des fragments du chromosome 2, 14 et 22 qui ont une longueur exprimée en paires de bases non inférieure à 1 Mb (1 million de pb) et qui contiennent un gène, ou la plus grande partie des séquences fonctionnelles de celui-ci, codant un anticorps humain, qui comprend la préparation d'une microcellule contenant le(les)dit(s) fragment(s) de chromosome et le transfert du(des)dit(s) fragment(s) de chromosome dans une cellule pluripotente non humaine par fusion avec la microcellule.

2. Procédé destiné à produire une cellule de souris pluripotente conservant de un à trois fragment(s) de chromosome humain choisi(s) dans le groupe des fragments du chromosome 2, 14 et 22 qui ont une longueur exprimée en paires de bases non inférieure à 1 Mb (1 million de pb) et qui contiennent un gène, ou la plus grande partie des séquences fonctionnelles de celui-ci, codant un anticorps humain, qui comprend la préparation d'une microcellule contenant le(les)dit(s) fragment(s) de chromosome et le transfert du(des)dit(s) fragment(s) de chromosome dans une cellule pluripotente par fusion avec la microcellule.

3. Procédé selon la revendication 1 ou 2, dans lequel le gène d'anticorps humain est le gène de chaîne lourde, et un fragment du chromosome 14 est conservé.

4. Procédé selon l'une quelconque des revendications 1 à 3, dans lequel le gène d'anticorps humain est le gène de chaîne légère κ, et un fragment du chromosome 2 est conservé.

5. Procédé selon l'une quelconque des revendications 1 à 4, dans lequel le gène d'anticorps humain est le gène de chaîne légère λ, et un fragment du chromosome 22 est conservé.

6. Procédé selon l'une quelconque des revendications 1 à 5, dans lequel le gène d'anticorps humain est le gène de chaîne légère κ et un fragment du chromosome 2 est conservé, et le gène d'anticorps humain est le gène de chaîne légère λ et un fragment du chromosome 22 est conservé.

7. Procédé selon l'une quelconque des revendications 1 à 6, dans lequel la microcellule contenant le(les)dit(s) fragment(s) de chromosome est induite à partir d'une cellule hybride préparée par la fusion d'une cellule depuis laquelle le(les)dit(s) fragment (s) de chromosome est(sont) dérivé(s), avec une cellule ayant une aptitude élevée à former une microcellule.

8. Procédé selon la revendication 7, dans lequel la microcellule contenant le(les)dit(s) fragment(s) de chromosome est induite à partir d'une cellule préparée par une autre fusion de la microcellule induite à partir de ladite cellule hybride avec une cellule ayant une aptitude élevée à former une microcellule.

9. Procédé selon la revendication 8, dans lequel la cellule à partir de laquelle le (les) dit (s) fragment(s) de chromosome est(sont) dérivé(s) est une cellule diploïde normale humaine.

10. Procédé selon l'une quelconque des revendications 7 à 9, dans lequel la cellule ayant une aptitude élevée à former une microcellule est une cellule A9 de souris.

11. Procédé selon l'une quelconque des revendications 1 à 10, dans lequel la cellule non humaine pluripotente est une cellule ES.

12. Procédé selon l'une quelconque des revendications 1 à 11, dans lequel la cellule non humaine pluripotente possède un gène interrompu, homologue audit gène, ou à la plus grande partie des séquences fonctionnelles de celui-ci, codant un anticorps humain sur le(les)dit(s) fragment(s) de chromosome.

13. Procédé selon l'une quelconque des revendications 1 à 12, dans lequel le(les)dit(s) fragment(s) de chromosome contenant ledit gène, ou la plus grande partie des séquences fonctionnelles de celui-ci, codant un anticorps humain est(sont) transféré(s) dans une cellule non humaine pluripotente ayant un gène interrompu, homologue audit gène, ou à la plus grande partie des séquences fonctionnelles de celui-ci, codant un anticorps humain et ensuite, une chimère est produite à partir de la cellule pluripotente en utilisant un embryon d'une souche de souris déficiente en ledit gène interrompu.

14. Procédé selon l'une quelconque des revendications 1 à 13, dans lequel la souche de souris déficiente en gène homologue audit gène, ou à la plus grande partie des séquences fonctionnelles de celui-ci, codant un anticorps humain est produite par recombinaison homologue dans un ciblage de gène.

15. Procédé destiné à produire une souris chimère selon l'une quelconque des revendications 1 à 14, dans lequel ladite souris chimère exprime ledit gène, ou la plus grande partie des séquences fonctionnelles de celui-ci, codant un anticorps humain sur le(les)dit(s) fragment(s) de chromosome.

16. Procédé destiné à produire une souris chimère selon la revendication 15, dans lequel ladite souris chimère transmet le(les)dit(s) fragment(s) de chromosome à sa descendance.

17. Souris chimère pouvant être obtenue selon le procédé de l'une quelconque des revendications 1 à 16 ou un descendant de celle-ci.

18. Souris chimère, ou descendant de celle-ci, conservant et exprimant un gène, ou la plus grande partie des séquences fonctionnelles de celui-ci, codant un anticorps humain à partir d'un fragment d'au moins un parmi le chromosome humain 2, 14 ou 22, qui possède une longueur exprimée en paires de bases de pas moins de 1 Mb.

19. Souris chimère selon la revendication 17 ou 18, ou descendant de celle-ci, dans laquelle le gène d'anticorps humain est le gène de chaîne lourde, et un fragment du chromosome 14 est conservé.

20. Souris chimère selon l'une quelconque des revendications 17 à 19, ou descendant de celle-ci, dans laquelle le gène d'anticorps humain est le gène de chaîne légère κ, et un fragment du chromosome 2 est conservé.

21. Souris chimère selon l'une quelconque des revendications 17 à 20, ou descendant de celle-ci, dans laquelle le gène d'anticorps humain est le gène de chaîne légère λ, et un fragment du chromosome 22 est conservé.

22. Souris chimère selon l'une quelconque des revendications 17 à 21, ou descendant de celle-ci, dans laquelle le gène d'anticorps humain est le gène de chaîne légère K et un fragment du chromosome 2 est conservé, et le gène d'anticorps humain est le gène de chaîne légère λ et un fragment du chromosome 22 est conservé.

23. Souris chimère selon l'une quelconque des revendications 17 à 22, ou descendant de celle-ci, qui est déficiente en un gène d'anticorps de souris qui est homologue d'anticorps humain.

24. Souris chimère selon la revendication 23, ou descendant de celle-ci, dans laquelle la déficience du gène d'anticorps de souris est provoquée par l'interruption dudit gène d'anticorps de souris par recombinaison homologue.

25. Souris chimère selon l'une quelconque des revendications 17 à 24, ou descendant de celle-ci, exprimant au moins une classe ou une sous-classe d'anticorps humain.

26. Souris chimère selon la revendication 25, ou descendant de celle-ci, dans laquelle la classe ou la sous-classe d'anticorps humain est choisie parmi les IgM, IgG, IgE, IgA, IgD et leurs sous-classes.

27. Souris chimère selon la revendication 17 ou 18 pouvant être obtenue par accouplement de la souris chimère selon la revendication 18, ou d'un descendant de celle-ci, avec une souche de souris déficiente en un gène homologue audit gène, ou à la plus grande partie des séquences fonctionnelles de celui-ci, codant un anticorps humain.

28. Souris chimère produite par accouplement de la souris chimère selon l'une quelconque des revendications 18 à 27 ou d'un descendant de celle-ci.

29. Tissu provenant de la souris chimère selon l'une quelconque des revendications 17 à 28, ou d'un descendant de celle-ci, ledit tissu conservant et exprimant un gène, ou la plus grande partie des séquences fonctionnelles de celui-ci, codant un anticorps humain à partir d'un à trois fragment(s) de chromosome humain choisi(s) dans le groupe des fragments du chromosome 2, 14 ou 22 qui ont une longueur exprimée en paires de bases de pas moins de 1 Mb.

30. Cellule provenant de la souris chimère selon l'une quelconque des revendications 17 à 28, ou d'un descendant de celle-ci, ladite cellule conservant et exprimant un gène, ou la plus grande partie des séquences fonctionnelles de celui-ci, codant un anticorps humain à partir d'un à trois fragment(s) de chromosome humain choisi(s) dans le groupe des fragments du chromosome 2, 14 ou 22 qui ont une longueur exprimée en paires de bases de pas moins de 1 Mb.

31. Cellule selon la revendication 30, qui est une cellule B ou une cellule de rate.

32. Hybridome préparé par la fusion de la cellule selon la revendication 31 avec une cellule de myélome.

33. Cellule de souris pluripotente pouvant être obtenue selon le procédé de l'une quelconque des revendications 2 à 14.

34. Utilisation de la cellule pluripotente de la revendication 33 pour produire une souris chimère.

35. Procédé destiné à produire un anticorps, qui comprend l'expression du gène, ou de la plus grande partie des séquences fonctionnelles de celui-ci, codant un anticorps humain dans la souris chimère selon l'une quelconque des revendications 17 à 28, ou un descendant de celle-ci, le tissu selon la revendication 29, la cellule selon la revendication 30 ou 31 ou l'hybridome selon la revendication 32, et la récupération dudit anticorps en tant que produit d'expression.

36. Procédé destiné à préparer un anticorps, qui comprend l'extraction d'un gène, ou de la plus grande partie des séquences fonctionnelles de celui-ci, codant un anticorps humain provenant de la souris chimère selon l'une quelconque des revendications 17 à 28, ou d'un descendant de celle-ci, le tissu selon la revendication 29, la cellule selon la revendication 30 ou 31 ou des cellules immortalisées dérivées de celle-ci, et la transformation dudit gène, ou de la plus grande partie des séquences fonctionnelles de celui-ci, dans une cellule d'animal ou d'insecte, la mise en culture de la cellule dans les conditions où le gène d'anticorps humain peut être exprimé, et le recueil de l'anticorps en tant que produit d'expression.

37. Procédé destiné à préparer un anticorps, qui comprend la création d'un ADNc comprenant un gène, ou la plus grande partie des séquences fonctionnelles de celui-ci, codant un anticorps humain à partir de la souris chimère selon l'une quelconque des revendications 17 à 28, ou d'un descendant de celle-ci, le tissu selon la revendication 29, la cellule selon la revendication 30 ou 31 ou des cellules immortalisées dérivées de celle-ci, et la transformation dudit ADNc dans une cellule d'animal ou d'insecte, la mise en culture de la cellule dans les conditions où le gène d'anticorps humain peut être exprimé, et le recueil de l'anticorps en tant que produit d'expression.

38. Procédé selon la revendication 36 ou 37, dans lequel la cellule d'animal ou d'insecte est choisie dans le groupe constitué des cellules CHO, cellules BHK, cellules d'hépatome, cellules de myélome et cellules SF9.

39. Procédé destiné à préparer un anticorps polyclonal, qui comprend l'immunisation de la souris chimère selon l'une quelconque des revendications 17 à 28, ou d'un descendant de celle-ci, avec un antigène et le recueil de l'anti-sérum à partir du sang ou de l'ascite de la souris.

40. Procédé destiné à préparer un anti-sérum qui comprend l'immunisation de la souris chimère selon l'une quelconque des revendications 17 à 28, ou d'un descendant de celle-ci, avec un antigène et le recueil de l'anti-sérum à partir du sang ou de l'ascite de la souris.

41. Procédé destiné à préparer un anticorps monoclonal, qui comprend l'immunisation de la souris chimère selon l'une quelconque des revendications 17 à 28, ou d'un descendant de celle-ci, avec un antigène et le recueil dudit anticorps monoclonal à partir d'un hybridome dérivé de ladite souris.

42. Procédé destiné à préparer un anticorps comprenant l'immunisation d'une souris selon l'une quelconque des revendications 17 à 28, ou d'un descendant de celle-ci, avec un antigène, l'extraction d'un gène, ou de la plus grande partie des séquences fonctionnelles de celui-ci, codant un anticorps humain à partir de ladite souris immunisée, la transformation dudit gène, ou de la plus grande partie des séquences fonctionnelles de celui-ci, dans une cellule, la mise en culture de ladite cellule et le recueil de l'anticorps contre l'antigène.

43. Procédé destiné à isoler un ADN codant un gène d'anticorps humain codé par un fragment d'au moins l'un parmi le chromosome humain 2, 14 ou 22, comprenant l'extraction dudit ADN à partir de la souris chimère selon l'une quelconque des revendications 17 à 28, ou d'un descendant de celle-ci, le tissu selon la revendication 29, la cellule selon la revendication 30 ou 31 ou des cellules immortalisées dérivées de celle-ci.
